# EUROPEAN PATENT APPLICATION

(11) **EP 2 338 892 A1**
(43) Date of publication of application: **29.06.2011**
(21) Application number: 09075562.0
(22) Date of filing: 18.12.2009
(51) Int. Cl.: C07F 7/18, C07F 9/38, C07F 9/40, C07F 9/655, A61K 31/662, A61K 31/665, A61P 25/00, A61P 35/00, C07B 59/00, A61K 51/04

(54) **Prostate specific membrane antigen inhibitors**

(71) Applicant: Bayer Schering Pharma Aktiengesellschaft, 13353 Berlin (DE)
(72) Inventor: The designation of the inventor has not yet been filed

(57) **Abstract**

This invention relates to novel compounds suitable for labelling by ¹⁸F and the corresponding ¹⁸F labelled compounds themselves, ¹⁹F-fluorinated analogues thereof and their use as reference standards, methods of preparing such compounds, compositions comprising such compounds, kits comprising such compounds or compositions and uses of such compounds, compositions or kits for diagnostic imaging by positron emission tomography (PET).

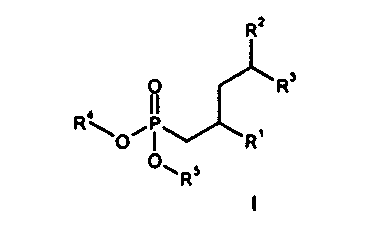

## Description

### Field of Invention

This invention relates to novel compounds suitable for labelling by ¹⁸F and the corresponding ¹⁸F labelled compounds themselves, ¹⁹F-fluorinated analogues thereof and their use as reference standards, methods of preparing such compounds, compositions comprising such compounds, kits comprising such compounds or compositions and uses of such compounds, compositions or kits for diagnostic imaging by Positron Emission Tomography (PET).

### Background

Molecular imaging has the potential to detect disease progression or therapeutic effectiveness earlier than most conventional methods in the fields of oncology, neurology and cardiology. Of the several promising molecular imaging technologies having been developed as optical imaging and MRI, PET is of particular interest for drug development because of its high sensitivity and ability to provide quantitative and kinetic data.

Prostate cancer is a leading cancer in the world, in particular in the US population where it is the second leading cause of cancer-related deaths in men. There are more than 300,000 new cases of prostate cancer diagnosed each year in the United States. Approximately US$2 billion is currently spent worldwide on surgical, radiation, drug therapy and minimally invasive treatments. Currently there is no effective therapy for relapsing, metastatic, androgen-independent prostate cancer. New agents that image prostate cancer are needed, preferably imaging agents containing radioisotopes, in particular positron emitting isotopes.

Positron emitting isotopes include carbon, nitrogen, and oxygen. These isotopes can replace their non-radioactive counterparts in target compounds to produce tracers that function biologically and are chemically identical to the original molecules for PET imaging. On the other hand, ¹⁸F is the most convenient labelling isotope due to its relatively long half life (109.6 min) which permits the preparation of diagnostic tracers and subsequent study of biochemical processes. In addition, its high ß+ yield and low ß+ energy (635 keV) are also advantageous.

The aliphatic ¹⁸F-fluorination reaction is of great importance for ¹⁸F-labeled radiopharmaceuticals which are used as *in vivo* imaging agents targeting and visualizing diseases, e.g. solid tumours or diseases of brain. A very important technical goal in using ¹⁸F-labelled radiopharmaceuticals is the quick preparation and administration of the radioactive compound due to the fact that the ¹⁸F isotopes have a short half-life of about only 110 minutes.

The best known example for PET imaging of diseases is 2-[¹⁸F]fluorodeoxyglucose ([¹⁸F]FDG), which is the most widely used PET radiopharmaceutical [J Nucl Med (1978), 19: 1154-1161].

However, a number of pitfalls and artefacts have been ascribed to FDG imaging and more continue to surface as the worldwide experience with FDG increases. The area most common for interpretative pitfalls with FDG is related to uptake in active skeletal muscle (Seminars in Nuclear Medicine, (2004), XXXIV, 2, pp.122-133). Many benign conditions can cause high accumulation of FDG creating the potential for false positive interpretation. Most of these artefacts are related to inflammatory, infective or granulomatous processes (Seminars in Nuclear Medicine, (2004), XXXIV, 2, pp.122-133, Seminars in Nuclear Medicine, (2004), XXXIV, 1, pp.56-69, (2004), J Nucl Med (2004), 45, pp. 695-700). Other tumours including mucosal associated lymphomas, small lymphocytic cell lymphoma, some neuroendocrine tumours, sclerotic bone metastases and renal cell carcinomas can be virtually inconspicuous due to low uptake or higher neighbouring background activity. Specifically related to PET-CT are pitfalls associated with breathing pattern differences between modalities, despite dedicated combined scanners (Seminars in Nuclear Medicine, (2004), XXXIV, 2, pp.122-133). For Prostate Cancer, uptake of [18F] FDG has been found to be low, likely due to the slow growing nature of many prostate tumours. As a consequence, in a large meta analysis (Gambhir SS, Czernin J, Schwimmer J, Silverman DH, Coleman RE, Phelps ME. A tabulated summary of the FDG PET literature. J Nucl Med. 2001 May;42(5 Suppl):1S-93S.) only 57% of prostatic carcinomas could be detected by [18F] FDG.

Current methods for imaging prostate cancer are predominantly computed tomography (CT), magnetic resonance (MR) and ultrasound, however, these methods are only anatomic methods and do not have the same sensitivity than SPECT or PET. The radiolabelled monoclonal antibody [111In]-Prostascint™ is currently a marketed imaging agent for prostate cancer, but the images obtained from this agent are difficult to interpret (Lange et al., Urology, 2001, 57, 402-406, Haseman et al., Cancer Biother. Radiopharm., 2000, 15, 131-140).

Especially for the PET imaging of prostate cancer, but also for other type of cancers, tetra-substituted ammonium derivatives labelled with C-11 and F-18 isotopes and based on choline structure have been described (e.g. JP09048747A, WO2001082864A2 (incl. US 2002061279A1). Among these derivatives [methyl-(C-11)]choline (CH), [F-18]fluorocholine (FCH), [F-18]fluoroethylcholine (FEC), [F-18]fluoromethylethylcholine (FMEC) and [F-18]fluoropropylcholine (FPC) are the best investigated compounds (see Scheme A; e.g. Nuclear Medicine (2001), 42(12), 1805-1814). Also known are [18F]fluorodihydrotestosterone (FDHT), anti-1-amino-3-[18F]fluorocyclobutyl-1-carboxylic acid (FACBC), [11C]acetate, and 1-(2-deoxy-2-[18F]fluoro-L-arabinofuranosyl)-5-methyuracil (FMAU) (Scher et al., Eur. J. Nucl. Med. Mol. Imaging 2007, 34, 45-53; Rinnab et al., BJU Int., 2007, 100, 786-793; Reske at al., J. Nucl. Med., 2006, 47, 1249-1254; Zophel et al., Eur. J. Nucl. Med. Mol. Imaging 2004, 31, 756-759; Vees et al., BJU Int., 2007, 99, 1415-1420; Larson et al., J. Nucl. Med., 2004, 45, 366-373; Schuster et al., J. Nucl. Med., 2007, 48, 56-63; Tehrani et al., J. Nucl. Med., 2007, 48, 1436-1441). These different agents all work via different mechanisms, each with its own advantage, e.g. low bladder activity for FACBC.

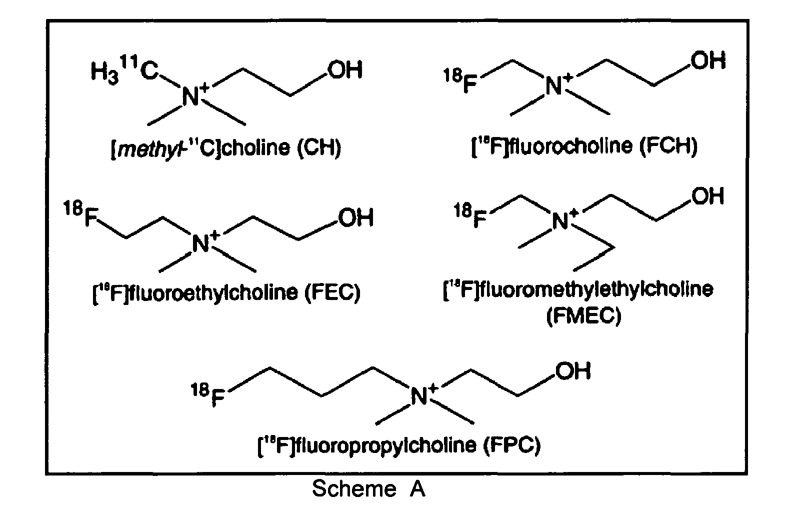

Initial clinical results of these above-mentioned compounds indicated somewhat better enrichment in prostate cancer tumours as compared to [18F]FDG. Nevertheless, imaging tracers with improved sensitivity/specificity profiles remain highly needed.

PSMA has been described as a highly promising target for prostate cancer imaging and therapy. PSMA, a trans-membrane 750 amino acid type II glycoprotein also known as GCPII or FOLH1, was first described in the context of neurotransmitter release in rat brain (Robinson MB, Blakely RD, Couto R, Coyle JT. Hydrolysis of the brain dipeptide N-acetyl-L-aspartyl-L-glutamate. Identification and characterization of a novel N-acetylated alpha-linked acidic dipeptidase activity from rat brain. J Biol Chem. 1987 Oct 25;262(30):14498-506.). Glutamate is known to play an important role as an excitatory neurotransmitter in both the central and peripheral nervous systems which in excess is associated with a number of neurological indications such as stroke, amyotrophic lateral sclerosis (ALS), chronic pain, epilepsy, and schizophrenia. A major source of glutamate in the nervous system is thought to be released via the hydrolysis of N-acetylaspartylglutamate (NAAG) to yield N-acetylaspartate and glutamate (Scheme B). A NAAG-hydrolyzing enzyme was proposed when the release of glutamate in rat brain cortex membranes treated with NAAG was observed [Riveros et al., Brain Res., 1984, 299, 393-395]. This enzyme was identified and characterized in the rat nervous system, which hydrolyzes NAAG into N-acetylaspartate and glutamate (Scheme B) and as termed N-acetylated-α-linked acidic dipeptidase (NAALADase) [Robinson et al, J. Biol. Chem., 1987, 262, 14498-14506].

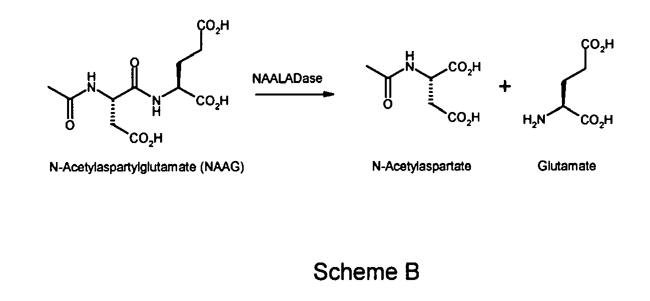

PSMA was identified as a potential biomarker for prostate cancer in 1996 [Carter et al., Proc. Natl. Acad. Sci. U.S.A., 1996, 93, 749-753] and confirmed as a promising target for prostate cancer imaging and therapy due to its abundant and specific expression both on primary and advanced prostate cancer cells (Schulke et al. 2003 PNAS USA, 100, 12590-12595). It was also found to be upregulated in the neovasculature of other solid tumours (Chang et al. 1999, CanRes, 59, 3192-3198).

The literature has identified a number of different classes of inhibitors for PSMA and these are shown in Scheme C [Tsukamoto et al., Drug Discovery Today, 2007, 12, 767-776; Liu et al., Biochemistry, 2008, 47, 12658-12660; Zhou et al., Nature Reviews, 2005, 4, 1015-1026].

The most potent compound of these, 2-(phosphonomethyl)pentanedioic acid, hereinafter referred to as 2-PMPA, has been identified as a powerful inhibitor of NAALADase (P. F. Jackson et al., J. Med. Chem. 1996, 39, 619). The stereospecificity of its bioactivity was thoroughly investigated to reveal the *(S)* enantiomer, featuring a glutamate-analogous absolute configuration at C-2, as the virtually sole bearer of NAALADase / GCP II inhibitory activity (D. Vitharana et al., Tetrahedron Asymmetry 2002, 13, 1609; T. Tsukamoto at al., J. Med. Chem. 2005, 48, 2319).

A series of patent applications describes the use of 2-PMPA and its analogues for the treatment of various diseases, in particular neurological disorders, such as compulsive disorders (US 5977090, WO 1998/013044), anxiety (WO 2001/001974), amyotrophic lateral sclerosis (WO 2001/091738), or opiod tolerance (WO 2004/078180), but also glaucoma, retinopathy, and age-related macular degeneration (WO 2001/092274). Structurally related, but distinct phosphinate derivatives are described as NAALADase inhibitors in WO1998/053812 and WO 1999/033847; prodrugs of 2-PMPA analogues, in particular phosphinates, for the therapy of *inter alia* neurological diseases and prostate cancer, are disclosed in WO 1999/033849. 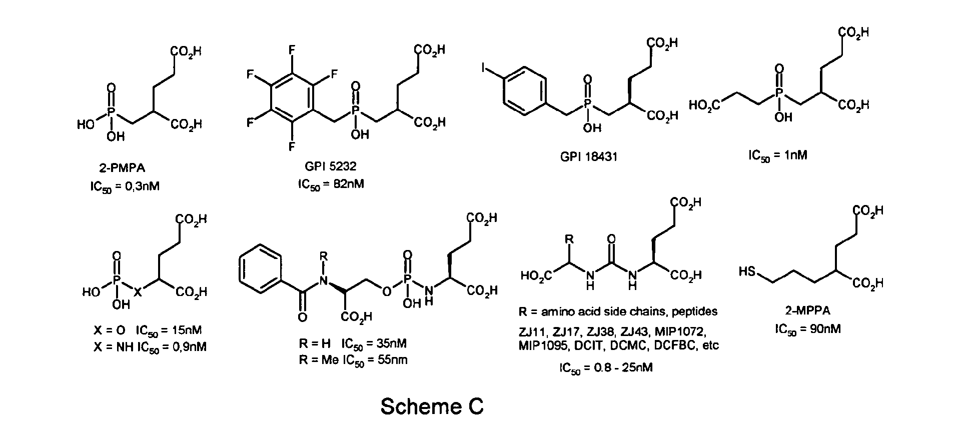 The state of the art regarding radiolabelled inhibitors of PSMA for the imaging of prostate cancer focuses on the urea class, e.g. [¹¹C]DCMC, [¹⁸F]DCFBC (US2004/0054190), [¹²³1]MIP1072 (WO2008/058192), [¹³¹I]MIP1095 (WO2008/058192) or [¹²⁵I]DCIT). There has been one example with a phosphoramidate peptidomimetic labelled with F-18 (US2007/0219165, Poster WMIC Nice 2008). These compounds are illustrated in Scheme D. 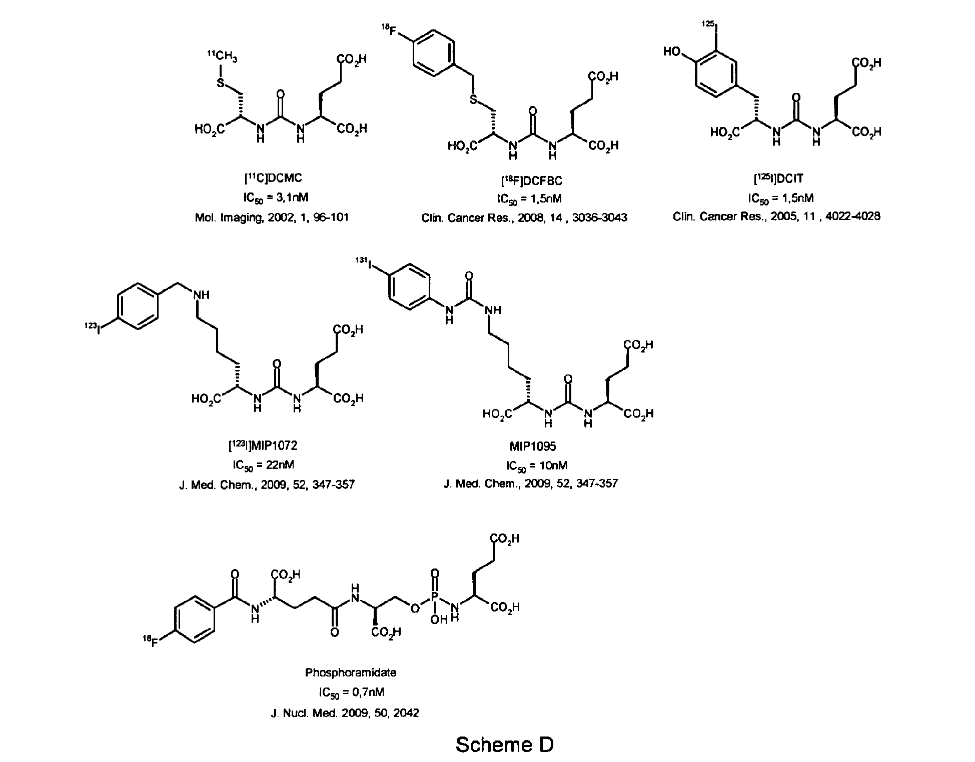 There are also examples of PSMA inhibitors radiolabelled with radiometals, i.e. [^{99m}Tc]L1 (J. Med. Chem., 2008, 51, 7933 and references cited within and WO2009/002529) and DUPA-^{99m}TC (Kularatne et al., Mol Pharmaceutics 2009, 6, 780; Kularatne et al., Mol. Pharamceutics 2009, 6, 790).

For all the inhibitors in Scheme C and D the right-hand side containing the pentanedicarboxylic acid portion remained unchanged and the alterations were all made on the left-hand side of the molecule. In a limited structure activity relation analysis (Scheme E) the pentanedicarboxylic portion on the urea inhibitor class has been investigated (J. Med. Chem., 2004, 47, 1729). The conclusion made from these investigations was that the pentanedicarboxylic portion has to be unchanged to maintain the binding affinity to PSMA. 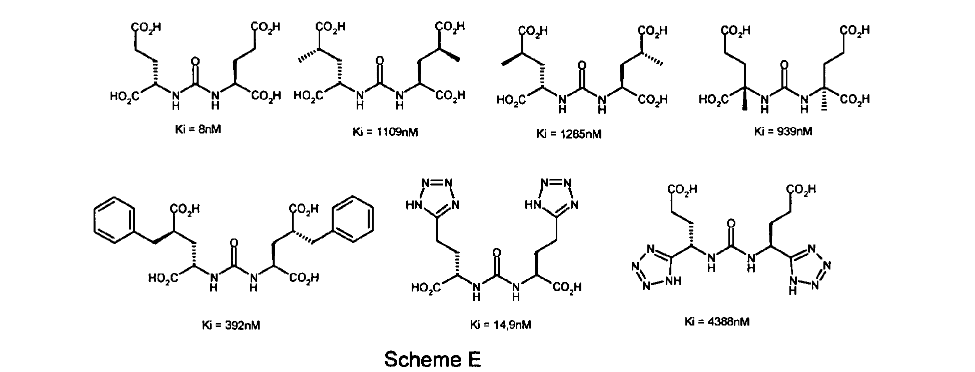 For the PMPA class the tight structure activity relation (Scheme F) is known (J. Med. Chem. 2003, 46, 1989 and J. Med. Chem. 2001, 44, 4170). The conclusions are that the chain length between the two carboxylic acid functions as well as the spacers between the zinc-binding phosphonic acid moiety are important.

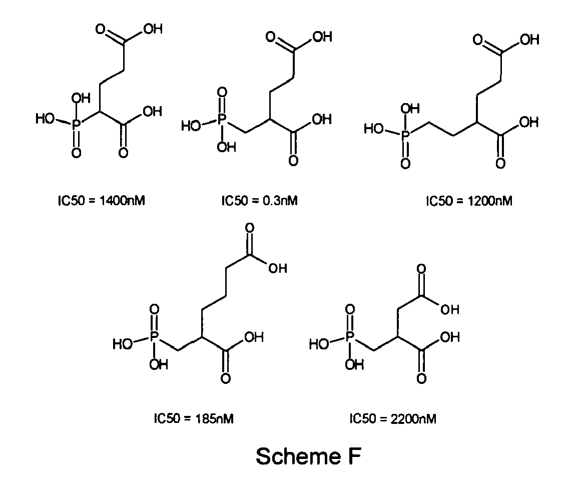

### Problem to be solved by the invention and its solution

Despite the aforementioned advances in finding suitable agents binding to PSMA for the imaging of prostate cancer, there remains a need for novel agents suitable for exploitation of the advantages of positron emission tomography *inter alia* with regard to spatial resolution, which also allow for practical use in a clinical PET centre. More specifically, the ¹⁸F labelled PSMA inhibitors known so far, including the two examples shown in Scheme D, depend on a multistep radiosynthesis involving so-called prosthetic groups, *i.e.* radiolabelled intermediates which need to be prepared separately from suitable starting materials and ¹⁸F-fluoride as produced by cyclotron. This extends the time required for the transformation of ¹⁸F-fluoride into the imaging agent and constitutes a practical impediment to routine clinical use.

Compounds of the present invention feature a fluorine substitution directly attached to the pentanedioic acid scaffold and yet have found to be potent inhibitors of PSMA, which is unexpected when considering the teachings of the SAR information from prior art as summarised in Scheme E. Moreover, the compounds of the present invention allow for the direct incorporation of ¹⁸F-fluoride without the necessity for so-called prosthetic groups, and are powerful agents for the imaging of prostate cancer.

### Summary of the Invention

This invention relates to compounds suitable for labelling by ¹⁸F and the corresponding ¹⁸F labelled compounds themselves, ¹⁹F-fluorinated analogues thereof (compound of formula I, IF18, I-F19 and I-LG) and their use for imaging diseases associated with altered expression of Prostate Specific Membrane Antigen PSMA or as reference standards, methods of preparing such compounds, compositions comprising such compounds, kits comprising such compounds or compositions and uses of such compounds, compositions or kits for diagnostic imaging by Positron Emission Tomography (PET).

### Detailed Description of the Invention

In a **first** aspect, the invention is directed to compounds of the formula 1, 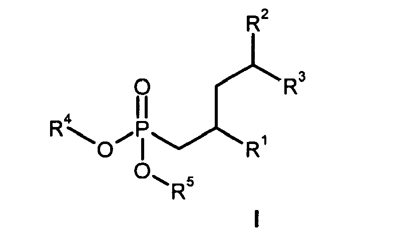 wherein
R¹ is C(=O)OR⁶;
R² is C(=O)OR , or 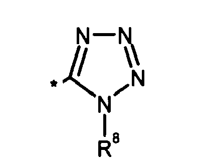 wherein the asterisk indicates the point of attachment into formula I;
R³ is selected from the group comprising ¹⁹F, ¹⁸F, and LG,
wherein LG is an appropriate leaving group, selected from the group comprising chloro, bromo, iodo, and -OS(=O)₂R⁹;
R⁴ and R⁵ are selected independently from each other from the group comprising hydrogen, optionally substituted C₁-C₁₀-alkyl, optionally substituted C₃-C₇-cycloalkyl, optionally substituted C₆-C₁₀-aryl, and optionally substituted C₇-C₁₄-arylalkyl, wherein zero, one or two of the carbon atoms constituting said alkyl group, cycloalkyl group, or the alkyl portion of said arylalkyl group, is optionally replaced by -C(=O)-, -NR¹⁰-, or -O- or
R⁴ and R⁵ together form an optionally substituted C₂-C₆ alkylene tether;
R⁶ and R⁷ are selected independently from each other from the group comprising hydrogen, optionally substituted C₁-C₁₀-alkyl, optionally substituted C₃-C₇-cycloalkyl, optionally substituted C₆-C₁₀-aryl, and optionally substituted C₇-C₁₄-arylalkyl, wherein zero, one or two of the carbon atoms constituting said alkyl group, cycloalkyl group, or the alkyl portion of said arylalkyl group, is optionally replaced by -C(=O)-, -NR¹⁰-, or -O-;
R⁸ is hydrogen, benzyl, or triphenylmethyl;
R⁹ is selected from the group comprising C₁-C₄-alkyl, C₁-C₄-haloalkyl, and phenyl, wherein alkyl and phenyl are optionally substituted by one or multiple groups, selected independently from each other, from the group comprising C₁-C₄-alkyl, C₁-C₄-haloalkyl, C₁-C₄-alkoxy, halo, cyano, and nitro;
R¹⁰ is selected from the group comprising hydrogen, C₁-C₄-alkyl, and acetyl;
and stereoisomers, stereoisomeric mixtures, and suitable salts thereof.

Preferably, compound of formula I is defined wherein
R¹ is C(=O)OR⁶;
R² is C(=O)OR⁷;
R³ is selected from the group comprising ¹⁹F, ¹⁸F, and -OS(=O)₂R⁹;
R⁴ and R⁵ are selected independently from each other from the group comprising hydrogen, optionally substituted C₁-C₈-alkyl, and optionally substituted C₇-C₁₀-arylalkyl;
R⁶ and R⁷ are selected independently from each other from the group comprising hydrogen, optionally substituted C₁-C₈-alkyl, and optionally substituted C₇-C₁₀-arylalkyl; and R⁹ is selected from the group comprising C₁-C₄-alkyl and phenyl, wherein phenyl is optionally substituted by one or two groups, selected independently from each other, from the group comprising C₁-C₄ alkyl, C₁-C₄-alkoxy, halo, and nitro.

Scheme F: compound of formula I and 3 embodiments I-F18, I-F19 and I-LG

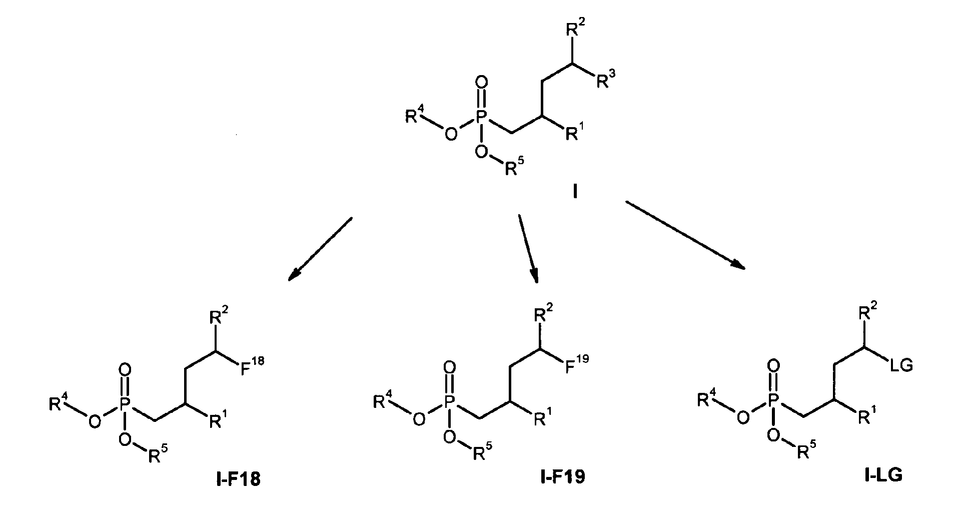

In a first embodiment, compounds of the formula I is defined such as R³ is ¹⁸F that corresponds to formula **I-F18**.

In a second embodiment, compounds of the formula I is defined such as R³ is ¹⁹F that corresponds to formula **I-F19.**

In a third embodiment, compounds of the formula I is defined such as R³ is LG that corresponds to formula **I-LG**
wherein LG means Leaving Group and is selected from the group comprising chloro, bromo, iodo, or -OS(=O)₂R⁹.

Preferably, LG is -OS(=O)₂R⁹
wherein R⁹ is selected from the group comprising C₁-C₄-alkyl, C₁-C₄-haloalkyl, and phenyl, alkyl and phenyl are optionally substituted by one or multiple groups, selected independently from each other, from the group comprising C₁-C₄-alkyl, C₁-C₄-haloalkyl, C₁-C₄-alkoxy, halo, cyano, and nitro.
Preferably, R⁹ is selected from the group comprising C₁-C₄-alkyl and phenyl wherein phenyl is optionally substituted by one or two groups, selected independently from each other, from the group comprising C₁-C₄ alkyl, C₁-C₄-alkoxy, halo, and nitro.
More preferably, LG is methanesulfonyloxy, ethanesulfonyloxy, benzenesulfonyloxy, *para-*toluenesulfonyloxy, *para*-nitrobenzenesulfonyloxy, or naphthalenesulfonyloxy.
Even more preferably, LG is *para-*toluenesulfonyloxy.

Preferred features are disclosed below and apply for the 3 embodiments corresponding to formula I-F18, I-F19 and I-LG and can be combined with each other.

Preferably, R¹ is C(=O)OR⁶
wherein R⁶ is selected from the group comprising hydrogen, optionally substituted C₁-C₈-alkyl, and optionally substituted C₇-C₁₀-arylalkyl. Preferably, R⁶ is hydrogen or methyl.

Preferably, R² is C(=O)OR⁷
wherein R⁷ is selected from the group comprising hydrogen, optionally substituted C₁-C₈-alkyl, and optionally substituted C₇-C₁₀-arylalkyl. Preferably, R⁷ is hydrogen or methyl.

Preferably, R⁴ and R⁵ are selected independently from each other from the group comprising hydrogen, optionally substituted C₁-C₈-alkyl, and optionally substituted C₇-C₁₀-arylalkyl. More preferably, R⁴ and R⁵ are hydrogen or benzyl. Even more preferably, R⁴ and R⁵ are hydrogen.

Preferably, R⁶ and R⁷ are selected independently from each other from the group comprising hydrogen, optionally substituted C₁-C₈-alkyl, and optionally substituted C₇-C₁₀-arylalkyl. More preferably, R⁶ and R⁷ are hydrogen.

Compounds of formula **I-F18** of the first embodiment are preferably defined wherein
R¹ is C(=O)OR⁶;
R² is C(=O)OR⁷;
R⁴ and R⁵ are selected independently from each other from the group comprising hydrogen, optionally substituted C₁-C₈-alkyl, and optionally substituted C₇-C₁₀-arylalkyl; and
R⁶ and R⁷ are selected independently from each other from the group comprising hydrogen, optionally substituted C₁-C₈-alkyl, and optionally substituted C₇-C₁₀-arylalkyl.

More preferably, compounds of formula **I-F18** are defined wherein
R¹ and R² are C(=O)OCH₃; and
R⁴ and R⁵ are benzyl.

More preferably, compounds of formula **I-F18** are defined wherein
R¹ and R² are carboxy; and
R⁴ and R⁵ are hydrogen.

Invention compounds of formula **I-F18** are selected from but not limited to
(*2S*,*4S*)-2-(¹⁸F)-Fluoro-4-(phosphonomethyl)pentanedioic acid and
(*2R*,*4S*)-2-(¹⁸F)-Fluoro-4-(phosphonomethyl)pentanedioic acid,
and mixtures and suitable salts thereof.

Preferably compound of formula **I-F18** is
(*2S*,*4S*)-2-(¹⁸F)-Fluoro-4-(phosphonomethyl)pentanedioic acid and suitable salts thereof.

Compounds of formula **I-F19** of the second embodiment are preferably defined wherein
R¹ is C(=O)OR⁶;
R² is C(=O)OR⁷;
R⁴ and R⁵ are selected independently from each other from the group comprising hydrogen, optionally substituted C₁-C₈-alkyl, and optionally substituted C₇-C₁₀-arylalkyl;
R⁶ and R⁷ are selected independently from each other from the group comprising hydrogen, optionally substituted C₁-C₈-alkyl, and optionally substituted C₇-C₁₀-arylalkyl.

More preferably, compounds of formula **I-F19** are defined wherein
R¹ and R² are C(=O)OCH₃; and
R⁴ and R⁵ are benzyl.

More preferably, compounds of formula **I-F19** are defined wherein
R¹ and R² are carboxy; and
R⁴ and R⁵ are hydrogen.

Invention compounds of formula **I-F19** are selected from but not limited to
(*2S*,*4S*)-2-Fluoro-4-(phosphonomethyl)pentanedioic acid and
(*2R*,*4S*)-2-Fluoro-4-(phosphonomethyl)pentanedioic acid
and mixtures thereof and suitable salts thereof.

Preferably compound of formula **I-F19** is
(*2S*,*4S*)-2-Fluoro-4-(phosphonomethyl)pentanedioic acid and suitable salts thereof.

Compounds of formula **I-LG** of the third embodiment are preferably defined wherein
R¹ is C(=O)OR⁶;
R² is C(=O)OR⁷;
R³ is -OS(=O)₂R⁹;
R⁴ and R⁵ are selected independently from each other from the group comprising optionally substituted C₁-C₈-alkyl, and optionally substituted C₇-C₁₀-arylalkyl;
R⁶ and R⁷ are selected independently from each other from the group comprising optionally substituted C₁-C₈-alkyl, and optionally substituted C₇-C₁₀-arylalkyl; and R⁹ is C₁-C₄-alkyl or phenyl, wherein phenyl is optionally substituted by one or two groups, selected independently from each other, from the group comprising C₁-C₄-alkyl, C₁-C₄-alkoxy, halo, and nitro,
and stereoisomers and mixtures thereof.

Preferably, compounds of formula **I-LG** are defined wherein
R¹ is C(=O)OR⁶;
R² is C(=O)OR⁷;
R³ is selected from the group comprising methanesulfonyloxy, ethanesulfonyloxy, benzenesulfonyloxy, *para-*toluenesulfonyloxy, *para-*nitrobenzenesulfonyloxy, and naphthalenesulfonyloxy;
R⁴ and R⁵ are selected independently from each other from the group comprising optionally substituted C₁-C₈-alkyl, and optionally substituted C₇-C₁₀-arylalkyl; and R⁶ and R⁷ are selected independently from each other from the group comprising hydrogen, optionally substituted C₁-C₈-alkyl, and optionally substituted C₇-C₁₀-arylalkyl;

More preferably, compounds of formula **I-LG** are defined wherein
R¹ is C(=O)OR⁶;
R² is C(=O)OR⁷;
R³ is *para*-toluenesulfonyloxy;
R⁴ and R⁵ are selected independently from each other from the group comprising optionally substituted C₁-C₈-alkyl, and optionally substituted C₇-C₁₀-arylalkyl; and
R⁶ and R⁷ are selected independently from each other from the group comprising, optionally substituted C₁-C₈-alkyl, and optionally substituted C₇-C₁₀-arylalkyl.

More preferably, compounds of formula **I-LG** are defined wherein
R¹ and R² are C(=O)OCH₃;
R³ is selected from the group comprising methanesulfonyloxy, ethanesulfonyloxy, benzenesulfonyloxy, *para*-toluenesulfonyloxy, *para-*nitrobenzenesulfonyloxy, and naphthalenesulfonyloxy; and
R⁴ and R⁵ are benzyl.

Even more preferably, compounds of formula **I-LG** are defined wherein
R¹ and R² are C(=O)OCH₃,
R³ is *para*-toluenesulfonyloxy; and
R⁴ and R⁵ are benzyl.

Invention compounds of formula **I-LG** are selected from but not limited to
Dimethyl (*2S*,*4S*)-2-{[bis(benzyloxy)phosphoryl]methyl}-4-(tosyloxy)pentanedioate and
Dimethyl (*2S*,*4R*)-2-{[bis(benzyloxy)phosphoryl]methyl}-4-(tosyloxy)pentanedioate
and mixtures thereof.

Preferably compound of formula **I-LG** is
Dimethyl (*2S*,*4R*)-2-{[bis(benzyloxy)phosphoryl]methyl}-4-(tosyloxy)pentanedioate.

In a **second aspect,** the invention is directed to a composition comprising compounds of formula I, I-F18, I-F19, I-LG or mixtures thereof and a pharmaceutically acceptable carrier or diluent.
The person skilled in the art is familiar with auxiliaries, vehicles, excipients, diluents, carriers or adjuvants which are suitable for the desired pharmaceutical formulations, preparations or compositions on account of his/her expert knowledge.
The administration of the compounds, pharmaceutical compositions or combinations according to the invention is performed in any of the generally accepted modes of administration available in the art. Intravenous deliveries are preferred.

Preferably, the composition comprises compounds of the general formula I-F18.

Generally, the compositions according to the invention is administered such that the dose of the active compound for imaging is in the range of 37 MBq (1 mCi) to 740 MBq (20 mCi). In particular, a dose in the range from 150 MBq to 370 MBq will be used.
In a preferred embodiment, the invention relates to a composition comprising 2-(¹⁸F)-Fluoro-4-(phosphonomethyl)pentanedioic acid, stereoisomers and mixtures thereof, and suitable salts thereof, and pharmaceutically acceptable carriers or diluents as described above.

In a more preferred embodiment, the invention relates to a composition comprising (*2R*,*4S*)-2-(¹⁸F)-Fluoro-4-(phosphonomethyl)pentanedioic acid, or (*2S*,*4S*)-2-(¹⁸F)-Fluoro-4-(phosphonomethyl)pentanedioic acid, mixtures thereof, and suitable salts thereof, and pharmaceutically acceptable carriers or diluents as described above.

In a particularly preferred embodiment, the invention relates to a composition comprising (*2S*,*4S*)-2-(¹⁸F)-Fluoro-4-(phosphonomethyl)pentanedioic acid and suitable salts thereof, and pharmaceutically acceptable carriers or diluents as described above.

In a **third aspect,** the invention is directed to methods for obtaining compounds of formula I, I-F18, I-F19, I-LG or mixtures thereof.

### Method for obtaining I-F18:

Two methods have been identified for obtaining compounds of formula I-F18:
- Direct synthesis of compounds of formula I-F18 and
- Indirect synthesis of compounds of formula I-F18.

### Direct method:

The direct method for obtaining compounds of formula I-F18 comprises the steps
- Coupling compound of Formula I-LG with a Fluorine atom (F) containing moiety wherein the Fluorine atom (F) containing moiety comprises ¹⁸F;
- Optionally deprotecting compound of formula I-F18 and/or
- Optionally converting obtained compound into a suitable salts of inorganic or organic bases thereof, hydrates, complexes, and solvates thereof.

The preferred features disclosed for compounds of general formula I-LG and I-F18 are herein incorporated.

### Indirect method:

The indirect method for obtaining compounds of formula I-F18 comprises the steps
- Coupling compound of Formula X with a Fluorine atom (F) containing moiety wherein the Fluorine atom (F) containing moiety comprises ¹⁸F for obtaining a compound of formula X-F18
   wherein 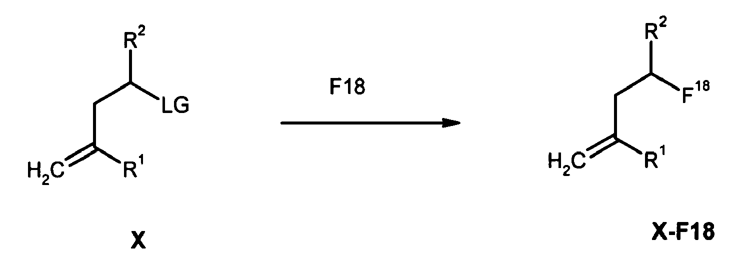 wherein
   R¹ is C(=O)OR⁶;
   R² is selected from the group comprising C(=O)OR⁷, or 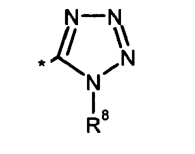 wherein the asterisk indicates the point of attachment to formula X and X-F18;
   R⁶ and R⁷ are selected independently from each other from the group comprising hydrogen, optionally substituted C₁-C₁₀-alkyl, optionally substituted C₃-C₇-cycloalkyl, optionally substituted C₆-C₁₀-aryl, or optionally substituted C₇-C₁₄-arylalkyl, wherein zero, one or two of the carbon atoms constituting said alkyl group, cycloalkyl group, or the alkyl portion of said arylalkyl group, is optionally replaced by -C(=O)-, -NR¹⁰-, or -O-;
   R⁸ is selected from the group comprising hydrogen, benzyl or triphenylmethyl;
   LG is an appropriate leaving group, selected from the group comprising chloro, bromo, iodo, and -OS(=O)₂R⁹;
   R⁹ is selected from the group comprising C₁-C₄-alkyl, C₁-C₄-haloalkyl, and phenyl, wherein alkyl and phenyl are optionally substituted by one or multiple groups, selected independently from each other, from the group comprising C₁-C₄-alkyl, C₁-C₄-haloalkyl, C₁-C₄-alkoxy, halo, cyano, and nitro;
   R¹⁰ is selected from the group comprising hydrogen, C₁-C₄-alkyl, and acetyl;
- Coupling a compound of Formula X-F18 with a compound of formula XI for obtaining a compound of formula I-F18 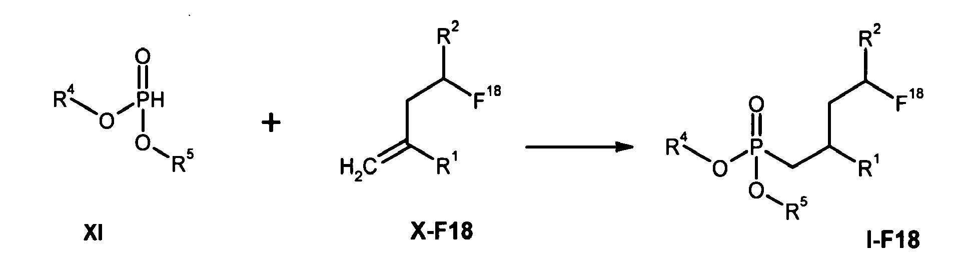 wherein
   R¹ is C(=O)OR⁶;
   R² is selected from the group comprising
   C(=O)OR⁷, or 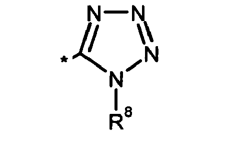 wherein the asterisk indicates the point of attachment to formula X-F18 and IF18;
   R⁴ and R⁵ are selected independently from each other from the group comprising hydrogen, optionally substituted C₁-C₁₀-alkyl, optionally substituted C₃-C₇-cycloalkyl, optionally substituted C₆-C₁₀-aryl, and optionally substituted C₇-C₁₄-arylalkyl, wherein zero, one or two of the carbon atoms constituting said alkyl group, cycloalkyl group, or the alkyl portion of said arylalkyl group, is optionally replaced by -C(=O)-, -NR¹⁰-, or -O-, or
   R⁴ and R⁵ together form an optionally substituted C₂-C₆ alkylene tether;
   R⁶ and R⁷ are selected independently from each other from the group comprising hydrogen, optionally substituted C₁-C₁₀-alkyl, optionally substituted C₃-C₇-cycloalkyl, optionally substituted C₆-C₁₀-aryl, or optionally substituted C₇-C₁₄-arylalkyl, wherein zero, one or two of the carbon atoms constituting said alkyl group, cycloalkyl group, or the alkyl portion of said arylalkyl group, is optionally replaced by -C(=O)-, -NR¹⁰-, or -O-;
   R⁸ is selected from the group comprising hydrogen, benzyl or triphenylmethyl;
   R¹⁰ is selected from the group comprising hydrogen, C₁-C₄-alkyl, and acetyl;
- Optionally deprotecting compound of formula I-F18 and/or
- Optionally converting obtained compound into a suitable salts of inorganic or organic bases thereof, hydrates, complexes, and solvates thereof.

The preferred features disclosed for compound of general formula I-LG and I-F18 are herein incorporated.

### Method for obtaining I-F19:

Two methods have been identified for obtaining compounds of formula I-F19:
- Direct synthesis of compounds of formula I-F19 and
- Indirect synthesis of compounds of formula I-F19.

### Direct method:

The direct method for obtaining compounds of formula I-F19 comprises the steps
- Reacting a compound of formula I-R11 with a Fluorine atom (F) containing moiety wherein the Fluorine atom (F) containing moiety comprises ¹⁹F;
- Optionally deprotecting compound of formula I-F19 and/or
- Optionally converting obtained compound into suitable salts of inorganic or organic bases thereof, hydrates, complexes, and solvates thereof.

The preferred features disclosed for compound of general formula I-LG and I-F19 are herein incorporated.
I-R11 is defined below.

### Indirect method:

The indirect method for obtaining compounds of formula I-F19 comprises the steps
- Reacting compound of Formula XII with a Fluorine atom (F) containing moiety wherein the Fluorine atom (F) containing moiety comprises ¹⁹F for obtaining a compound of formula X-F19
   wherein 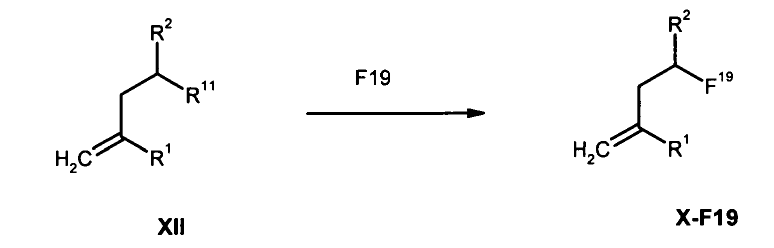 wherein
   R¹ is C(=O)OR⁶;
   R² is C(=O)OR⁷, or 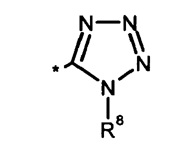 wherein the asterisk indicates the point of attachment to formula XII and X-F19;
   R⁶ and R⁷ are selected independently from each other from the group comprising hydrogen, optionally substituted C₁-C₁₀-alkyl, optionally substituted C₃-C₇-cycloalkyl, optionally substituted C₆-C₁₀-aryl, and optionally substituted C₇-C₁₄-arylalkyl, wherein zero, one or two of the carbon atoms constituting said alkyl group, cycloalkyl group, or the alkyl portion of said arylalkyl group, is optionally replaced by -C(=O)-, -NR¹⁰-, or -O-;
   R⁸ is selected from the group comprising hydrogen, benzyl, or triphenylmethyl;
   R⁹ is selected from the group comprising C₁-C₄-alkyl, C₁-C₄-haloalkyl, and phenyl, wherein alkyl and phenyl are optionally substituted by one or multiple groups, selected independently from each other, from the group comprising C₁-C₄-alkyl, C₁-C₄-haloalkyl, C₁-C₄-alkoxy, halo, cyano, and nitro;
   R¹⁰ is selected from the group comprising hydrogen, C₁-C₄-alkyl, and acetyl,;
   R¹¹ is OH or OS(=O)2R⁹,
- Coupling a compound of Formula X-F19 with a compound of formula XI for obtaining a compound of formula I-F19
   wherein 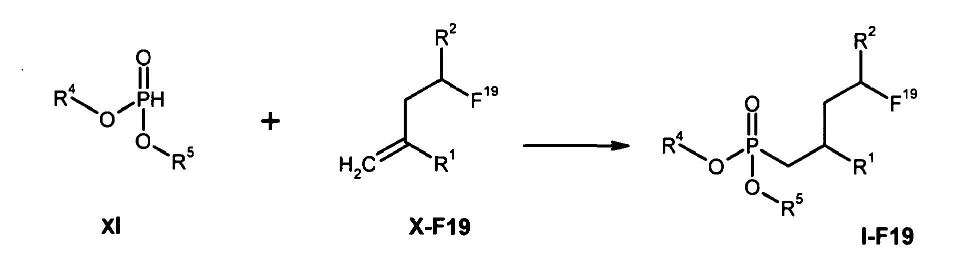 wherein
   R¹ is C(=O)OR⁶;
   R² is C(=O)OR , or 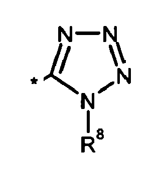 wherein the asterisk indicates the point of attachment to formula X-F19 and IF19;
   R⁴ and R⁵ are selected independently from each other from the group comprising hydrogen, optionally substituted C₁-C₁₀-alkyl, optionally substituted C₃-C₇-cycloalkyl, optionally substituted C₆-C₁₀-aryl, and optionally substituted C₇-C₁₄-arylalkyl, wherein zero, one or two of the carbon atoms constituting said alkyl group, cycloalkyl group, or the alkyl portion of said arylalkyl group, is optionally replaced by -C(=O)-, -NR¹⁰-, or -O-, or,
   R⁴ and R⁵ together form an optionally substituted C₂-C₆ alkylene tether;
   R⁶ and R⁷ are selected independently from each other from the group comprising hydrogen, optionally substituted C₁-C₁₀-alkyl, optionally substituted C₃-C₇-cycloalkyl, optionally substituted C₆-C₁₀-aryl, and optionally substituted C₇-C₁₄-arylalkyl, wherein zero, one or two of the carbon atoms constituting said alkyl group, cycloalkyl group, or the alkyl portion of said arylalkyl group, is optionally replaced by -C(=O)-, -NR¹⁰-, or -O-;
   R⁸ is selected from the group comprising hydrogen, benzyl, or triphenylmethyl;
   R¹⁰ is selected from the group comprising hydrogen, C₁-C₄-alkyl, and acetyl;
- Optionally deprotecting compound of formula I-F19 and/or
- Optionally converting obtained compound into a suitable salts of inorganic or organic bases thereof, hydrates, complexes, and solvates thereof.

Preferably, the indirect method for obtaining I-F19 is concerning compound of formula I-F19, X-F19 or XI wherein
R¹ is C(=O)OR⁶;
R² is C(=O)OR⁷;
R⁴ and R⁵ are selected independently from each other from the group comprising optionally substituted C₁-C₈-alkyl and optionally substituted C₇-C₁₀-arylalkyl; and
R⁶ and R⁷ are selected independently from each other from the group comprising optionally substituted C₁-C₈-alkyl and optionally substituted C₇-C₁₀-arylalkyl.

The preferred features disclosed for compound of general formula I-F19 are herein incorporated.

### Method for obtaining I-LG:

Two methods have been identified for obtaining compounds of formula I-LG:
- Direct synthesis of compounds of formula I-LG and
- Indirect synthesis of compounds of formula I-LG.

### Direct method:

The direct method for obtaining I-LG comprises the steps
- Coupling compound of Formula I-R12 with an agent suitable for conversion of R¹² into an LG moiety as defined *supra,* such as an appropriate sulfonyl halide, sulfonyl anhydride (for the introduction of OS-(=O)₂R⁹), or a combination of a phosphane, such as triphenyl phosphane, and a carbon tetrahalide, such as tetrabromomethane (for the introduction of chloro, bromo, and iodo). 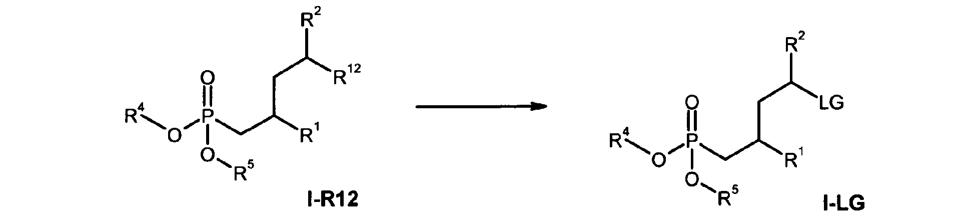 wherein
   R¹ is C(=O)OR⁶; R² is C(=O)OR⁷, or 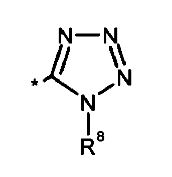 wherein the asterisk indicates the point of attachment to formula I-R12 and I-LG;
   R¹² is OH,
   LG is an appropriate leaving group, selected from the group comprising chloro, bromo,
   iodo, and -OS(=O)₂R⁹,
   R⁴ and R⁵ are selected independently from each other from the group comprising hydrogen, optionally substituted C₁-C₁₀-alkyl, optionally substituted C₃-C₇-cycloalkyl, optionally substituted C₆-C₁₀-aryl, and optionally substituted C₇-C₁₄-arylalkyl, wherein zero, one or two of the carbon atoms constituting said alkyl group, cycloalkyl group, or the alkyl portion of said arylalkyl group, is optionally replaced by -C(=O)-, -NR¹⁰-, or -O-,
   or R⁴ and R⁵ together form an optionally substituted C₂-C₆ alkylene tether;
   R⁶ and R⁷ are selected independently from each other, from the group comprising hydrogen, optionally substituted C₁-C₁₀-alkyl, optionally substituted C₃-C₇-cycloalkyl, optionally substituted C₆-C₁₀-aryl, and optionally substituted C₇-C₁₄-arylalkyl, wherein zero, one or two of the carbon atoms constituting said alkyl group, cycloalkyl group, or the alkyl portion of said arylalkyl group, is optionally replaced by -C(=O)-, -NR¹⁰-, or -O-;
   R⁸ is selected from the group comprising hydrogen, benzyl, or triphenylmethyl;
   R⁹ is selected from the group comprising C₁-C₄-alkyl, C₁-C₄-haloalkyl, and phenyl,
   wherein alkyl and phenyl are optionally substituted by one or multiple groups, selected independently from each other, from the group comprising C₁-C₄-alkyl, C₁-C₄-haloalkyl, C₁-C₄-alkoxy, halo, cyano, and nitro;
   R¹⁰ is selected from the group comprising hydrogen, C₁-C₄-alkyl, and acetyl;
   and stereoisomers, stereoisomeric mixtures, and suitable salts thereof,
- Optionally deprotecting compound of formula I-LG and/or
- Optionally converting obtained compound into a suitable salts of inorganic or organic acids thereof, hydrates, complexes, and solvates thereof.

The preferred features disclosed for R¹; R² R⁴ R⁵, R⁶ ,R⁷, R⁸ and R⁹ are herein incorporated.

### Indirect method:

The indirect method for obtaining I-LG comprises the steps
- Coupling a compound of Formula XI with a compound of formula X-LG for obtaining a compound of formula I-LG 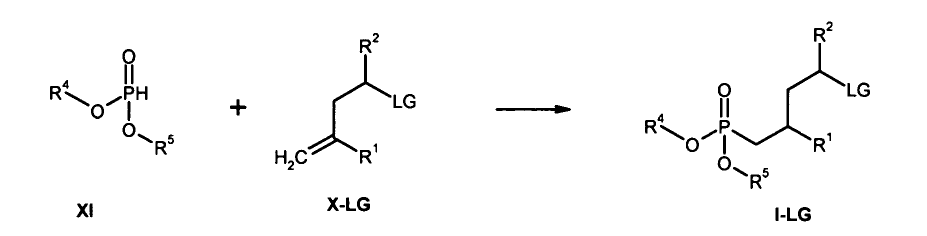 wherein
   R¹ is C(=O)OR⁶;
   R² is selected from the group comprising C(=O)OR⁷, or 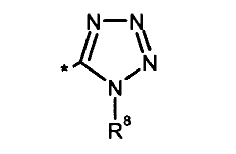 wherein the asterisk indicates the point of attachment to formula X-LG and I-LG;
   LG is an appropriate leaving group, selected from the group comprising chloro, bromo, iodo, and -OS(=O)₂R⁹;
   R⁴ and R⁵ are selected independently from each other from the group comprising hydrogen, optionally substituted C₁-C₁₀-alkyl, optionally substituted C₃-C₇-cycloalkyl, optionally substituted C₆-C₁₀-aryl, and optionally substituted C₇-C₁₄-arylalkyl, wherein zero, one or two of the carbon atoms constituting said alkyl group, cycloalkyl group, or the alkyl portion of said arylalkyl group, is optionally replaced by -C(=O)-, -NR¹⁰-, or -O- or
   R⁴ and R⁵ together form an optionally substituted C₂-C₆ alkylene tether;
   R⁶ and R⁷ are selected independently from each other, from the group comprising hydrogen, optionally substituted C₁-C₁₀-alkyl, optionally substituted C₃-C₇-cycloalkyl, optionally substituted C₆-C₁₀-aryl, and optionally substituted C₇-C₁₄-arylalkyl, wherein zero, one or two of the carbon atoms constituting said alkyl group, cycloalkyl group, or the alkyl portion of said arylalkyl group, is optionally replaced by -C(=O)-, -NR¹⁰-, or -O-;
   R⁸ is selected from the group comprising hydrogen, benzyl, or triphenylmethyl;
   R⁹ is selected from the group comprising C₁-C₄-alkyl, C₁-C₄-haloalkyl, and phenyl, wherein alkyl and phenyl are optionally substituted by one or multiple groups, selected independently from each other, from the group comprising C₁-C₄-alkyl, C₁-C₄-haloalkyl, C₁-C₄-alkoxy, halo, cyano, and nitro;
   R¹⁰ is selected from the group comprising hydrogen, C₁-C₄-alkyl, and acetyl;
- Optionally deprotecting compound of formula I-LG and/or
- Optionally converting obtained compound into a suitable salts of inorganic or organic acids thereof, hydrates, complexes, and solvates thereof.

The preferred features disclosed for compound of general formula I-LG are herein incorporated.

Preferably, the indirect method for obtaining I-LG comprises the steps
- Coupling a compound of Formula XI with a compound of formula X-LG for obtaining a compound of formula I-LG
   wherein 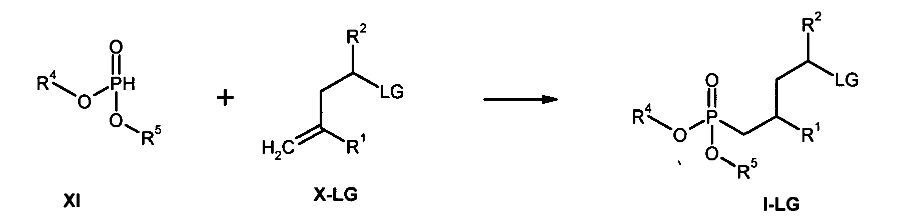 wherein
   R¹ is C(=O)OR⁶;
   R² is C(=O)OR⁷,
   LG is -OS(=O)₂R⁹,
   R⁴ and R⁵ are selected independently from each other from the group comprising optionally substituted C₁-C₈-alkyl and optionally substituted C₇-C₁₀-arylalkyl;
   R⁶ and R⁷are selected independently from each other from the group comprising optionally substituted C₁-C₈-alkyl and optionally substituted C₇-C₁₀-arylalkyl;
   R⁹ is selected from the group comprising C₁-C₄-alkyl and phenyl, wherein phenyl is optionally substituted by one or two groups, selected independently from each other, from the group comprising C₁-C₄ alkyl, C₁-C₄-alkoxy, halo, and nitro;
   Preferably, LG is methanesulfonyloxy, ethanesulfonyloxy, benzenesulfonyloxy, *para-*toluenesulfonyloxy, *para-*nitrobenzenesulfonyloxy, or naphthalenesulfonyloxy.

Said indirect method of obtaining I-LG is performed in a suitable inert solvent, in the presence of a suitable base, optionally in a microwave reactor in case the reaction is performed at an elevated temperature, a temperature between 0° C and 100°C, and at a pressure up to 5 bar.

Suitable inert solvents are exemplified by but not limited to amides such as *N,N-*dimethylformamide, *N,N*-dimethylacetamide, or *N*-methylpyrrolidinone, ethers such as tetrahydrofurane, 1,2-dimethoxyethane, or dioxane, halogenated hydrocarbons such as dichloromethane or chloroform, or others such as dimethylsulfoxide.

Suitable bases are exemplified by but not limited to alkali carbonates, such as sodium carbonate or potassium carbonate, alkali bicarbonates such as potassium bicarbonate, or organic bases such as triethylamine, *N,N*-diisopropylethylamine, pyridine, *N-*methylmorpholine, *N*-methylpiperidine, or DBU (1,8-Diazabicyclo(5.4.0)-undec-7-ene).

Preferred inert solvents are *N,N*-dimethylformamide or tetrahydrofuran.

Preferred bases are potassium carbonate, or DBU (1,8-Diazabicyclo(5.4.0)-undec-7-ene).

Preferably, the indirect method for obtaining I-LG is concerning compound of formula I-LG, X-LG or XI wherein
R¹ and R² are C(=O)OCH₃;
LG is *para*-toluenesulfonyloxy;
R⁴ and R⁵ are benzyl,
the inert solvent is tetrahydrofuran,
the base is DBU (1,8-Diazabicyclo(5.4.0)-undec-7-ene),
and the temperature range is between 0°C and 50 °C.

The Fluorine atom (F) containing moiety comprising ¹⁸F can be chelated complexes known to those skilled in the art, e.g. 4,7,13,16,21,24-Hexaoxa-1,10-diazabicyclo[8.8.8]-hexacosane K18F (crown ether salt Kryptofix K18F), 18-crown-6 ether salt K18F, K¹⁸F, H¹⁸F, KH¹⁸ F₂, Rb18F, Cs¹⁸F, Na¹⁸F, or tetraalkylammonium salts of ¹⁸F known to those skilled in the art, e.g.[F-18] tetrabutylammonium fluoride, or tetraalkylphosphonium salts of ¹⁸F known to those skilled in the art, e.g.[F-18] tetrabutylphosphonium fluoride. Most preferably, the Fluorine atom (F) containing moiety is Cs18F, K¹⁸F, H¹⁸F, or KH¹⁸F₂.

The reagents, solvents and conditions which can be used for this fluorination are common and well-known to the skilled person in the field. See, *e.g*., J. Fluorine Chem., 27 (1985):177-191. Preferably, the solvents used in the present method are DMF, DMSO, acetonitrile, DMA, or mixture thereof, preferably the solvent is DMSO.

The Fluorine atom (F) containing moiety comprising ¹⁹F is a reagent suitable for the conversion of -OH or -OS(=O)₂R⁹ into an organic ¹⁹F fluoride. Such reagents are exemplified by but not limited to inorganic salts and/or adducts of hydrofluoric acid, e.g. sodium fluoride, potassium fluoride, potassium hydrogen difluoride, or cesium fluoride as such or in combination with chelating reagents, e.g. aminopolyether 2.2.2 (K2.2.2) ; organic salts and/or adducts of hydrofluoric acid such as tetra n-butylammonium fluoride (TBAF) or triethylamine tris-hydrofluoride; hypervalent fluorosilicates, e.g. tetrabutylammonium triphenyldifluorosilicate; sulfur fluorides, e.g. (diethylamino)sulfur trifluoride (DAST); sulfonyl fluorides, e.g. 1,1,2,2,3,3,4,4,4-nonafluorobutane-1-sulfonyl fluoride; *N*-fluoropyridinium salts, e.g. N-fluoropyridinium triflate; N-fluorosulfonimides, e.g. *N*-fluorobenzenesulfonimide; aliphatic N-fluoroamines derivatives, e.g. 1-chloromethyl-4-fluoro-1,4-diazoniabicyclo[2.2.2]octane bis(tetrafluoroborate) (Selectfluor®). As known to the person skilled in the art, said reagents may be used alone or in combination, e.g. 1,1,2,2,3,3,4,4,4-nonafluorobutane-1-sulfonyl fluoride in combination with triethylamine tris-hydrofluoride. For further methods, see publications in reach of the person skilled in the art, e.g. Ritter T. et al. Current Opinion in Drug Discovery & Development 2008, 11(6), 803-819, Kirk, K.L. Organic Process Research & Development 2008, 12, 305-321, and references cited therein.

Embodiments and preferred features can be combined together and are within the scope of the invention. The preferred features disclosed for compound of general formula I, I-F18, IF19, I-LG are herein incorporated.

In a **fourth aspect**, the invention is directed to compounds of general formula I or I-F18 or mixtures thereof for the manufacture of an imaging tracer or radiopharmaceutical agent for imaging diseases associated with altered expression of Prostate Specific Membrane Antigen PSMA. Preferably, altered expression of Prostate Specific Membrane Antigen PSMA refers to elevated expression of Prostate Specific Membrane Antigen PSMA.
In other words, the invention is directed to the use of the invented compounds of general formula I and I-F18 for the manufacture of an imaging tracer for imaging diseases associated with elevated expression of Prostate Specific Membrane Antigen PSMA.

The compounds of general formula I and I-F18) are herein defined as above and encompass all embodiments and preferred features.
The imaging tracer is Positron Emission Tomography PET suitable imaging tracer.

The invention is also directed to a method for imaging or diagnosing of diseases associated with elevated expression of Prostate Specific Membrane Antigen PSMA comprising the steps:
- Administering to a mammal an effective amount of a compound comprising compounds of general formula I or I-F18,
- Obtaining images of the mammal and
- Assessing images.

The invention is directed to the use compounds of general formula I, wherein R³ is ¹⁸F-Fluoro, for the manufacture of an imaging tracer.

In a preferred embodiment, the invention is directed to the use of 2-(¹⁸F)-Fluoro-4-(phosphonomethyl)pentanedioic acid, stereoisomers and mixtures thereof, and salts thereof, for the manufacture of an imaging tracer for imaging diseases associated with elevated expression of Prostate Specific Membrane Antigen PSMA.

In an even more preferred embodiment, the invention is directed to the use of (*2R*,*4S*)-2-(¹⁸F)-Fluoro-4-(phosphonomethyl)pentanedioic acid and/or (*2S*,*4S*)-2-(¹⁸F)-Fluoro-4-(phosphonomethyl)pentanedioic, mixtures thereof, and salts thereof, for the manufacture of an imaging tracer for imaging diseases associated with elevated expression of Prostate Specific Membrane Antigen PSMA.

In a particularly preferred embodiment, the invention is directed to the use of (*2S*,*4S*)-2-(¹⁸F)-Fluoro-4-(phosphonomethyl)pentanedioic and salts thereof for the manufacture of an imaging tracer for imaging diseases associated with elevated expression of Prostate Specific Membrane Antigen PSMA.

In another preferred embodiment said diseases relate to prostate cancer and its metastases.

In a **fifth aspect,** the invention is directed to the use of compounds of general formula I, IF18 or I-F19 for conducting biological assays and chromatographic identification. More preferably, the use relates to compounds of general formula I wherein R³ is ¹⁸F or ¹⁹F, more preferably ¹⁹F.

Compounds of general formula I wherein the fluorine isotope is ¹⁹F are useful as references and/or measurement agents.

The compounds of general formula I are herein defined as above and encompass all embodiments and preferred features.

In a **sixth aspect**, the invention is directed to a method for inhibiting NAALADase activity by contacting invention compounds of formula I with proteins exhibiting NAALADase activity in-vitro or in-vivo. Additionally, the compound of invention of formula I can be coupled to a detectable label e.g. fluorescent dyes.

Preferably, the invention compound is a compound of formula I-F18 or I-F19.

In a **seventh aspect,** the present invention provides a kit comprising a sealed vial containing a predetermined quantity of a compound of Formula I, I-F18, I-F19 or I-LG, stereoisomers thereof and their mixtures, and suitable salts of inorganic or organic bases and acids thereof, hydrates, complexes, and solvates thereof. Optionally the kit comprises a pharmaceutically acceptable carrier, diluent, excipient or adjuvant.

### General synthesis of compounds of the invention

The synthesis of the compounds of the invention can be accomplished in multiple ways using synthetic methods described in the literature and databases in reach of the person skilled in the art.

As used herein, protecting groups, as well as methods to introduce and to remove them, are well known to the person skilled in the art and described the literature in ample variety, see *e.g*. T. W. Greene, P. G. M. Wuts, Protective groups in Organic Synthesis, 3rd edition, Wiley&Sons, New York 1999; P. J. Kocieński, Protecting groups, 3rd edition, Thieme, Stuttgart 2005**.**

More specifically, compounds of the invention according to the general formulae la, lb, Ic, ld, and le can be synthesised *e.g*. starting from glyoxylates II and halomethylacrylates III, preferably bromomethylacrylates, as outlined in scheme 1. Such building blocks are known to the person skilled in the art, partially available from commercial sources depending on their ester substitution, and also described in the literature. For II, see e.g. S. Coulton and I. François, J. Chem. Soc. Perkin Trans. 1, 1991, 2699; J. E. Bishop, J. F. O'Donnell, H. Rapoport, J. Org. Chem. 1991, 56, 5079; J. Våbenø, M. Brisander, T. Lejon, K. Luthmann, J. Org. Chem. 2002, 67, 9186; and for III, see e.g. J. Villieras, M. Rambeaud, Synthesis 1982, 924; B. M. O'Leary, T. Szabo, N. Svenstrup, C. A. Schalley, A. Lützen, M. Schäfer, J. Rebek, Jr., J. Am. Chem. Soc. 2001, 123, 11519. In the presence of metallic Indium, **II** and **III** form hydroxylated glutarates IV (see e.g. P. H. Lee, K. Lee., S. Chang, Synth. Comm. 2001, 31, 3189; T.-P. Loh, J.-M. Huang, K.-C. Xu, S.-H. Goh, J. J. Vittal, Tetrahedron Lett. 2000, 41, 6511), the hydroxy group of which can be protected readily to give **V**.
Intermediate **V** may also be useful to change R⁶ and/or R⁷, should protecting groups strategy warrant so to do, by methods known to the person skilled in the art. In order to illustrate but not to limit the scope of such variation of R⁶ and R⁷, reference is made *e.g*. to A. Hayen, R. Koch, W. Saak, D. Haase, J. O. Metzger, J. Am. Chem. Soc. 2000, 122, 12458. Michael addition of phosphites **VI** leads to intermediates **VII** which, upon mild deprotection, can be converted into hydroxy esters **VIII** without or with acceptable levels of lactonisation. Phosphites **VI** are well known to the person skilled in the art, often commercially available, or can be prepared according to literature protocols, see e.g. H. Goldwhite and B. C. Saunders, J. Chem. Soc. 1957, 2409. Conversion of the hydroxy group in **VIII** into a leaving group as defined in the definitions section, such as a sulfonic ester, can be accomplished using standard methods to give precursor molecules la enabling radiosynthesis with ¹⁸F fluoride. Stereoisomers of the species involved may be prepared by chiral HPLC separation.

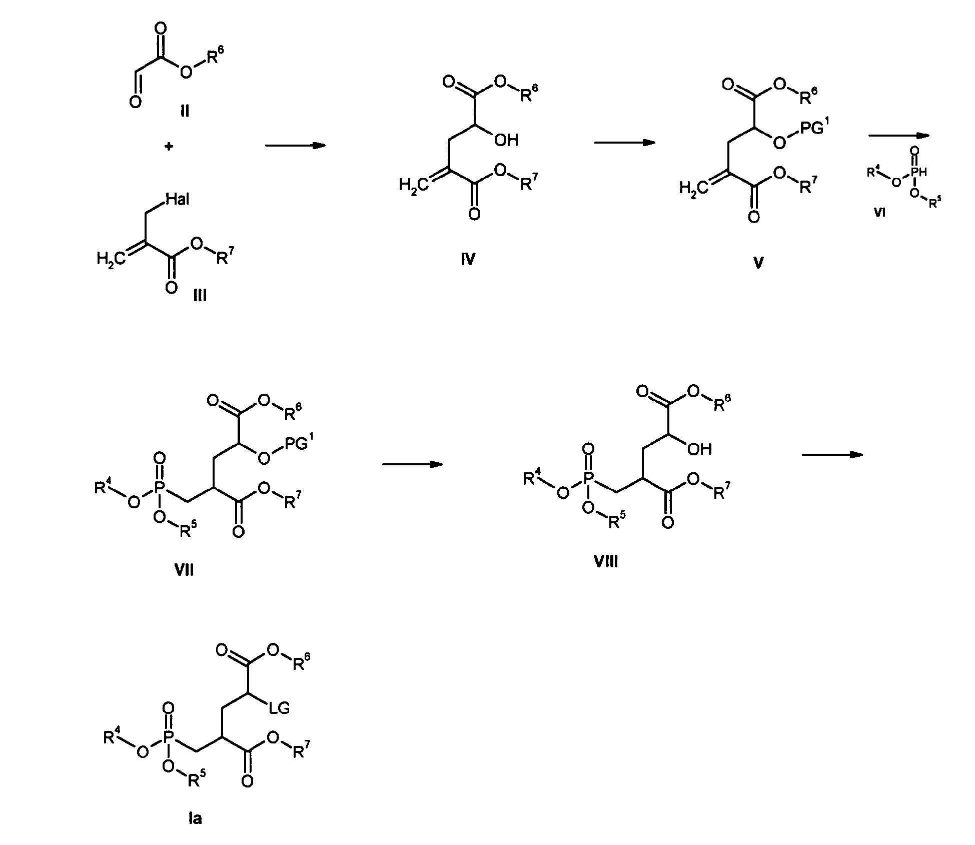

### Scheme 1: Preparation of precursors Ia from glyoxylates II and acrylates III, wherein Hal is halogen, preferably bromo, and wherein R⁴,R⁵, R⁶, R⁷, and LG, are defined as in the claims and description of this invention, and wherein PG¹ is a suitable hydroxy protecting group known to the person skilled in the art and as described in the literature cited herein.

Under suitable reaction conditions it is also possible to approach compounds **Ia** in a more concise way by transferring the hydroxy group in **IV** into a suitable leaving group as in **Va** and to directly convert **Va** into **Ia** by means of Michael addition of phosphites **VI,** as shown in scheme 2. Surprisingly, DBU, which is widely known as a reagent suitable for the induction of elimination reactions, has been identified as a particularly suitable basic reagent to catalyse additions of phosphites **VI** to glutarates of the formula **Va.**

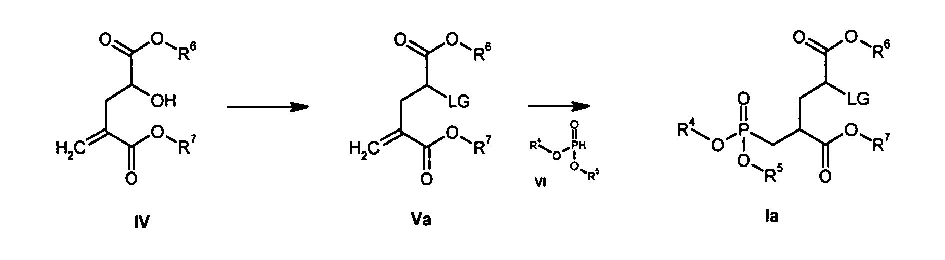

### Scheme 2: Preparation of precursors Ia from intermediates IV, wherein R⁴, R⁵, R⁶, R⁷, and LG, are defined as in the claims and description of this invention.

The radiosynthesis of the ¹⁸F labelled compounds of the invention can be accomplished in multiple ways using known methods described in the literature and databases in reach of the person skilled in the art.

More specifically, compounds of the invention according to the general formulae **Ib** and **Ic** can be synthesised starting from **Ia** as outlined in scheme 3. Such nucleophilic fluorinations are known to the person skilled in the art and also described in the literature, for reviews and cited references within see *e.g*. Cai et al., Eur. J. Org. Chem., 2008, 2853; Ametamey et al., Chem. Rev., 2008, 108, 1501, Miller et al., Angew. Chem. Int. Ed. 2008, 47, 8998.

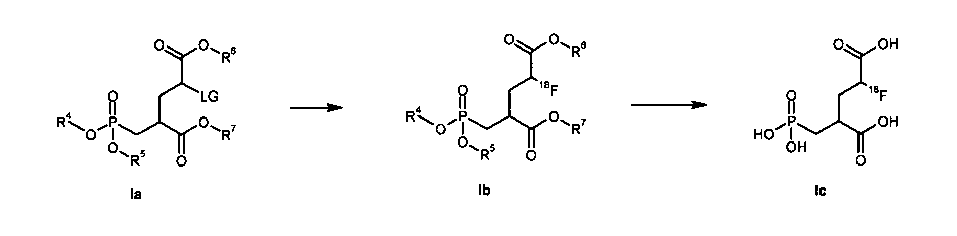

### Scheme 3: Preparation of ¹⁸F labelled compounds of the invention Ib and Ic from precursors Ia, wherein R⁴, R⁵, R⁶, R⁷, and LG, are defined as in the claims and description of this invention.

In a similar way, precursor molecules **Ia** can be converted into ¹⁹F fluorinated intermediates **Id** as shown in scheme 4 by nucleophilic fluorination with subsequential removal of protecting groups in one or more steps eventually giving rise to ¹⁹F compounds **Ie.**

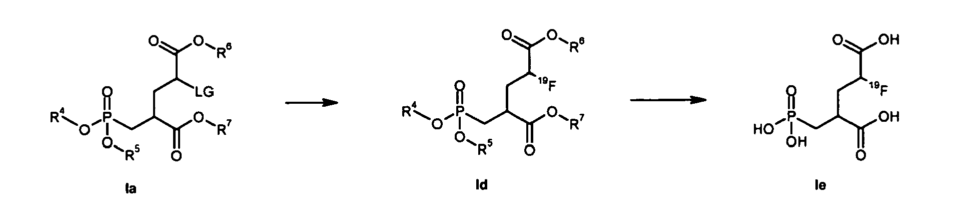

### Scheme 4: Preparation of ¹⁹F substituted compounds of the invention Id and Ie from precursors Ia, wherein R⁴, R⁵, R⁶, R⁷, and LG, are defined as in the claims and description of this invention.

Alternatively, ¹⁹F substituted compounds of the invention according to the general formulae **Id** and **Ie** can be approached starting from intermediates **IV** by fluorination of the hydroxy group by methods known to the person skilled in the art to give ¹⁹F fluorides **IX.** Such intermediates can be directly converted into intermediates **Id** by Michael addition of phosphites **VI** under mild basic conditions without concomitant elimination of hydrogen fluoride. Deprotection as described above can be employed to effect conversion into **Ie.** Again, reference is made to the possibility to separate stereoisomers by means of chiral HPLC.

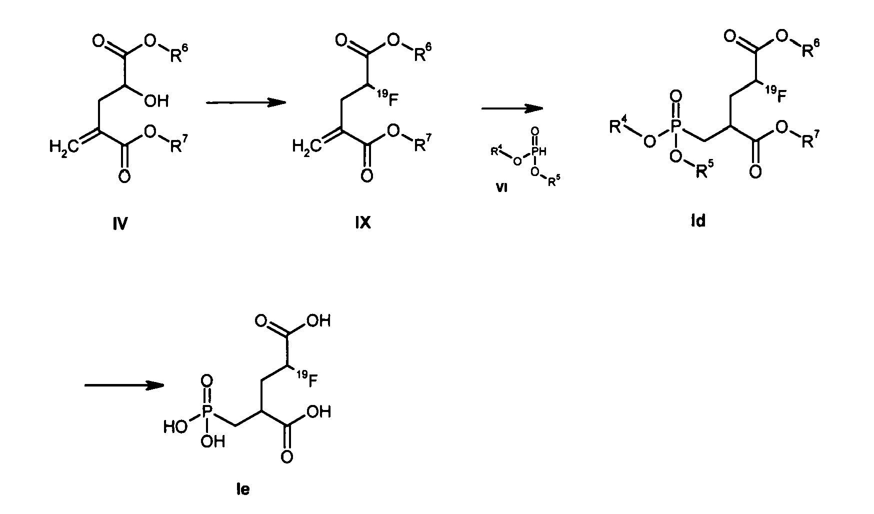

### Scheme 5: Preparation of ¹⁹F substituted compounds of the invention Id and Ie from intermediates IV, wherein R⁴, R⁵, R⁶, and R⁷, are defined as in the claims and description of this invention.

The tetrazole moieties can be built up by [2+3]-cycloaddition reaction of the corresponding nitrile with an azide (e.g. M. E. Safdy et al., J. Med. Chem. 1982, 25, 723). If not already present in the respective starting materials, the corresponding nitriles can be synthesized by dehydration of the corresponding primary amides with dehydrating agent like POCl₃ (S. E. Webber et al., J. Med. Chem. 1998, 41, 2786) or trifluoroacetic acid anhydride (e.g. K. S. Sarma et al. in Proceedings of the 4th Int. Peptide Symposium 2007), or by nucleophilic substitution of a suitable leaving group like halide or sulfonate by a cyanide (e.g. A. V. Kelin et al., J. Am. Chem. Soc. 2001, 123(9), 2074). Alternatively, primary amides can be directly converted into azides by reacting with azide derivatives such as trimethylsilyl azide in the presence of diazodicarboxylates, such as diisopropyl diazodicarboxylate (DIAD), and suitable phosphanes, such as triphenyl phosphane, as described e.g. by A. P. Kozikowski, J. Zhang, F. Nan, P. A. Petukhov, E. Grajkowska, J. T. Wroblewski, T. Yamamoto, T. Bzdega, B. Wroblewska, J. H. Neale, J. Med. Chem. 2004, 47, 1729.

### Definitions

The terms used in the present invention are defined below but are not limiting the invention scope.

As used herein, the term "alkyl" refers to a C₁-C₁₀ straight chain or branched chain alkyl group such as, for example methyl, ethyl, propyl, isopropyl, butyl, isobutyl, *tert-*butyl, pentyl, isopentyl, neopentyl, heptyl, hexyl, decyl. Preferably, alkyl is C₁-C₆ straight chain or branched chain alkyl or C₇-C₁₀ straight chain or branched chain alkyl.

As used herein, the term "cycloalkyl", refers to a C₃-C₇ cyclic alkyl group such as, for example, cyclopropyl, cyclobutyl, cyclopentyl, cyclohexyl, cycloheptyl.

As used herein, the term "alkoxy" refers to alkyl groups respectively linked to the respective scaffold by an oxygen atom, i.e. -O-, with the alkyl portion being as defined above, such as for example methoxy, ethoxy, isopropoxy, *tert-*butoxy, hexyloxy.

As used herein, the term "alkylsulfanyl" refers to alkyl groups respectively linked to the respective scaffold by a sulfur atom, i.e. -S-, with the alkyl portion being as defined above, such as for example methanesulfanyl, ethylsulfanyl, butylsulfanyl

As used herein, the term "alkylcarbonyl" refers to alkyl groups respectively linked to the respective scaffold by a carbonyl group, i.e. -C(=O)-, with the alkyl portion being as defined above, such as for example acetyl, propionyl, pivaloyl, butyryl.

As used herein, the term "alkylsulfonyl" refers to alkyl groups respectively linked to the respective scaffold by a sulfonyl group, i.e. -S(=O)₂-, with the alkyl portion being as defined above, such as for example methanesulfonyl, ethylsulfonyl, butylsulfonyl.

As used herein, the term "haloalkyl" refers to alkyl groups substituted with at least one or multiple halogen atom, fluoro, chloro, bromo, and/or iodo, selected independently from each other with the alkyl portion being as defined above, such as for example trifluoromethyl, bromomethyl, chloroethyl, 4-iodo-3-fluorobutyl, or nonafluorobutyl.

As used herein, the term "arylalkyl" refers to C₇-C₁₄ alkyl groups substituted with one or two aryl groups which are optionally substituted as listed below, with the alkyl portion being as defined above, such as for example benzyl, phenethyl, 2-methyl-3-phenylpropyl, 2-naphthylethyl, or *para-*methoxyphenylbutyl.

The term "aryl" as employed herein by itself or as part of another group refers to monocyclic or bicyclic aromatic groups containing from 6 to 12 carbons in the ring portion, preferably 6-10 carbons in the ring portion, such as phenyl, naphthyl or tetrahydronaphthyl.

The term "halo" refers to fluoro, chloro, bromo, and iodo.

Whenever the term "substituted" is used, it is meant to indicate that one or more hydrogens on the atom indicated in the expression using "substituted" is *l* are replaced by one ore multiple moieties from the group comprising halogen, hydroxyl, nitro, C₁-C₆-alkylcarbonyl, cyano, trifluoromethyl, C₁-C₆-alkylsulfonyl, C₁-C₆-alkyl, C₁-C₆-alkoxy and C₁-C₆-alkylsulfanyl, provided that the regular valency of the respective atom is not exceeded, and that the substitution results in a chemically stable compound, i. e. a compound that is sufficiently robust to survive isolation to a useful degree of purity from a reaction mixture, and formulation into a pharmaceutical composition.

As used herein, Cₙ-Cₘ indicates the range of number of carbon atoms the respective moiety may feature, illustrated by but not limited to e.g. C₁-C₆-alkyl or C₁-C₆ alkoxy, which may feature 1, 2, 3, 4, 5, or 6 carbon atoms not covering optional additional substitution.

If chiral centres or other forms of isomeric centres are not otherwise defined in a compound according to the present invention, all forms of such stereoisomers, including enantiomers and diastereoisomers, are intended to be covered herein. Compounds containing chiral centres may be used as racemic mixture or as an enantiomerically enriched mixture or as a diastereomeric mixture or as a diastereomerically enriched mixture, or these isomeric mixtures may be separated using well-known techniques, and an individual stereoisomer maybe used alone. In cases wherein compounds may exist in tautomeric forms as it is the case e.g. in tetrazole derivatives, each tautomeric form is contemplated as being included within this invention whether existing in equilibrium or predominantly in one form.

Suitable salts of the compounds according to the invention include salts of mineral acids, carboxylic acids and sulfonic acids, for example salts of hydrochloric acid, hydrobromic acid, sulfuric acid, phosphoric acid, methanesulfonic acid, ethanesulfonic acid, toluenesulfonic acid, benzenesulfonic acid, naphthalene disulfonic acid, acetic acid, trifluoroacetic acid, propionic acid, lactic acid, tartaric acid, malic acid, citric acid, fumaric acid, maleic acid and benzoic acid.

Suitable salts of the compounds according to the invention also include salts of customary bases, such as, by way of example and by way of preference, alkali metal salts (for example sodium salts and potassium salts), alkaline earth metal salts (for example calcium salts and magnesium salts) and ammonium salts, derived from ammonia or organic amines having 1 to 16 carbon atoms, such as, by way of example and by way of preference, ethylamine, diethylamine, triethylamine, ethyldiisopropylamine, monoethanolamine, diethanolamine, triethanolamine, dicyclohexylamine, dimethylaminoethanol, procaine, dibenzylamine, N-methylmorpholine, arginine, lysine, ethylenediamine and N-methylpiperidine.

"NAALADase activity" refers to an enzymatic activity that catabolizes N-acetyl-aspartyl-glutamate (NAAG) to N-acetylaspartate (NAA) and glutamate.

The term "detectable label" as used here includes fluorescent labels such as but not limited to Alexa Fluor dyes, BODIPY dyes, fluorescein-based dyes, rhodamine-based dyes, coumarin-based dyes, and pyrene based dyes, or one half of a specific binding pair, e.g. biotin of the biotin streptavidin binding pair, including biotin, oligonucleotides of DNA or RNA, or lipids, or radioisotopes such as but not limited to 3H, 11C, 14C, 18F, 32P, 33P, 35mCl, 76Br, 77Br, 123I, 124I, 125I, 131I, or chelating structures (such as but not limited to DOTA, DTPA, CHX-A", PCTA, or DO3A) able to bind radioisotopes such as but not limited to 111In, 68Ga, 67Ga, 177Lu, 86Y, 94mTc, 99mTc, 186Re, 188Re or MRI contrast agents such as but not limited to Gadolinium.

### Description of the Figures

Figure 1: PET Image 110-130 min after injection of ∼10 MBq of Example F15a in LNCaP tumor bearing mouse.
Figure 2: PET Image 110-130 min after injection of ∼10 MBq of Example F15a and 22 µg Example F3 in LNCaP tumor bearing mouse.
Figure 3: Example F13 PET Image 50-70 min after injection of ∼10 MBq of in LNCaP tumor bearing mouse.
Figure 4: Example F14 PET Image 50-70 min after injection of ∼10 MBq in LNCaP tumor bearing mouse.
Figure 5. NOE HNMR spectrum of the lactone derived from Intermediate D8
Figure 6: X-ray plot of Example F9a: (2*R*,4*R*)-2-Fluoro-4-(phosphonomethyl)pentanedioic acid
Figure 7: X-ray plot of Example F12; Dimethyl-(2*S*,4*S*)-2-Fluoro-4-(phosphonomethyl)pentanedioate

### Experimental Section

### Abbreviations

| | |
|---|---|
| 18-c-6 | 1,4,7,10,13,16-hexaoxacyclooctadecane |
| br | Broad signal (in NMR data) |
| CI | Chemical ionisation |
| d | Doublet |
| DAD | Diode array detector |
| dd | Doublet of doublet |
| ddd | Doublet of doublet of doublet |
| dt | Doublet of triplet |
| DMF | *N*,*N-*Dimethylformamide |
| DMSO | Dimethylsulfoxide |
| EI | Electron ionisation |
| ELSD | Evaporative light scattering detector |
| ESI | Electrospray ionisation |
| EtOAc | Ethyl acetate |
| Fmoc | Fluorenylmethyloxycarbonyl |
| HPLC | High pressure liquid chromatography |
| GBq | Giga Bequerel |
| K_{2.2.2} | 4, 7, 13, 16, 21, 24-hexaoxa-1,10-diazabicyclo[8.8.8]-hexacosane |
| MBq | Mega Bequerel |
| MS | Mass spectrometry |
| MTB | Methyl *tert-*butyl ether |
| m | Multiplet |
| mc | Centred multiplet |
| NMR | Nuclear magnetic resonance spectroscopy : chemical shifts (6) are given in ppm. |
| q | Quadruplett (quartet) |
| PMB | *para-*Methoxybenzyl |
| RT | Room temperature |
| s | Singlet |
| t | Triplet |
| TBS | *tert-*Butyldimethyl silyl |
| THF | Tetrahydrofuran |
| THP | Tetrahydropyran |
| UPLC | Ultra performance liquid chromatography |

**General:** All solvents and chemicals were obtained from commercial sources and used without further purification. Anhydrous solvents and inert atmosphere (nitrogen or argon) were used if not stated otherwise. The preceding table lists the abbreviations used in this paragraph and in the Intermediates and Examples sections as far as they are not explained within the text body. NMR peak forms are stated as they appear in the spectra, possible higher order effects have not been considered.

Reactions employing microwave irradiation can be run with a commercial laboratory microwave oven for organic syntheses, such as the Biotage Initiator® microwave, optionally equipped with a robotic unit. Reactions were monitored by methods known to the person skilled in the art, such as thin-layer chromatography on suitable stationary phases, such as silica gel coated plates of aluminium or glass, or HPLC/MS analyses *e.g*. according to HPLC method A1.

The compounds and intermediates produced according to the methods of the invention may require purification. Purification of organic compounds is well known to the person skilled in the art and there may be several ways of purifying the same compound. In some cases, no purification may be necessary. In certain cases, the compounds may be purified by crystallization. In some cases, impurities may be removed by trituration using a suitable solvent. In some cases, the compounds may be purified by column chromatography, or preparative HPLC according to the preparative HPLC methods listed below.

Column chromatography, as used hereinafter, typically refers to preparative liquid chromatography on a suitable stationary phase, such as commercial silica gel or prepacked silica gel cartridges, *e.g*. from Separtis such as Isolute® Flash silica gel or Isolute® Flash NH₂ silica gel in combination with *e.g*. an automated column chromatography system, and eluents such as gradients of hexane/EtOAc or dichloromethane/ethanol. Said automated chromatography systems are known to the person skilled in the art and are commercially available (*e.g*. FlashMaster II® by Argonaut/Biotage, SP4® by Biotage, Isolera Four® by Biotage, ISCO Companion®, and the likes).

### HPLC methods

### Method A1

SYSTEM: Waters Acquity UPLC-MS: Binary Solvent Manager, Sample Manager/Organizer,
Column Manager, PDA, ELSD, SQD 3001
COLUMN: Acquity UPLC BEH C18 1.7 50x2.1mm
SOLVENT: A = H₂O + 0.1% HCOOH; B = acetonitrile
GRADIENT: 0-1.6 min 1-99% B, 1.6-2.0 min 99% B
FLOW: 0.8 mL/min
TEMPERATURE: 60 °C
DETECTION: DAD scan range 210-400 nm, MS ESI+, ESI-, scan range 160-1000 m/z,
ELSD

### Method A2

SYSTEM: Dionex: Pump 680, ASI 100, Knauer: UV-Detektor K-2501 COLUMN: Chiralpak IA 5µm 150x4.6 mm

SOLVENT: Hexane / Ethanol 90:10 (isocratic)
FLOW: 1.0 mL/min
TEMPERATURE: 25°C
DETECTION: UV 210 nm

### Method A3

SYSTEM: Dionex: Pump 680, ASI 100, Knauer: UV-Detektor K-2501
COLUMN: Chiralpak IC 5µm 150x4.6 mm

SOLVENT: Hexane / 2-Propanol 50:50 (isocratic)
FLOW: 1.0 mL/min
TEMPERATURE: 25 °C DETECTION: UV 210 nm

### Method A4

SYSTEM: Dionex: Pump 680, ASI 100, Knauer: UV-Detektor K-2501
COLUMN: Chiralcel OD-H 5µm 150x4.6 mm
SOLVENT: Hexane / 2-Propanol 80:20 (isocratic)
FLOW: 1.0 mL/min
TEMPERATURE: 25 °C
DETECTION: UV 210 nm

### Method A5

SYSTEM: Dionex: Pump 680, ASI 100, Knauer: UV-Detektor K-2501
COLUMN: Chiralpak IC 5µm 150x4.6 mm

SOLVENT: Hexane / 2-Propanol 80:20 (isocratic)
FLOW: 1.0 mL/min
TEMPERATURE: 25 °C
DETECTION: UV 210 nm

### Method A6

SYSTEM: Dionex: Pump 680, ASI 100, Knauer: UV-Detektor K-2501
COLUMN: Chiralcel OD-H 5µm 150x4.6 mm
SOLVENT: Hexane / Ethanol 85:15 (isocratic)
FLOW: 1.0 mL/min
TEMPERATURE: 25°C
DETECTION: UV 210 nm

### Method A7

SYSTEM: Dionex: Pump 680, ASI 100, Knauer: UV-Detektor K-2501
COLUMN: Chiralpak IA 5µm 150x4.6 mm
SOLVENT: Methanol / Ethanol 50:50 (isocratic)
FLOW: 1.0 mL/min
TEMPERATURE: 25°C
DETECTION: UV 210 nm

### Method P1

SYSTEM: Agilent: Prep 1200, 2xPrep Pump, DLA, MWD, Prep FC, ESA: Corona
COLUMN: Chiralpak IA 5µm 250x30 mm
SOLVENT: Hexane / Ethanol 90:10 (isocratic)
FLOW: 50 mL/min
TEMPERATURE: room temperature
DETECTION: UV 210 nm

### Method P2

SYSTEM: Dionex: Pump P 580, Gilson: Liquid Handler 215, Knauer: UV-Detektor K-2501
COLUMN: Chiralpak IC 5µm 250x30 mm
SOLVENT: Hexane / 2-Propanol 50:50 (isocratic)
FLOW: 30 mL/min
TEMPERATURE: room temperature
DETECTION: UV 210 nm

### Method P3

SYSTEM: Dionex: Pump P 580, Gilson: Liquid Handler 215, Knauer: UV-Detektor K-2501
COLUMN: Chiralcel OD-H 5µm 250x20 mm
SOLVENT: Hexane / 2-Propanol 80:20 (isocratic)
FLOW: 20 mL/min
TEMPERATURE: room temperature
DETECTION: UV 210 nm

### Method P4

SYSTEM: Dionex: Pump P 580, Gilson: Liquid Handler 215, Knauer: UV-Detektor K-2501
COLUMN: Chiralpak IC 5µm 250x30 mm
SOLVENT: Hexane / 2-Propanol 80:20 (isocratic)
FLOW: 40 mL/min
TEMPERATURE: room temperature
DETECTION: UV 210 nm

### Method P5

SYSTEM: Agilent: Prep 1200, 2xPrep Pump, DLA, MWD, Prep FC, ESA: Corona
COLUMN: Chiralcel OD-H 5µm 250x20 mm

SOLVENT: Hexane / Ethanol 85:15 (isocratic)
FLOW: 20 mL/min
TEMPERATURE: room temperature
DETECTION: UV 210 nm

### Method P6

SYSTEM: Dionex: Pump P 580, Gilson: Liquid Handler 215, Knauer: UV-Detektor K-2501
COLUMN: Chiralcel OD-H 5µm 250x20 mm
SOLVENT: Hexane / Ethanol 85:15 (isocratic)
FLOW: 20 mL/min
TEMPERATURE: room temperature DETECTION: UV 210 nm

### Method P7

SYSTEM: Agilent: Prep 1200, 2xPrep Pump, DLA, MWD, Prep FC, ESA: Corona
COLUMN: Chiralpak AD-H 5µm 250x20 mm
SOLVENT: Hexan / 2-Propanol 50:50
FLOW: 15 mL/min
TEMPERATURE: room temperature
DETECTION: UV 210 nm

### Method P8

SYSTEM: Waters Autopurification system: Pump 254, Sample Manager 2767, CFO,
DAD 2996, ELSD 2424, SQD 3001
COLUMN: Atlantis C18 5µm 150x19 mm
SOLVENT: A = H₂O + 0.1 % formic acid; B: acetonitrile
GRADIENT: 0-1 min 0% B, 1-7.5 min 0-25% B, 7.5-7.6 min 25-100% B, 7.6-10 min 100% B FLOW: 25 mL/min
TEMPERATURE: room temperature
DETECTION: DAD scan range 210-400 nm, MS ESI+, ESI-, scan range 160-1000 m/z,
ELSD

### Assignment of Stereochemistry

**General remarks and methods of assignment.** The assignment of absolute configurations was deduced based on X-ray analyses of crystals grown from example compounds F9a and F12, and a nuclear Overhauser effect experiment on lactones derived from Intermediates D7 and D8.

*Numbering:* Identical compounds which have been approached *via* a different synthetic route and/or from which different points of evidence regarding absolute stereochemistry and/or biological activity have been generated, are referred to in the Experimental Section as Intermediates/Example compounds *e.g*. A1, A1a, A1b, and so forth. This numbering has been chosen to ensure maximum clarity regarding the generation of evidence on absolute stereochemistry.

To correlate the examples and intermediates not mentioned above, and to assign absolute configurations to these, the following methods listed below were used.
*i. HPLC retention time* correlation using specified HPLC methods, in particular chiral HPLC methods, as listed in the preceding paragraph.
*ii. HNMR data:* Most of the compounds and intermediates feature two stereogenic centres. Hence, these compounds exist in four stereoisomeric forms, more specifically they form two diastereomers as pairs of enantiomers. As known to the person skilled in the art, the enantiomers can not be distinguished in an achiral environment and hence give identical NMR spectra (within limits of technical variability, such as multiplet breadth in dependence of the acquisition frequency, such variability of otherwise identical spectra will be referred to as NMR equivalency herein). Thus, identity of NMR spectra of two stereoisomers proves an enantiomeric relationship, whilst different NMR spectra evidence diastereomeric relationship. 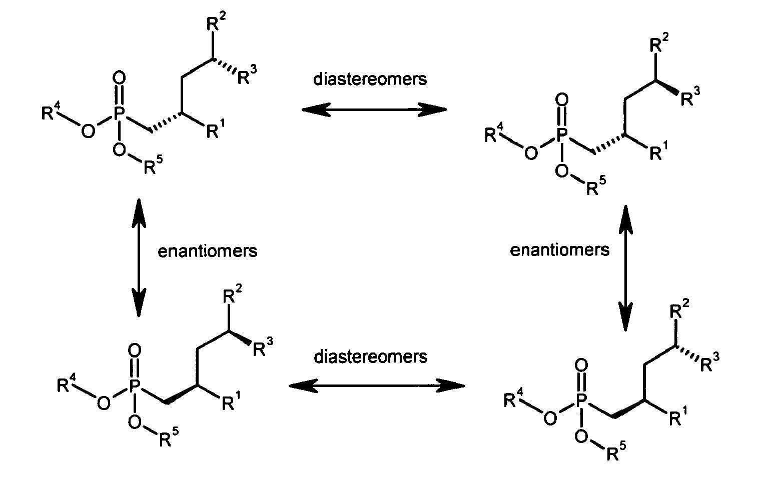
*iii. Correlation via stereocentres featuring common absolute configuration:* The addition of a phosphite to α-methylene glutarate derivatives is a key step in the syntheses of the examples of the invention, such as in the synthesis of Intermediates D5 and D6 or, as exemplarily shown below, of the Example compounds F6a and F7a. Said α-methylene glutarate derivatives can be readily prepared in enantiomerically pure form by preparative chiral HPLC, as described *e.g*. for the Intermediates D1 and D2, and E2 and E3, respectively. Their reaction with phosphites (after OH protection in case of D1 and D2) leads to two but not four stereoisomers hence it is concluded the configuration of the stereogenic centre present in the glutarate synthon is not compromised in the addition reaction, and that the example compounds F6a and F7a must have common absolute configurations at the respective stereocentre, *i.e*. C-4. The same argument applies *e.g*. to the synthesis of example compounds P7, P8, P9 and P10 from Intermediates D1 and D2.

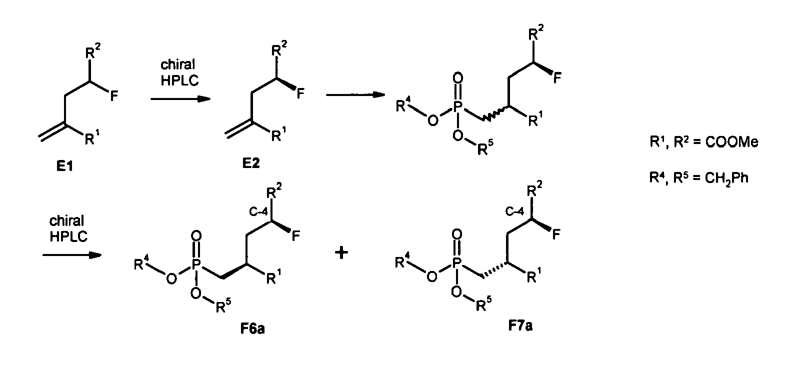

**Specific assignments.** X-ray data taken from crystals of Example F12 indicate (2*S*,4*S*) configuration which then in conclusion also applies to example F6a, from which it is formed by hydrogenolysis. Chiral HPLC retention correlation and HNMR equivalence prove the identity of Example F6a with example compound F6. F6, in turn, has been deprotected to give F10 which has shown a IC₅₀ = 7.0 nM as specified in Biology example 1. For assignments to be discussed further below it is noteworthy that Example compound F6 has been directly prepared from Example compound P9. From Example compound P9 also the ¹⁸F-Fluoro example compound F13 has been prepared, from which, in turn, Biology examples 3, 5, 6 and 7 have been generated.

Furthermore, the assignment made is corroborated by additional X-ray data of example compound F9a, which has been generated by deprotection of example compound F5a. X-ray data of Example compound F9a evidence (2*R*,4*R*) configuration. Identity of the NMR data of examples F5a and F6a, but different HPLC retention times using the same HPLC method indicate an enantiomeric relationship between two said examples. This is also in line with the fact that they have been prepared from different fluoroglutarate enantiomers Intermediate E3 (example compound F5a), and Intermediate E2 (example compound F6a), respectively, indicating opposite configuration at C-4. The sketch below summarises the assignments made and the evidence supporting these:

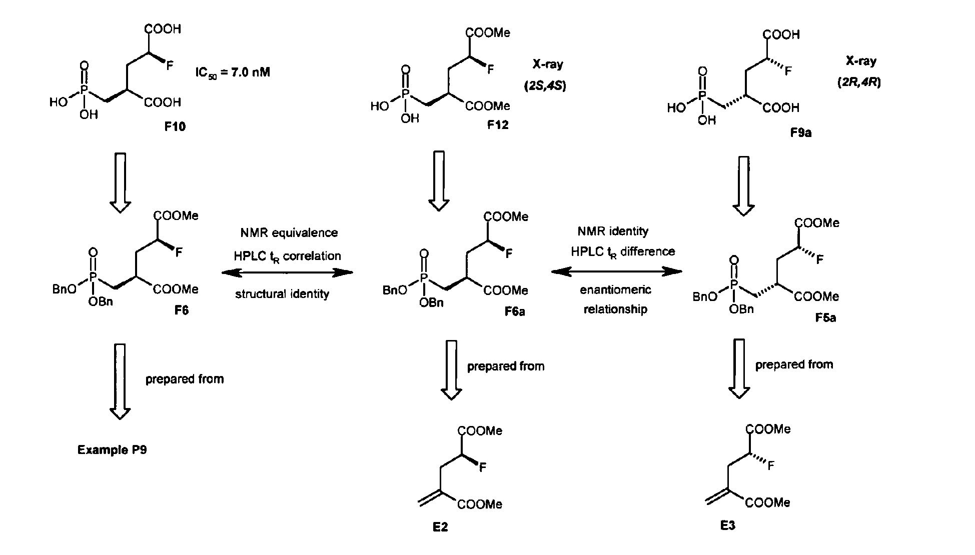

Example compound P9, from which a substantial proportion of biological data disclosed herein has been generated, has been shown to correspond to Intermediate D9 with regard to its absolute configuration by synthetic conversion of D9 into P9. Intermediate D9 was desilylated and tosylated as described in the protocols section to give a sample identical with example compound P9 as judged by NMR spectrum and chiral HPLC retention time correlation.

Two stereoisomers of Intermediate D9, Intermediates D7 and D8, were employed for further investigations of their relative configurations. Of these, D7 features an identical NMR spectrum and a different HPLC retention time as compared to D9, and has been prepared from enantiomer D1 of the respective hydroxyglutarate synthon whilst D9 was prepared from D2, thus proving D7 but not D8 as the enantiomer of D9. Furthermore, Intermediates D7 and D8 must share identical configuration at C-4 since they both were prepared from Intermediate D1.

Intermediates D7 and D8 were converted into corresponding lactones as described in the protocols section and were investigated by NMR including studies on a nuclear Overhauser effect (NOE) between the protons indicated in the sketch below. Upon irradiation of the proton at C-2 in the lactone formed from Intermediate D8, a pronounced NOE effect was found for the proton attached to C-4, indicating that the two methine protons attached to the lactone ring are located on the same side of the ring, *i.e*. feature a cis configuration. Since no NOE between the protons attached to C-2 and C-4 was found for the lactone derived from Intermediate D7 it was concluded that this lactone features *trans* configuration. Eventually, as D7 and D9 are enantiomers and hence display identical relative configurations, it was concluded that D9 - and P9 alike - must have either (*2R,4S*) or (2*S*,4*R*) configuration. However, since P9 has been directly converted into example F6 for which (2*S*,4*S*) configuration has been shown as described above and only C-4 gets involved in the conversion from Example P9 into F6, P9, and hence also Intermediate D9, must have (2*S*,4*R*) configuration.

This is in line with an S_{N}2 mechanism applying for the fluoride displacement of the tosylate leaving group which would be expected by the person skilled in the art, see *e.g*. Liu, P.; Sharon, A.; Chu, C. K. J. Fluorine Chem. 2008, 129, 743-766, Katzenellenbogen, J. A. J. Fluorine Chem. 2001, 109, 49-54, Fritz-Langhals, E. Tetrahedron Asymmetry 1994, 5, 981-986, Fritz-Langhals, E.; Schütz, G. Tetrahedron. Lett. 1993, 34, 293-296, Colonna, S. Gelbard, G.; Cesarotti, E. J. Chem. Soc., Perkin Trans. 1, 1979, 2248 - 2252, European Patent EP 0749954.

Furthermore, the results correspond to the known stereospecificity of the bioactivity of 2-PMPA, the non-fluorinated analogue of 2-fluoro-4-(phosphonomethyl)pentadioic acid (D. Vitharana et al., Tetrahedron Asymmetry 2002, 13, 1609; T. Tsukamoto at al., J. Med. Chem. 2005, 48, 2319). The sketch below summarises the assignments made and the evidence supporting these:

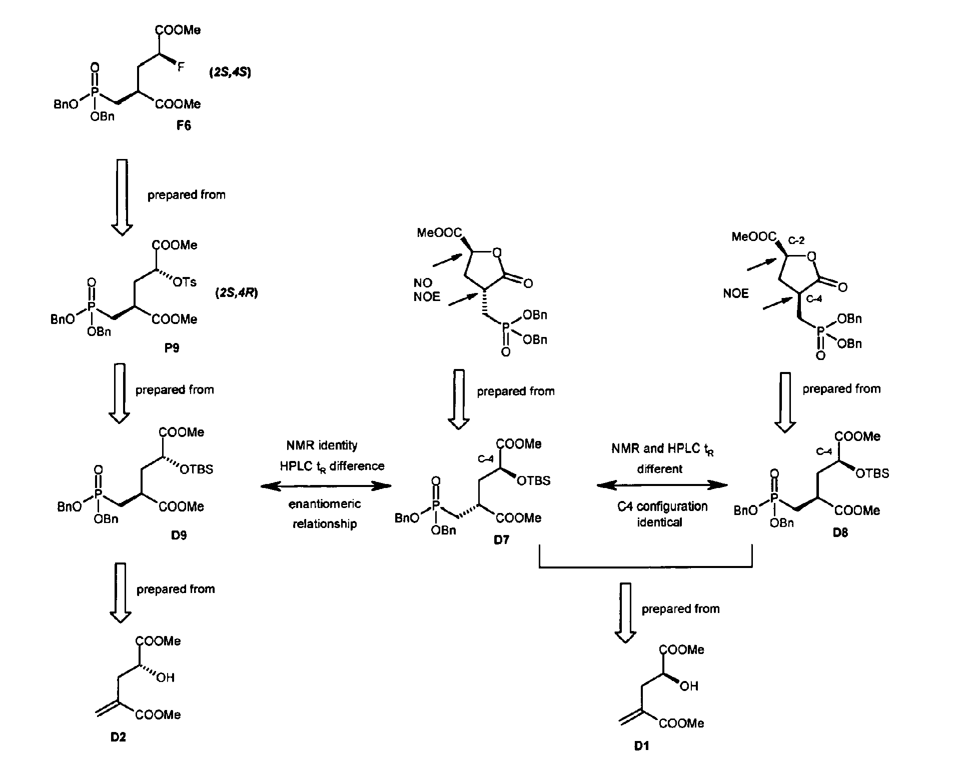

By correlating HPLC retention and NMR data, and correlation to the respective enantiomerically pure a-methyleneglutarate synthon, all other non-racemic Intermediates and Example compounds not hitherto mentioned in this paragraph could be assigned with regard to their absolute configuration.

### General procedures

**GP1: (TBS introduction)** To a cooled (0°C) solution of the starting alcohol (1.00 eq) in dichloromethane (approx. 5 mL / mmol) was added *N-*Methylimidazole (1.40 eq), followed by *tert-*butyldimethylchloro silane (TBS Chloride, 1.30 eq). The cooling bath was removed and the mixture was allowed to stir overnight at room temperature. Subsequently, the double volume of dichloromethane was added and the solution was washed with brine twice. The organic layer was dried over sodium sulfate and evaporated. The residue was purified by column chromatography on silica.

**GP2: (Tosylation)** To a cooled (0°C) solution of the starting alcohol (1.00 eq) in pyridine (approx. 20 mL per mmol) was added *para-*toluenesulfonyl anhydride (2.00 eq). and the mixture was stirred for a period ranging from 2 h at 0°C up to 16 h at room temperature, as judged by TLC or HPLC/MS analysis according to method A1 (or as specified in the respective individual protocol). The reaction mixture was then partitioned between water and dichloromethane, the organic layer was then washed with water and brine, dried over sodium sulfate, and evaporated. The crude product was used crude or was purified by column chromatography over silica.

### Preparation of Intermediates

### Intermediate A1: Dimethyl (RS)-2-hydroxy-4-methylenepentanedioate

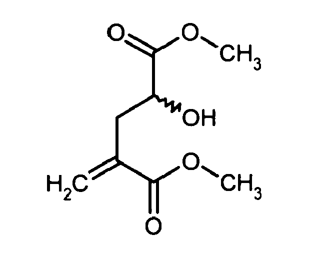

In a three-necked flask equipped with a powerful mechanical stirrer, to a cooled (+5°C, ice-water bath) mixture of methyl glyoxylate (10.0 grams, 114 mmol), methyl (2-bromomethyl) acrylate (22.5 g, 1.10 eq), methanol (80 mL), and 0.3 N aqueous hydrochloric acid (80 mL) was added powdered Indium (100 mesh, 13.0 g, 1.00 eq.) in several portions maintaining the temperature below 35 °C (which did no longer require full cooling during the addition of the later portions). The cooling bath was then completely removed, several freshly broken glass sherds (from a Pasteur pipette, to prevent clotting of the Indium) were added and the mixture was stirred vigorously for 4 h, during which the mixture cooled down to room temperature. The mixture was decanted off all solids and the supernatant was concentrated *in vacuo.* The residue was saturated with solid sodium chloride and then extracted by MTB; the residue remaining hereafter was loaded on Celite and was washed with MTB (4x) and EtOAc (1x). The combined organic layers were dried over sodium sulfate, passed over a plug of Celite, and evaporated. The residue was purified by column chromatography over silica to give 16.8 g of the target compound in 94 % purity (74 % yield).
¹H NMR (300 MHz, CDCl₃) δ ppm 2.65 (dd, 1 H) 2.87 (dd, 1 H) 3.07 (s br, 1 H) 3.77 (s, 3 H), 3.78 (s, 3 H) 4.34 - 4.44 (m, 1 H) 5.74 (m, 1 H) 6.30 (m, 1 H).
MS (CI): [M + H]⁺ = 189.
MS (CI): [M + NH₄]⁺ = 206.

### Intermediate A2: Dimethyl (RS)-2-{[tert-butyl(dimethyl)silyl]oxy}-4-methylenepentanedioate

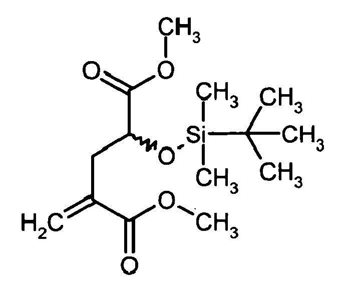

Intermediate A2 was prepared according to General Procedure 1 from Intermediate A1 (2.00 g, 10.6 mmol). Yield 2.69 g (84 %).
¹H NMR (300 MHz, CDCl₃) δ ppm 0.00 (s, 3 H) 0.04 (s, 3 H) 0.87 (s, 9 H) 2.59 (dd, 1 H) 2.83 (dd, 1 H) 3.72 (s, 3 H) 3.76 (s, 3 H) 4.43 (dd, 1 H) 5.67 (m, 1 H) 6.26 (m, 1 H).
MS (EI): [M + H]⁺ = 303.

### Intermediate A3: Dimethyl rac-2-{[bis(benzyloxy)phosphoryl]methyl}-4-{[tert-butyl(dimethyl)silyl]oxy}pentanedioate

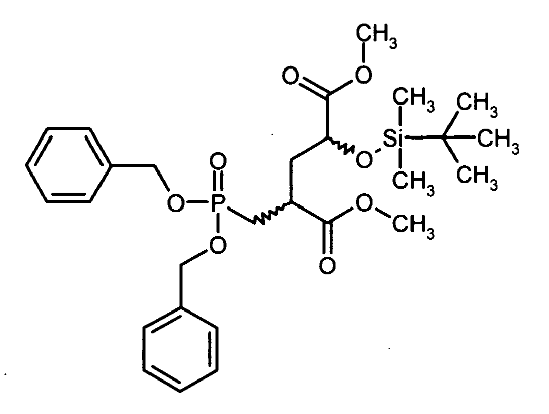

A mixture of Intermediate A2 (1.88 g, 6.22 mmol), dibenzyl phosphite (1.72 mL, 1.25 eq), and finely powdered potassium carbonate (1.29 g, 1.50 eq) in DMF (40 mL) was heated to 90°C for 2 h and was then allowed to stir at room temperature for 60 h. The mixture was then evaporated and partitioned between EtOAc and 5 % aqueous citric acid. The organic layer was washed with half-concentrated brine, dried over sodium sulfate, and evaporated. The residue was purified by column chromatography over silica to give the desired product (3.43 g, 98 % yield) as an oil.
¹H NMR (300 MHz, CDCl₃) δ ppm -0.02 - 0.08 (m, 6 H) 0.88 (s, 9 H, major diastereomer) 0.89 (s, 9 H, minor diastereomer) 1.84 - 2.35 (m, 4 H) 2.86 - 3.12 (m, 1 H) 3.53 (s, 3 H, major diastereomer) 3.55 (m, 3 H, minor diastereomer) 3.67 (m, 3 H, major diastereomer) 3.68 (m, 3 H, minor diastereomer) 4.14 - 4.24 (m, 1 H) 4.90 - 5.07 (m, 4 H) 7.28 - 7.40 (m, 10 H).
MS (ESI): [M + H]⁺ = 565.

### Intermediate B1: Dimethyl rac-2-methylene-4-[(3,4,5,6-tetrahydro-2H-pyran -2-yl)oxy]pentanedioate

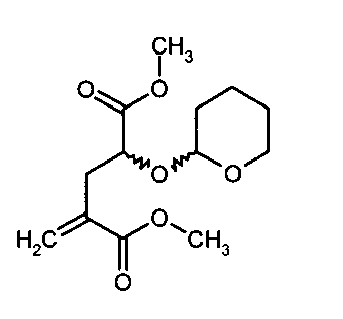

To a solution of Intermediate A1 (3.63 g, 19.3 mmol) in dichloromethane (125 mL) was added 3,4-dihydro-*2H-*pyrane (3.52 mL, 2.00 eq), and pyridinium *para-*toluenesulfonate (PPTS; 727 mg, 0.15 eq), and the resulting mixture was stirred for 2 days at room temperature. The mixture was then evaporated and the residue was purified by column chromatography to give the target compound (4.80 g, 91 % yield) as an oily mixture of stereoisomers.
¹H NMR (300 MHz, CDCl₃) δ ppm 1.45 - 1.93 (m, 6 H) 2.67 - 2.93 (m, 2 H) 3.32 - 3.58 (m, 2 H + 1 H major diastereomer) 3.72 (s, 3 H) 3.77 (s, 3 H) 3.82 - 3.95 (m, 1 H minor diastereomer) 4.22 - 4.31 (m, 1 H minor diastereomer) 4.50 - 4.58 (m, 1 H major diastereomer) 4.63 (t, 1 H minor diastereomer) 4.69 - 4.75 (m, 1 H major diastereomer) 5.68 (m, 1 H minor diastereomer) 5.75 (m, 1 H major diastereomer) 6.24 (m, 1 H minor diastereomer) 6.28 (m, 1 H major diastereomer).
MS (ESI): [M + Na]⁺ = 295.
MS (CI): [M + NH₄]⁺ = 290.

### Intermediate B2: Dimethyl rac-2-{[bis(benzyloxy)phosphoryl]methyl} -4-[(3,4,5,6-tetrahydro-2H-pyran-2-yl)oxy]pentanedionate

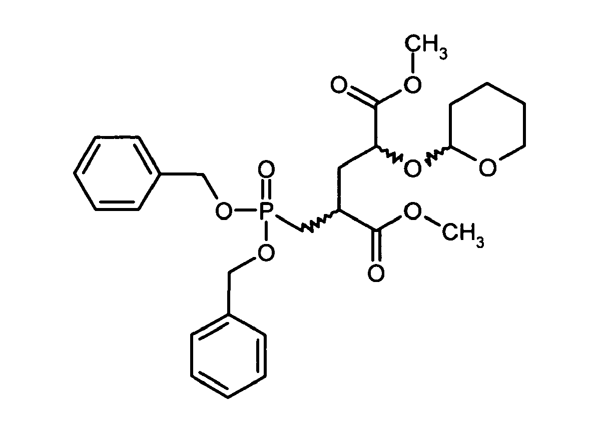

To a solution of Intermediate B1 (2.00 mg, 7.35 mmol, 1.00 eq) in DMF (6 mL) was added dibenzyl phosphite (3.25 mL, 2.00 eq) and finely powdered potassium carbonate (2.23 g, 2.20 eq.) and the mixture was heated to 90°C for 2 hours by means of a microwave oven. The mixture was then evaporated, partitioned between EtOAc and 5% aqueous citric acid, and the organic layer was then washed with half-concentrated brine, dried over sodium sulfate, and evaporated. Column chromatography of the residue gave the desired product (3.00 g, 76 % yield) as an oily mixture of stereoisomers.
¹H NMR (400 MHz, CDCl₃) δ ppm 1.35 - 2.39 (m, 10 H), 2.82 - 3.16 (m, 1 H) 3.34 - 3.51 (m, 1 H) 3.52 - 3.60 (m, 3 H) 3.67 (s, 3 H) 3.67 - 4.02 + 4.30 - 4.38 (2 m, 2 H together) 4.52 - 4.71 (m, 1 H), 4.88 - 5.09 (m, 4 H), 7.27 - 7.42 (m, 10 H).
MS (CI): [M - THP]⁺ = 451.

The same product was obtained by stirring Intermediate B1 (1.00 eq) with dibenzyl phosphite (1.25 eq), DBU (1,8-Diazabicyclo(5.4.0)undec-7-en, 1.25 eq) at room temperature for 22 h, addition of dibenzyl phosphite (1.00 eq), and additional stirring at room temperature for 2 days, and work-up as described above. The yield was then 41 %.

### Intermediate B3: Bis(4-methoxybenzyl) rac-2-methylene-4-[(3,4,5,6-tetrahydro-2H-pyran-2-yl)oxy]pentanedioate

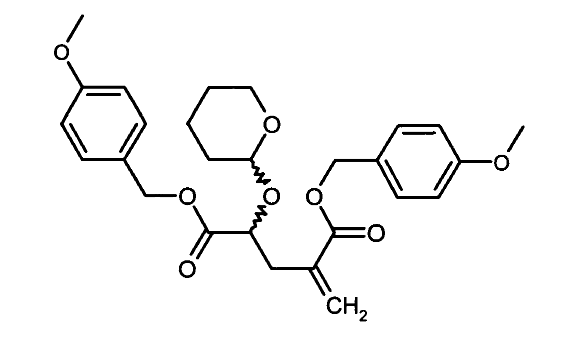

To a solution of Intermediate B1 (240 mg, 881 µmol) in methanol (5 mL) was added aqueous 1 N sodium hydroxide (3 mL). The mixture was stirred overnight at room temperature and was then brought to pH 7 by addition of 2 N hydrochloric acid. After evaporation of methanol and re-dissolution in water (55 mL), the mixture was evaporated by lyophilisation. An aliquot of the residue (215 mg of 390 mg) was dissolved in DMF (10 mL), treated with 4-methoxybenzyl chloride (202 µL, 2.0 eq basing on the aliquot employed) and caesium carbonate (510 mg, 2.1 eq), and was stirred overnight at room temperature, followed by heating in a microwave oven (0.5 h 100 °C, 1 h 120 °C). Subsequently, the mixture was evaporated and subjected to column chromatography without further work-up to give 121 mg (51 % overall yield reflecting the aliquotation) of the target product as mixture of isomers.
¹H NMR (300 MHz, CDCl₃) δ ppm 1.36 - 1.88 (m, 6 H) 2.66 - 2.98 (m, 2 H) 3.26 - 3.42 (m, 1 H) 3.60 - 3.77 (m, 1 H) 3.77 - 3.86 (m, 6 H) 4.28 (mc, 1 H minor isomer) 4.51 - 4.60 (m, 1 H) 4.67 - 4.73 (m, 1 H, major isomer) 5.01 - 5.19 (m, 4 H) 5.59 (d, 1 H minor isomer) 5.68 (d, 1 H major isomer) 6.20 (d, 1 H minor isomer) 6.26 (d, 1 H major isomer) 6.82 - 6.93 (m, 4 H) 7.23 - 7.37 (m, 4 H).
MS (CI): [M + NH₄]⁺ = 502.

### Intermediate C1: Dimethyl (RS)-2-methylene-4-(tosyloxy)pentanedionate

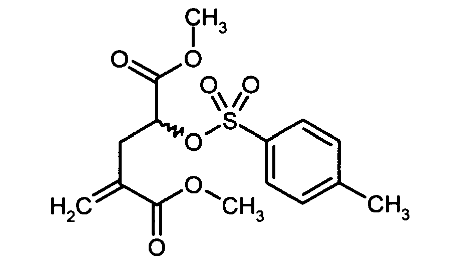

Intermediate C1 was prepared from Intermediate A1 (1.00 g, 5.31 mmol) according to General Procedure 2. Yield 1.80 g (99 %).
¹H-NMR (400 MHz, CDCl₃): δ ppm = 2.45 (s, 3 H) 2.69 (dd, 1 H) 2.90 (dd, 1 H) 3.67 (s, 3 H) 3.68 (s, 3 H) 5.06 (dd, 1 H) 5.67 (s, 1 H) 6.21 (s, 1 H) 7.32 (d, 2 H) 7.75 (d, 2 H).
MS (ESI): [M + H]⁺ = 343.

### Intermediate D1: Dimethyl (S)-2-hydroxy-4-methylenepentanedioate

### Intermediate D2: Dimethyl (R)-2-hydroxy-4-methylenepentanedioate

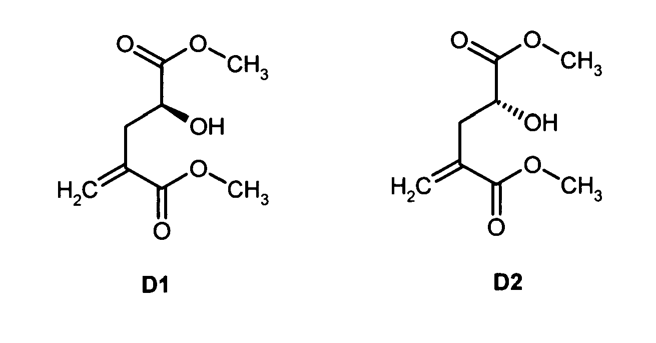

Intermediate A1 was separated into its enantiomers D1 and D2 by preparative chiral HPLC using HPLC method P1.
**Intermediate D1:** t_{R} = 6.5 min (HPLC Method A2)
**Intermediate D2:** t_{R} = 7.9 min (HPLC Method A2)
As evident to the person skilled in the art, the HNMR spectra generated from Intermediates D1 and D2 were both equivalent to the HNMR spectrum of Intermediate A1.

### Intermediate D3: Dimethyl (S)-2-{[tert-butyl(dimethyl)silyl]oxy}-4-methylenepentanedioate

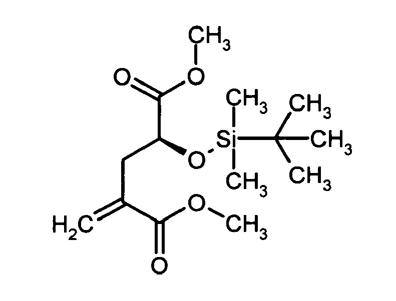

Intermediate D3 was prepared from Intermediate D1 (4.15 g, 22.1 mmol) according to General Procedure 1. Yield 6.17 g (93 %).
MS (ESI): [M + H]⁺ = 303.

### Intermediates D4:Dimethyl (R)-2-{[tert-butyl(dimethyl)silyl]oxy}-4-methylenepentanedioate

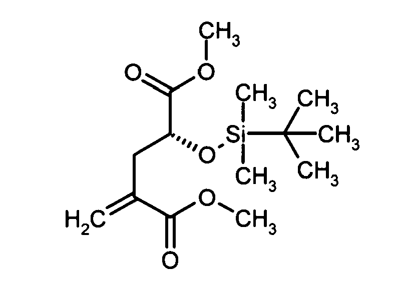

Intermediate D4 was prepared from Intermediate D2 (3.60 g, 19.1 mmol) according to General Procedure 1. Yield 4.73 g (82 %).
MS (ESI): [M + H]⁺ = 303.

### Intermediate D5: Dimethyl (2RS,4S)-2-{[bis(benzyloxy)phosphoryl]methyl}-4-{[tert-butyl(dimethyl)silyl]oxy}pentanedioate

### Intermediate D6: Dimethyl (2RS,4R)-2-{[bis(benzyloxy)phosphoryl]methyl}-4-{[tert-butyl(dimethyl)silyl]oxy}pentanedioate

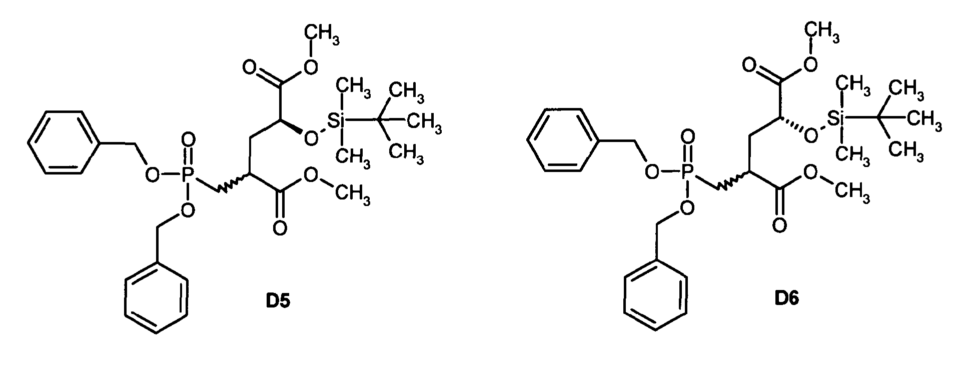

### Intermediate D5

To a solution of D3 (6.17 g, 20.4 mmol, 1.00 eq) in DMF (100 mL) was added dibenzyl phosphite (5.63 mL, 1.25 eq.) and finely powdered potassium carbonate (4.23 g, 1.50 eq), and the mixture was heated to 90°C for 2 h, followed by stirring at room temperature for 16 h. The mixture was evaporated and partitioned between EtOAc and 5 % aqueous citric acid. The organic layer was washed with half-concentrated brine, dried over sodium sulfate, and evaporated. Column chromatography of the residue gave the product as an oil (9.13 g, 79 % yield).

### Intermediate D6

In similar fashion, Intermediated D6 was prepared from Intermediate D4.

As evident to the person skilled in the art, the HNMR spectra generated from Intermediates D5 and D6 were both equivalent to the HNMR spectrum of Intermediate A3
MS (ESI): [M + H]⁺ = 565.

### Intermediate D7: Dimethyl (2R,4S)-2-{[bis(benzyloxy)phosphoryl]methyl}-4-{[tert-butyl(dimethyl)silyl]oxy}pentanedioate Intermediate D8: Dimethyl (2S,4S)-2-{[bis(benzyloxy)phosphoryl]methyl}-4-{[tert-butyl(dimethyl)silyl]oxy}pentanedioate

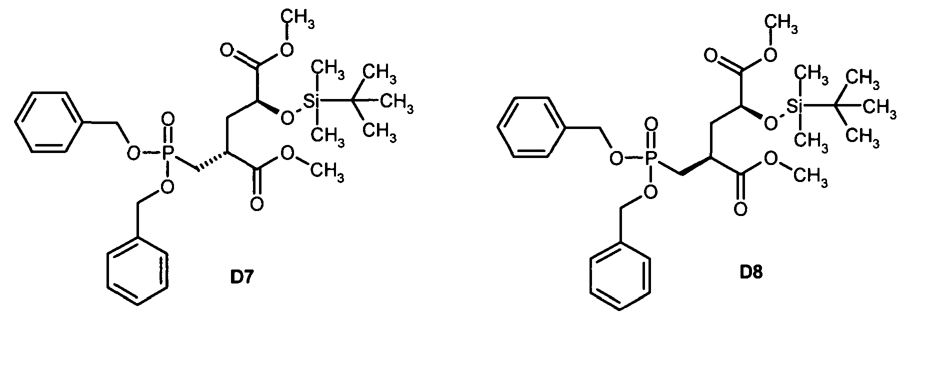

Intermediate D5 (2.00 g) was separated into the respective single isomers by means of preparative chiral HPLC, Method P2, to give the Intermediates D7 (710 mg) and D8 (1.01 g).

### Intermediate D7:

t_{R} = 6.1 min (HPLC Method A3)
¹H NMR (400 MHz, CDCl₃) δ ppm 0.02 (s, 3 H) 0.05 (s, 3 H) 0.89 (s, 9 H) 1.86 - 1.98 (m, 2 H) 2.09 - 2.18 (m, 1 H) 2.21 - 2.33 (m, 1 H) 2.98 - 3.11 (m, 1 H) 3.55 (s, 3 H) 3.68 (s, 3 H) 4.20 (dd, 1 H) 4.91 - 5.06 (m, 4 H) 7.29 - 7.39 (m, 10 H).
MS (ESI): [M + H]⁺ = 565.

### Intermediate D8:

t_{R} = 7.4 min (HPLC Method A3)
¹H NMR (400 MHz, CDCl₃) δ ppm 0.02 (s, 3 H) 0.04 (s, 3 H) 0.88 (s, 9 H) 1.85 - 2.18 (m, 3 H) 2.19 - 2.31 (m, 1 H) 2.89 - 3.02 (m, 1 H) 3.53 (s, 3 H) 3.67 (s, 3 H) 4.18 - 4.25 (m, 1 H) 4.90 - 5.06 (m, 4 H) 7.29 - 7.39 (m, 10 H).
MS (ESI): [M + H]⁺ = 565.

### Intermediate D9: Dimethyl (2S,4R)-2-{[bis(benzyloxy)phosphoryl]methyl}-4-{[tert-butyl(dimethyl)silyl]oxy}pentanedioate Intermediate D10: Dimethyl (2R,4R)-2-{[bis(benzyloxy)phosphoryl]methyl}-4-{[tert-butyl(dimethyl)silyl]oxy}pentanedioate

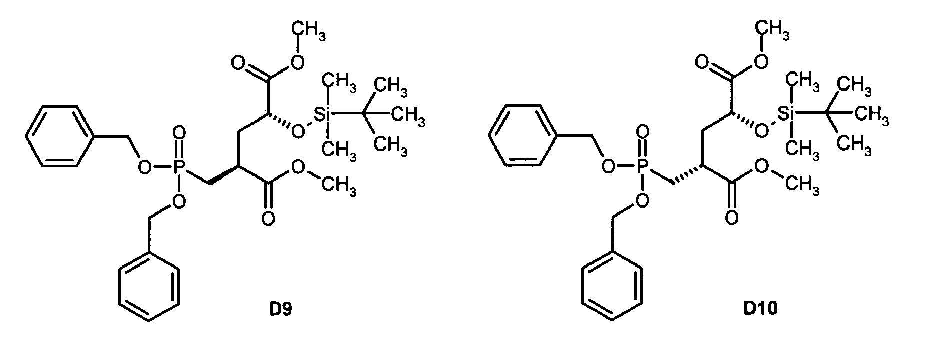

Intermediate D6 (1.00 g) was separated into the respective single isomers by means of preparative chiral HPLC, Method P3, to give the Intermediates D9 (315 mg) and D10 (460 mg).

### Intermediate D9:

t_{R} = 3.8 min (HPLC Method A4)
¹H NMR (400 MHz, CDCl₃) δ ppm 0.02 (s, 3 H) 0.05 (s, 3 H) 0.89 (s, 9 H) 1.86 - 1.98 (m, 2 H) 2.09 - 2.18 (m, 1 H) 2.21 - 2.33 (m, 1 H) 2.98 - 3.11 (m, 1 H) 3.55 (s, 3 H) 3.68 (s, 3 H) 4.20 (dd 1 H) 4.91- 5.06 (m, 4 H) 7.29 - 7.39 (m, 10 H).
MS (ESI): [M + H]⁺ = 565.

### Derivatisation of Intermediate D9 for correlation of stereochemistry with Example P9

To a solution of example D9 (32 mg, 56.7 µmol) in methanol (0.50 mL) was added (*1S*)-(+)-camphorsulfonic acid monohydrate (3.0 mg, 0.2 eq) and the mixture was stirred at room temperature for 17 h. The mixture was diluted with toluene (2 mL) and evaporated to dryness. The residue was again diluted with toluene (2 mL) and evaporated. A solution of *para-*toluenesulfonyl anhydride (55.5 mg, 3.00 eq) in pyridine (0.30 mL) was added at a temperature of 0 °C. The mixture was allowed to warm up to room temperature and was stirred for 1.5 h before being partitioned between 5 % aqueous citric acid and MTB. The organic layer was washed with brine, dried over sodium sulfate, and evaporated. The mixture was filtered over a short plug of silica (EtOAc / hexane) to give 17 mg of a crude product. Chemical impurities were removed by HPLC (HPLC method P7) yielding 8 mg of the corresponding tosylate found to be identical with example P9 as judged by comparison of HPLC retention and HNMR data.
t_{R} = 9.0 min (HPLC Method A6)
¹H NMR (400 MHz, CDCl₃) δ ppm 1.83 - 1.95 (m, 1 H) 2.10 - 2.31 (m, 3 H) 2.43 (s, 3 H) 2.81
- 2.95 (m, 1 H) 3.51 (s, 3 H) 3.62 (s, 3 H) 4.88 - 5.05 (m, 5 H) 7.28 - 7.40 (m, 12 H) 7.77 (d, 2 H).

### Intermediate D10:

t_{R} = 5.2 min (HPLC Method A4)
¹H NMR (400 MHz, CDCl₃) δ ppm 0.02 (s, 3 H) 0.04 (s, 3 H) 0.88 (s, 9 H) 1.85 - 2.18 (m, 3 H) 2.19 - 2.31 (m, 1 H) 2.89 - 3.02 (m, 1 H) 3.53 (s, 3 H) 3.67 (s, 3 H) 4.18 - 4.25 (m, 1 H) 4.90 - 5.06 (m, 4 H) 7.29 - 7.39 (m, 10 H).
MS (ESI): [M + H]⁺ = 565.

### Formation of lactones from Intermediates D7 and D8 and investigations on nuclear Overhauser effect for assignment of relative configurations of C-2 and C-4

### Methyl (2S,4R)-4-{[bis(benzyloxy)phosphoryl]methyl}-5-oxotetrahydrofuran-2-carboxylate (Lactone from Intermediate D7)

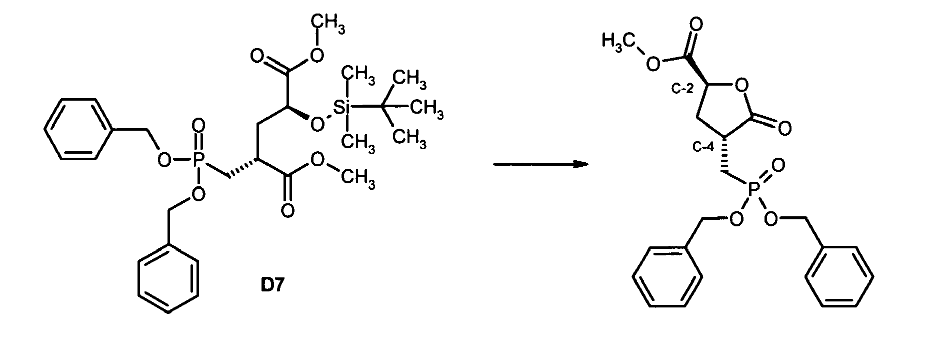

To a solution of Intermediate D7 (22 mg, 39 µmol) in methanol (0.30 mL) was added (*1S*)-(+)-camphorsulfonic acid monohydrate (2.0 mg, 0.2 eq), and the mixture was stirred for 18 h at room temperature. An aliquot (1/3) was taken, diluted with toluene (1 mL) and evaporated to dryness. EtOAc and toluene (2 mL each) were added, and the mixture was extracted with aqueous sodium bicarbonate and brine, filtered over cotton, and evaporated. Intermediate HNMR analysis showed complete conversion into the corresponding γ-hydroxy ester. This was dissolved in toluene (2 mL), (*1S*)-(+)-camphorsulfonic acid monohydrate (25 mg) was added and the mixture was stirred for at room temperature until completion (typically after 6 to 24 h). The mixture was then diluted with EtOAc (2 mL). The mixture was then extracted with aqueous sodium bicarbonate and brine, filtered over cotton, and evaporated to give the corresponding lactone.
¹H NMR (500 MHz, CDCl₃) δ ppm 1.70-1.81 (m, 1 H), 2.25 - 2.35 (m, 1 H), 2.47 (ddd, 1 H), 2.55 - 2.63 (m, 1 H), 2.86 - 2.98 (m, 1 H), 3.79 (s, 3 H), 4.85 (dd, 1 H), 4.92 - 5.00 (m, 2 H), 5.03 - 5.10 (m, 2 H); 7.30 - 7.41 (m, 10 H).

### Methyl (2S,4S)-4-{[bis(benzyloxy)phosphoryl]methyl}-5-oxotetrahydrofuran-2-carboxylate (Lactone from Intermediate D8)

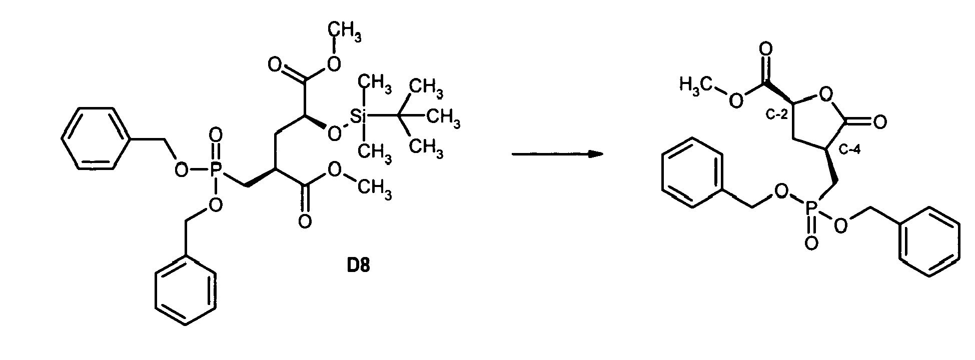

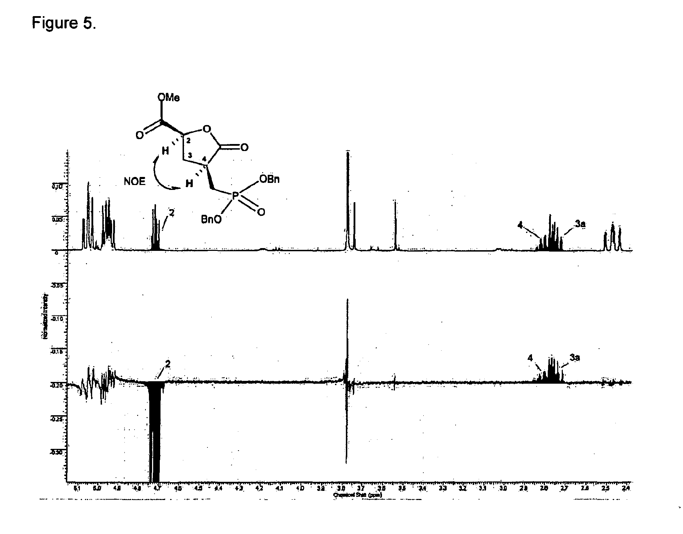

Intermediate D8 (32 mg, 57 µmol) was converted into the corresponding lactone by an analogous procedure.
¹H NMR (500 MHz, CDCl₃) δ ppm 1.76 - 1.87 (m, 1 H), 1.98 - 2.07 (m, 1 H), 2.48 (ddd, 1 H), 2.73 - 2.86 (m, 2 H), 3.79 (s, 3 H), 4.73 (dd, 1 H), 4.93 - 5.00 (m, 2 H), 5.03 - 5.10 (m, 2 H); 7.30-7.41.
A pronounced nuclear Overhauser effect (NOE) was found for the signal of the proton attached to C-4 (signal is a part of a multiplet around 2.8 ppm) upon irradiation on the proton attached to C-2, the signal of which appears at 4.73 ppm.This effect indicates relative cis-configuration of the respective protons (Figure 5). No similar effect was found for the lactone formed from intermediate D7, to which *trans*-configuration was assigned in turn.

### Intermediate E1: Dimethyl (RS)-2-fluoro-4-methylenepentanedioate

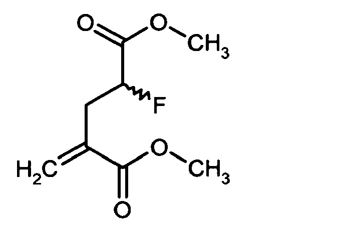

To a solution of Intermediate A1 (6.00 g, 31.9 mmol) in THF (60 mL) was added at room temperature perfluorobutanesulfonic acid fluoride (11.7 mL, 2.00 eq), triethylamine trihydrofluoride (10.4 mL, 2.00 eq.) and triethylamine (26.7 mL, 6.00 eq.). After stirring for 20 h at room temperature, the reaction mixture was partitioned between water and dichloromethane. The organic layer was separated and washed with a brine/water mixture (1:1), dried over sodium sulfate and concentrated under reduced pressure. The crude product was purified by column chromatography (silica, hexane / EtOAc) to give Intermediate E1 (4.37 g, 72 % yield).
¹H-NMR (400 MHz, CDCl₃): δ ppm 2.76 - 2.88 (m, 1 H) 2.94 - 3.06 (m, 1 H) 3.79 (s, 3 H) 3.80 (s, 3 H) 5.18 (ddd, 1 H) 5.78 (s, 1 H) 6.35 (s, 1 H).
MS (ESI): [M + H]⁺ = 191.

### Intermediates E2 and E3: Enantiomers:

### Example E2: Dimethyl (S)-2-fluoro-4-methylenepentanedioate Example E3: Dimethyl (R)-2-fluoro-4-methylenepentanedioate

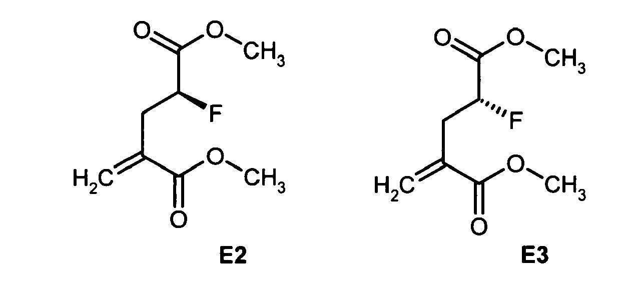

Intermediate E1 was separated into its enantiomers E2 and E3 by preparative chiral HPLC using HPLC method P4.
**Intermediate E2:** t_{R} = 4.8 min (HPLC Method A5)
**Intermediate E3:** t_{R} = 6.3 min (HPLC Method A5)

As evident to the person skilled in the art, the HNMR spectra generated from Intermediates E2 and E3 were both equivalent to the HNMR spectrum of Intermediate E1.

### Example compounds of the invention (Precursor compounds)

### Example P1:

### Dimethyl rac-2-{[bis(benzyloxy)phosphoryl]methyl}-4-(mesyloxy)pentanedioate

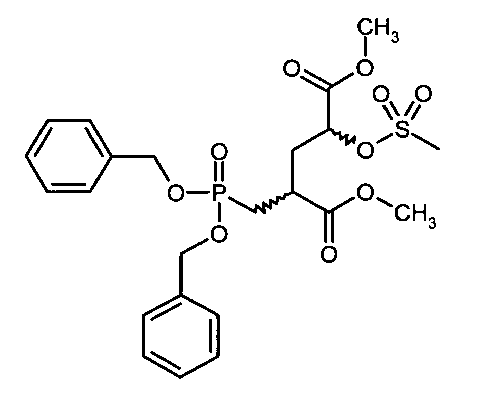

To a solution of intermediate A3 (600 mg, 1.06 mmol, 1.00 eq) in THF (7.5 mL) was added hydrogen fluoride-pyridine complex (1.52 g, 50 eq) under ice cooling. The cooling bath was removed and the mixture was stirred for 3 h at room temperature. A solution prepared from potassium hydroxide (1.80 g), boric acid (3.95 g), and water (30 mL) was carefully added with ice cooling, until pH7 was reached. The mixture was concentrated *in vacuo* and then partitioned between MTB and water. The organic layer was washed with brine, dried over sodium sulfate, and evaporated to give the crude intermediate γ-hydroxy ester which was very prone to lactonisation (0.55 g crude). An aliquot (0.28 g) was then dissolved in pyridine (5 mL) and treated with methanesulfonic anhydride (182 mg, 1.68 eq. basing on the aliquot) under ice cooling. After complete addition, the cooling bath was removed and stirring at room temperature was continued for 3 h. The reaction mixture was partitioned between MTB and half-concentrated brine, and the organic layer was again washed with half-concentrated brine, dried over sodium sulfate, and evaporated. The crude product was purified by repeated column chromatography to give a pure sample of Example P1 as mixture of stereoisomers (86 mg, 30 % yield based on aliquotation).
¹H-NMR (300 MHz, CDCl₃): δ ppm 1.86 - 2.12 (m, 1 H) 2.14 - 2.41 (m, 3 H) 2.86 - 3.06 (m, 1 H) 3.11 (s, 3 H, minor diastereomer) 3.12 (s, 3 H, major diastereomer) 3.65 (s, 3 H, major diastereomer) 3.61 (s, 3 H, minor diastereomer) 3.75 (s, 3 H, major diastereomer) 3.77 (s, 3 H, minor diastereomer) 4.90 - 5.09 (m, 5 H) 7.30 - 7.42 (m, 10 H).
MS (ESI): [M + H]⁺ = 529.

### Example P2:

### Dimethyl rac-2-{[bis(benzyloxy)phosphoryl]methyl}-4-{[(4-nitrophenyl) sulfonyl]oxy}pentanedioate

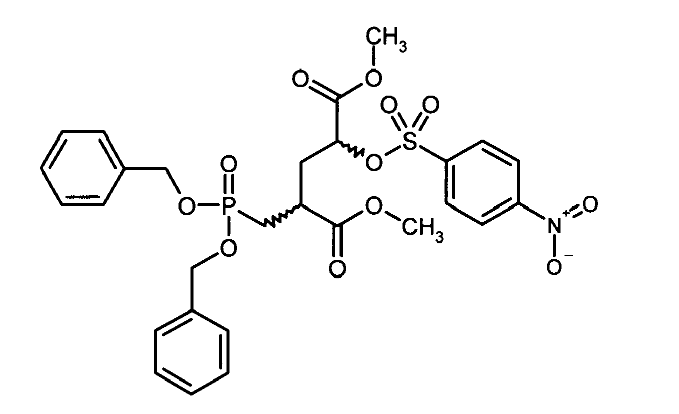

To a solution of intermediate A3 (600 mg, 1.06 mmol, 1.00 eq) in THF (7.5 mL) was added hydrogen fluoride-pyridine complex (1.52 g, 50 eq) under ice cooling. The cooling bath was removed and the mixture was stirred for 3 h at room temperature. A solution prepared from potassium hydroxide (1.80 g), boric acid (3.95 g), and water (30 mL) was carefully added with ice cooling, until pH7 was reached. The mixture was concentrated *in vacuo* and then partitioned between MTB and water. The organic layer was washed with brine, dried over sodium sulfate, and evaporated to give the crude intermediate γ-hydroxy ester which was very prone to lactonisation (0.55 g crude). An aliquot (0. 27 g) was then dissolved in dichloromethane (5 mL) with ice cooling, followed by the addition of silver (I) trifluoromethanesulfonate (316 mg, 2.00 eq basing on the aliquot), pyridine (495 µL, 10.0 eq), and 4-nitrobenzenesulfonyl chloride (136 mg, 1.00 eq). The mixture was stirred at room temperature, and repeatedly, portions of silver (I) trifluoromethanesulfonate (1.00 eq) and 4-nitrobenzenesulfonyl chloride (0.50 eq) were added to drive the reaction to an acceptable turnover. After 2 days, the reaction mixture was partitioned between dichloromethane and water, the organic layer was washed with brine, dried over sodium sulfate, and evaporated. Column chromatography of the residue yielded 16 mg of example P2 as a mixture of stereoisomers (5 % yield based on aliquotation).
¹H-NMR (300 MHz, CDCl₃): δ ppm 1.83 - 2.09 (m, 1 H) 2.14 - 2.39 (m, 3 H) 2.79 - 3.02 (m, 1 H) 3.55 (s, 3 H) 3.62 (s, 3 H, major diastereomer) 3.66 (s, 3 H, minor diastereomer) 4.88 - 5.14 (m, 5 H) 7.29 - 7.43 (m, 10 H) 8.05 - 8.14 (m, 2 H) 8.29 - 8.39 (m, 2 H).
MS (ESI): [M + H]⁺ = 636.

### Example P3:

### Dimethyl rac-2-{[bis(benzyloxy)phosphoryl]methyl}-4-[(phenylsulfonyl)oxy]pentanedionate

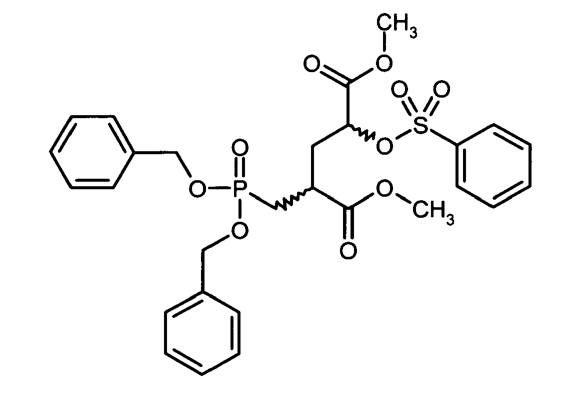

To a solution of intermediate A3 (500 mg, 885 µmol, 1.00 eq) in THF (7.5 mL) was added hydrogen fluoride-pyridine complex (1.52 g, 50 eq) under ice cooling. The cooling bath was removed and the mixture was stirred for 3 h at room temperature. A solution prepared from potassium hydroxide (1.80 g), boric acid (3.95 g), and water (30 mL) was carefully added with ice cooling, until pH7 was reached. The mixture was concentrated *in vacuo* and then partitioned between MTB and water. The organic layer was washed with brine, dried over sodium sulfate, and evaporated to give the crude intermediate γ-hydroxy ester which was very prone to lactonisation (372 mg crude). An aliquot thereof (142 mg) was dissolved in pyridine (3 mL), and benzenesulfonic anhydride (225 mg, 2.40 eq. basing on the aliquot employed) was added whilst cooling with ice. The mixture was stirred for 16 h at room temperature, was partitioned between MTB and half-concentrated brine, and the organic layer was again washed with half-concentrated brine, dried over sodium sulfate, and evaporated. The crude product was purified by column chromatography to give Example P3 as mixture of stereoisomers (103 mg, 51 % yield based on aliquotation).
¹H-NMR (300 MHz, CDCl₃): δ ppm 1.80 - 2.35 (m, 4 H) 2.75 - 3.01 (m, 1 H) 3.51 (s, 3 H, minor diastereomer) 3.52 (s, 3 H, major diastereomer) 3.55 (s, 3 H, major diastereomer), 3.61 (s, 3 H, minor diastereomer) 4.87 - 5.08 (m, 4 H), 7.28 - 7.43 (m, 10 H) 7.48 - 7.57 (m, 2 H) 7.60 - 7.68 (m, 1 H) 7.87 - 7.94 (m, 2 H).
MS (ESI): [M + H]⁺ = 591.

### Example P4a: Process A

### Dimethyl rac-2-{[bis(benzyloxy)phosphoryl]methyl}-4-(tosyloxy)pentanedioate

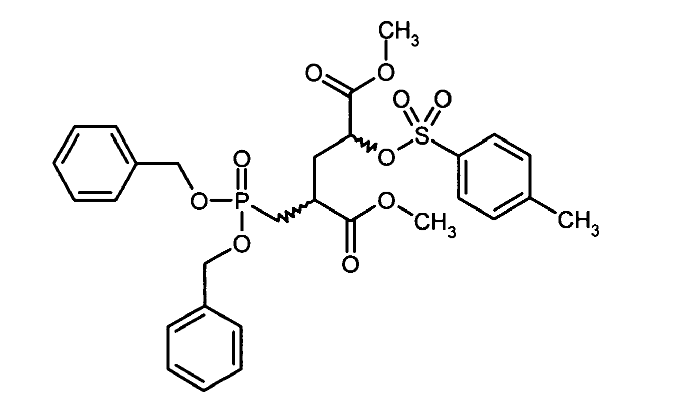

To an ice-cooled solution of Intermediate A3 (1.27 g, 2.25 mmol) in THF (20 mL) was added hydrogen fluoride - pyridine complex (3.91 mL, 20 eq), and the mixture was stirred at room temperature for 4 hours. Subsequently, a solution prepared from potassium hydroxide (3.60 g), boric acid (8.00 g), and water (60 mL) was carefully added until pH reached 7. The mixture was partitioned between water and MTB, and the organic layer was washed with brine, dried over sodium sulfate, and evaporated. The residual crude γ-hydroxy ester (980 mg) was immediately dissolved in pyridine (20 mL), and *para-*toluenesulfonyl anhydride (1.43 g, 2.00 eq) was added, and the mixture was stirred for 16 h at room temperature. The mixture was then partitioned between water and MTB, and the organic layer was washed with 5% aqueous citric acid and with brine, dried over sodium sulfate, and evaporated. The residue was purified by column chromatography to give the target compound as an oily mixture of stereoisomers (890 mg, 65 % yield).
¹H-NMR (400 MHz, CDCl₃): δ ppm 1.83 - 2.07 (m, 1 H) 2.10 - 2.33 (m, 3 H) 2.43 (s, 3 H) 2.81 - 2.98 (m, 1 H) 3.51 (s, 3 H, minor diastereomer) 3.53 (s, 3 H, major diastereomer) 3.57 (s, 3 H, major diastereomer), 3.62 (s, 3 H, minor diastereomer) 4.88 - 5.05 (m 5 H) 7.28 - 7.41 (m, 12 H) 7.75 - 7.81 (m, 2 H).
MS (ESI): [M + H]⁺ = 605.

### Example P4b: Process B

### Dimethyl rac-2-{[bis(benzyloxy)phosphoryl]methyl}-4-(tosyloxy)pentanedioate

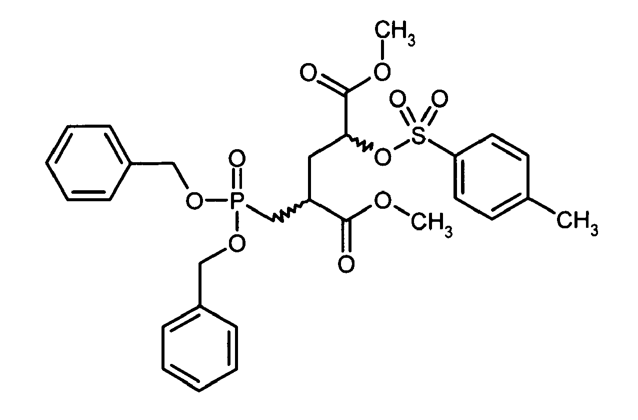

To a solution of Intermediate B2 (150 mg, 281 µmol) in methanol (5 mL) was added pyridinium *para-*toluenesulfonate (PPTS, 7.1 mg, 0.10 eq.) and the resulting mixture was stirred at 55 °C for 2 h. The mixture was evaporated to dryness, and the residual crude γ-hydroxy ester was dissolved in pyridine (3 mL). *para-*Toluenesulfonic anhydride (183 mg, 2.00 eq.) was added, and the mixture was stirred at room temperature for 16 h. The mixture was concentrated *in vacuo* and then partitioned between MTB and 5 % aqueous citric acid. The organic layer was washed with brine, dried over sodium sulfate, and evaporated. Column chromatography over silica (hexane / EtOAc) gave the target compound as a mixture of stereoisomers (115 mg, 68 % yield).
HNMR and MS data were in line with those reported for Example P4a.

### Example P4c: Process C

### Dimethyl rac-2-{[bis(benzyloxy)phosphoryl]methyl}-4-(tosyloxy)pentanedioate

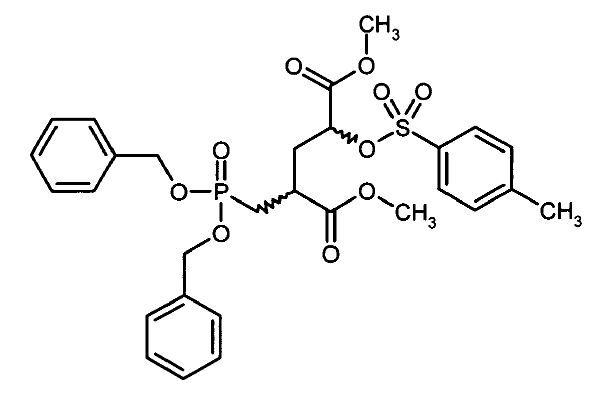

To a solution of Intermediate C1 (1.32 g, 3.81 mmol) in THF (40 mL) was added DBU (1,8-Diazabicyclo(5.4.0)undec-7-en, 717 µL, 1.25 eq) and dibenzyl phosphite (1.70 mL, 2.00 eq.) and the mixture was stirred at room temperature for 1.5 h. The mixture was concentrated *in vacuo* and then partitioned between EtOAc and 5 % aqueous citric acid. The organic layer was washed with brine, dried over sodium sulfate, and evaporated. The residue was purified by column chromatography on silica (hexane / EtOAc) to give the desired product (1.42 g, 61 % yield) as a mixture of stereoisomers.
HNMR and MS data were in line with those reported for Example P4a.

### Example P5: Dimethyl (2RS,4S)-2-{[bis(benzyloxy)phosphoryl]methyl}-4-(tosyloxy)pentanedioate

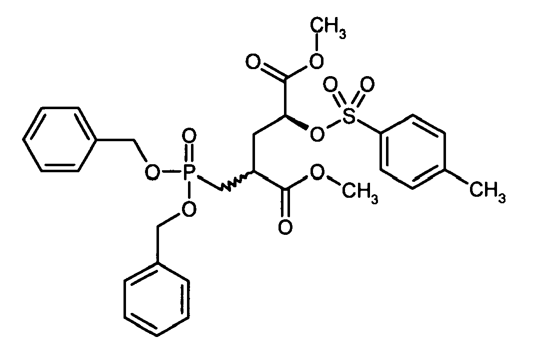

**Example P5**: To an ice-cooled solution of Intermediate D5 (4.57 g, 8.09 mmol) in THF (60 mL) was added hydrogen fluoride - pyridine complex (70 % HF, 14.7 mL, 70 eq). The mixture was stirred 3.5 h at room temperature. A solution prepared from potassium hydroxide (10 g), boric acid (13 g), and water (100 mL) was added carefully until pH had reached 7. The mixture was partitioned between water and MTB, and the organic layer was washed with brine, was dried over sodium sulfate, and evaporated. The residual crude γ-hydroxy ester (3.24 g) was immediately dissolved in pyridine (60 mL). *para-*Toluenesulfonyl anhydride (4.70 g, 2.00 eq, based on crude hydroxy ester) was added, and the mixture was stirred at room temperature for 16 h, concentrated *in vacuo,* and subsequently partitioned between 5 % aqueous citric acid and MTB. The organic layer was washed with half-concentrated brine, dried over sodium sulfate, and evaporated. Column chromatography on silica (hexane / EtOAc) gave the desired product as an oil (1.52 g, 31 % yield) which was used for separation into single stereoisomers (Examples P7 and P8) without further characterisation.
MS (ESI): [M + H]⁺ = 605.

### Example P6: Dimethyl (2RS,4R)-2-{[bis(benzyloxy)phosphoryl]methyl}-4-(tosyloxy)pentanedioate

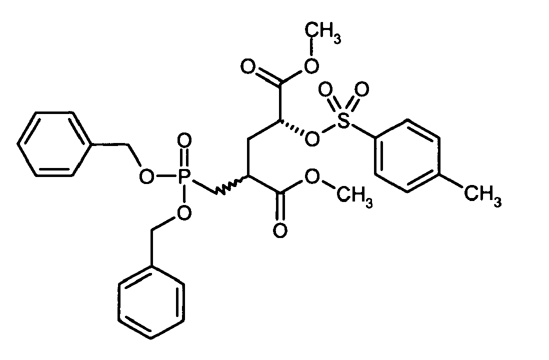

**Example P6:** To an ice-cooled solution of Intermediate D6 (3.32 g, 5.88 mmol) in THF (50 mL) was added hydrogen fluoride - pyridine complex (70 % HF, 7.64 mL, 50 eq). The mixture was stirred 4.5 h at room temperature. A solution prepared from potassium hydroxide (10 g), boric acid (13 g), and water (100 mL) was added carefully until pH had reached 7. The mixture was partitioned between water and MTB, and the organic layer was washed with brine, was dried over sodium sulfate, and evaporated. The residual crude γ-hydroxy ester (2.71 g) was immediately dissolved in pyridine (50 mL). *para-*Toluenesulfonyl anhydride (3.93 g, 2.00 eq, based on crude hydroxy ester) was added, and the mixture was stirred at room temperature for 16 h, concentrated *in vacuo,* and subsequently partitioned between 5 % aqueous citric acid and MTB. The organic layer was washed with half-concentrated brine, dried over sodium sulfate, and evaporated. Column chromatography on silica (hexane / EtOAc) gave the desired product as an oil (2.62 g, 74 % yield) which was used for separation into single stereoisomers (Examples P9 and P10) without further characterisation.
MS (ESI): [M + H]⁺ = 605.

### Example P7: Dimethyl (2S,4S)-2-{[bis(benzyloxy)phosphoryl]methyl}-4-(tosyloxy)pentanedioate

### Example P8: Dimethyl (2R,4S)-2-{[bis(benzyloxy)phosphoryl]methyl}-4-(tosyloxy)pentanedioate

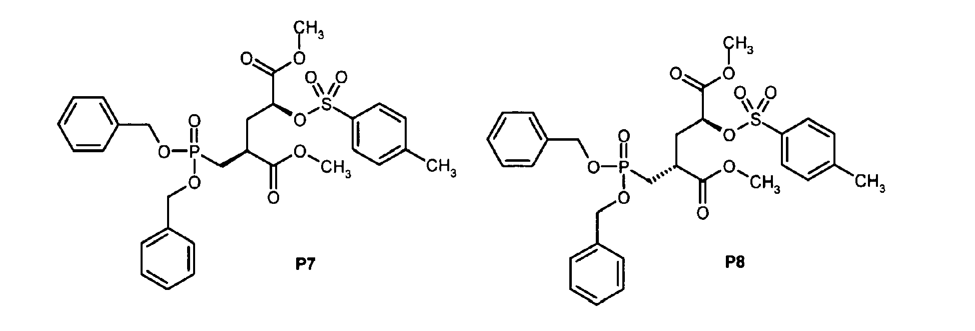

Example P5 (1.52 g) was separated into the respective single isomers by means of preparative chiral HPLC, Method P5, to give the Examples P7 (673 mg) and P8 (423 mg). The samples thus obtained contained minor residues of ethanol which could be removed by extended high-vacuum evaporation.

Example P6 (2.62 g) was separated into the respective single isomers by means of preparative chiral HPLC, Method P6, to give the Examples P9 (859 mg) and P10 (1.34 g). The samples thus obtained contained minor residues of ethanol which could be removed by extended high-vacuum evaporation.

### Example P7:

t_{R} = 9.9 min (HPLC Method A6)
¹H NMR (400 MHz, CDCl₃) δ ppm 1.95 - 2.07 (m, 1 H) 2.12 - 2.33 (m, 3 H) 2.43 (s, 3 H) 2.85
- 2.98 (m, 1 H) 3.53 (s, 3 H) 3.57 (s, 3 H) 4.89 - 5.05 (m, 5 H) 7.28 - 7.40 (m, 12 H) 7.78 (d, 2 H).

### Example P8:

t_{R} = 14.0 min (HPLC Method A6)
¹H NMR (400 MHz, CDCl₃) δ ppm 1.83 - 1.95 (m, 1 H) 2.10 - 2.31 (m, 3 H) 2.43 (s, 3 H) 2.81
- 2.95 (m, 1 H) 3.51 (s, 3 H) 3.62 (s, 3 H) 4.88 - 5.05 (m, 5 H) 7.28 - 7.40 (m, 12 H) 7.77 (d, 2 H).

### Example P9: Dimethyl (2S,4R)-2-{[bis(benzyloxy)phosphoryl]methyl}-4-(tosyloxy)pentanedioate

### Example P10: Dimethyl (2R,4R)-2-{[bis(benzyloxy)phosphoryl]methyl}-4-(tosyloxy)pentanedioate

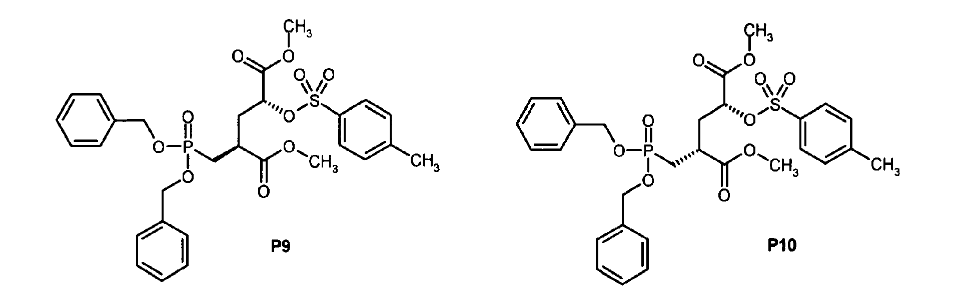

### Example P9:

t_{R} = 9.1 min (HPLC Method A6)
¹H NMR (400 MHz, CDCl₃) δ ppm 1.83 - 1.95 (m, 1 H) 2.10 - 2.31 (m, 3 H) 2.43 (s, 3 H) 2.81
- 2.95 (m, 1 H) 3.51 (s, 3 H) 3.62 (s, 3 H) 4.88 - 5.05 (m, 5 H) 7.28 - 7.40 (m, 12 H) 7.77 (d, 2 H).

### Example P10:

t_{R} = 11.5 min (HPLC Method A6)
¹H NMR (400 MHz, CDCl₃) δ ppm 1.95 - 2.07 (m, 1 H) 2.12 - 2.33 (m, 3 H) 2.43 (s, 3 H) 2.85
- 2.98 (m, 1 H) 3.53 (s, 3 H) 3.57 (s, 3 H) 4.89 - 5.05 (m, 5 H) 7.28 - 7.40 (m, 12 H) 7.78 (d, 2 H).

Alternatively, separation of the four stereoisomers P7 to P10 was achieved from a mixture of all four stereoisomers (Example P4a, ) by means of chiral HPLC, Method P6.
**Example P7:** t_{R} = 9.9 min (HPLC Method A6)
**Example P8:** t_{R} = 14.0 min (HPLC Method A6)
**Example P9:** t_{R} = 9.0 min (HPLC Method A6)
**Example P10:** t_{R} = 11.5 min (HPLC Method A6)

### Example compounds of the invention (¹⁹F compounds)

### Example F1: Dimethyl rac-2-{[bis(benzyloxy)phosphoryl]methyl}-4-fluoropentanedioate

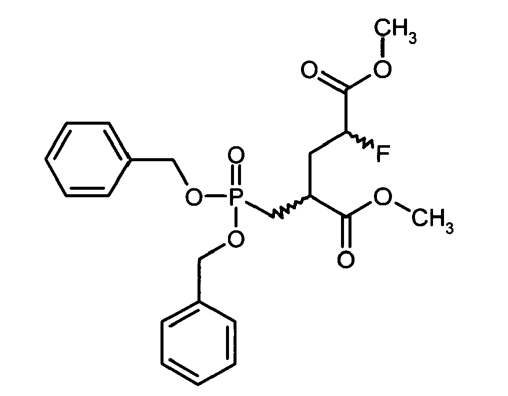

To a solution of Intermediate E1 (50 mg, 0.26 mmol) and dibenzyl phosphite (86 mg, 1.25 eq) in DMF (3 mL) was added finely powdered potassium carbonate (mesh 325, 55 mg, 1.50 eq), and the resulting mixture was heated to 50 °C for 4 h in a microwave oven. After cooling to room temperature, the mixture was partitioned between EtOAc and 5% aqueous citric acid. The organic layer was washed with half-concentrated brine, dried over sodium sulfate, and evaporated. The residue was purified by column chromatography over silica gel (hexane / EtOAc) to give the target compound as a mixture of stereoisomers (99 mg, 84 % yield).
¹H NMR (400 MHz, CDCl₃) δ ppm 1.88 - 2.04 (m, 1 H) 2.10 - 2.39 (m, 3 H) 2.91 - 3.08 (m, 1 H) 3.55 (s, 3 H, minor diastereomer) 3.58 (s, 3 H, major diastereomer) 3.76 (s, 3 H, minor diastereomer) 3.77 (s, 3 H, major diastereomer) 4.82 - 5.08 (m, 5 H) 7.29 - 7.41 (m, 10 H).
MS (ESI): [M + H]⁺ = 453.

### Example F2: Dimethyl rac-2-[(dimethoxyphosphoryl)methyl] -4-fluoropentanedioate

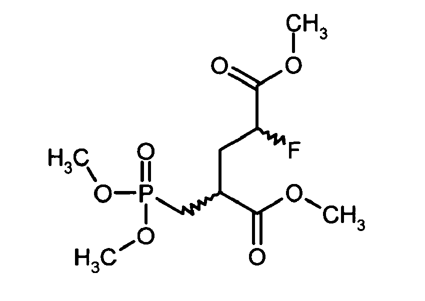

To a solution of Intermediate E1 (220 mg, 1.16 mmol) in DMF (3 mL) was added dimethyl phosphite (135 µL, 1.25 eq.), and finely powdered potassium carbonate (325 mesh, 240 mg, 1.50 eq.), and the mixture was stirred for 2 h at 50 °C in a microwave oven. After cooling to room temperature, the mixture was partitioned between dichloromethane and 5% aqueous citric acid. The organic layer was washed with brine, dried over sodium sulfate, and evaporated. The residue was purified by column chromatography over silica gel (dichloromethane / methanol) to give the target compound as a mixture of stereoisomers (210 mg, 54 % yield).
¹H NMR (400 MHz, CDCl₃) δ ppm 1.92 - 2.08 (m, 1 H) 2.17 - 2.48 (m, 3 H) 2.97 - 3.10 (m, 1 H) 3.71 - 3.83 (m, 12 H) 4.97 (ddd, 1 H, major diastereomer) 5.03 (ddd, 1 H, minor diastereomer).
MS (ESI): [M + H]⁺ = 300.

### Example F3: rac-2-Fluoro-4-(phosphonomethyl)pentanedioic acid

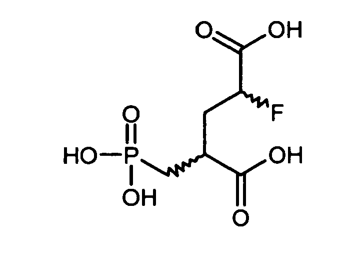

A mixture of Example F1 (100 mg, 221 µmol) in 6 N hydrochloric acid (1.33 mL) was stirred at 100°C in a microwave oven. After cooling to room temperature, the mixture was diluted with water and evaporated by lyophilisation to give the crude target compound which was not purified otherwise (100 mg, >100 % crude yield).
¹H NMR (400 MHz, d₆-DMSO) δ ppm 1.63 - 1.80 (m, 1 H) 1.85 - 2.29 (m, 3 H) 2.64 - 2.80 (m, 1 H) 4.81 - 5.11 (m, 1 H). CO₂H and PO₃H₂ protons form a broad peak with adventitious water at 5.4 ppm.
MS (ESI): [M - H]⁻ = 243.

### Examples F4, F5, F6, and F7: Preparation from Precursors, i.e. Examples P7, P8, P9 and P10

### Example F4: Dimethyl (2S,4R)-2-{[bis(benzyloxy)phosphoryl]methyl}-4-fluoropentanedioate:

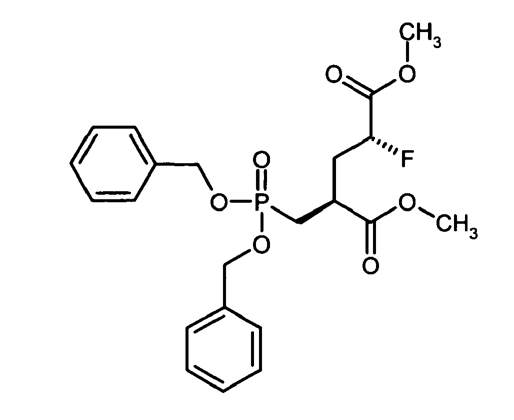

To a solution of Example P7 (214 mg, 354 µmol) in DMF (2 mL) was added potassium fluoride (144 mg, 7.00 eq.) and crown ether 18-crown-6 (93.6 mg, 1.00 eq), and the mixture was stirred at 100 °C for 3 h in a microwave oven. After cooling to room temperature, the mixture was concentrated *in vacuo* and then partitioned between MTB and water. The organic layer was washed with brine, dried over sodium sulfate, and evaporated. The residue was purified by column chromatography on silica (hexane / EtOAc) to give the desired compound (83 mg, 52 % yield) containing one stereoisomer as main component as judged by the analytical data below.
t_{R} = 8.8 min (HPLC Method A6)
¹H NMR (400 MHz, CDCl₃) δ ppm 1.88 - 2.00 (m, 1 H) 2.12 - 2.39 (m, 3 H) 2.95 - 3.08 (m, 1 H) 3.58 (s, 3 H) 3.77 (s, 3 H) 4.82 - 5.08 (m, 5 H) 7.29 - 7.41 (m, 10 H).

### Example F5: Dimethyl (2R,4R)-2-{[bis(benzyloxylphosphoryl]methyl}-4-fluoropentanedioate

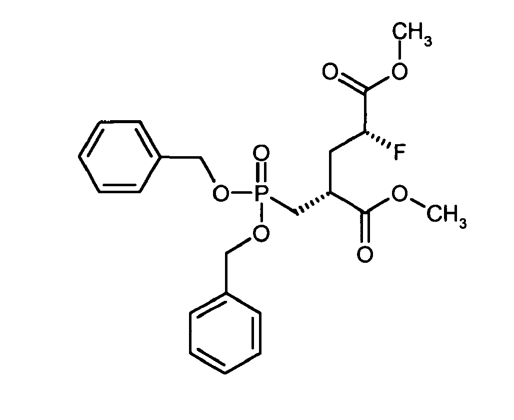

To a solution of Example P8 (137 mg, 227 µmol) in DMF (1 mL) was added potassium fluoride (92.1 mg, 7.00 eq.) and crown ether 18-crown-6 (59.9 mg, 1.00 eq), and the mixture was stirred at 100 °C for 3.5 h in a microwave oven. After cooling to room temperature, the mixture was concentrated *in vacuo* and then partitioned between MTB and water. The organic layer was washed with brine, dried over sodium sulfate, and evaporated. The residue was purified by column chromatography on silica (hexane / EtOAc) to give the desired compound (41 mg, 40 % yield) containing one stereoisomer as main component as judged by the analytical data below.
t_{R} = 9.6 min (HPLC Method A6)
¹H NMR (400 MHz, CDCl₃) δ ppm 1.92 - 2.04 (m, 1 H) 2.13 - 2.37 (m, 3 H) 2.91 - 3.05 (m, 1 H) 3.55 (s, 3 H) 3.76 (s, 3 H) 4.85 - 5.08 (m, 5 H) 7.29 - 7.41 (m, 10 H).

### Example F6: Dimethyl (2S,4S)-2-{(bis(benzyloxy)phosphoryl]methyl} -4-fluoropentanedioate

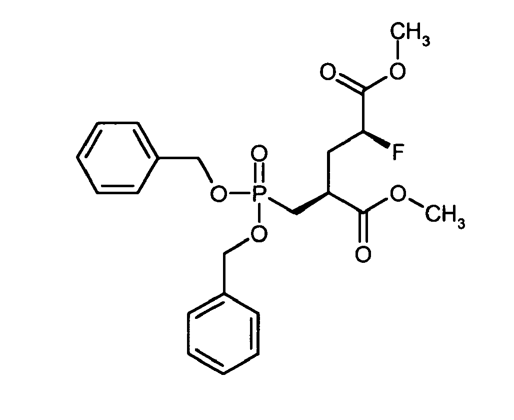

To a solution of Example P9 (108 mg, 179 µmol) in DMF (1 mL) was added potassium fluoride (72.6 mg, 7.00 eq.) and crown ether 18-crown-6 (47.2 mg, 1.00 eq), and the mixture was stirred at 100 °C for 3 h and one additional hour at 95 °C in a microwave oven. After cooling to room temperature, the mixture was concentrated *in vacuo* and then partitioned between MTB and water. The organic layer was washed with brine, dried over sodium sulfate, and evaporated. The residue was purified by column chromatography on silica (hexane / EtOAc) to give the desired compound (47 mg, 59 % yield) containing one stereoisomer as main component as judged by the analytical data below.
t_{R} = 10.4 min (HPLC Method A6)
¹H NMR (300 MHz, CDCl₃) δ ppm 1.90 - 2.05 (m, 1 H) 2.11 - 2.39 (m, 3 H) 2.88 - 3.06 (m, 1 H) 3.55 (s, 3 H) 3.76 (s, 3 H) 4.82 - 5.10 (m, 5 H) 7.28 - 7.43 (m, 10 H).

### Example F7: Dimethyl (2R,4S)-2-{[bis(benzyloxy)phosphoryl]methyl} -4-fluoropentanedioate

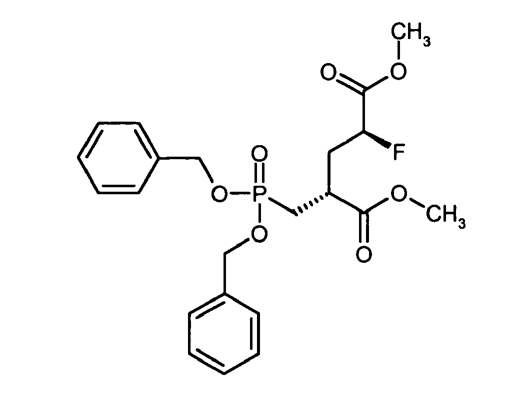

To a solution of Example P10 (191 mg, 316 µmol) in DMF (1 mL) was added potassium fluoride (128 mg, 7.00 eq.) and crown ether 18-crown-6 (83.5 mg, 1.00 eq), and the mixture was stirred at 100 °C for 2:45 h in a microwave oven. After cooling to room temperature, the mixture was concentrated *in vacuo* and then partitioned between MTB and water. The organic layer was washed with brine, dried over sodium sulfate, and evaporated. The residue was purified by column chromatography on silica (hexane / EtOAc) to give the desired compound (59 mg, 42 % yield) containing one stereoisomer as main component as judged by the analytical data below.

t_{R} = 7.2 min (HPLC Method A6)
¹H NMR (400 MHz, CDCl₃) δ ppm 1.88 - 2.00 (m, 1 H) 2.12 - 2.39 (m, 3 H) 2.95 - 3.08 (m, 1 H) 3.58 (s, 3 H) 3.77 (s, 3 H) 4.82 - 5.08 (m, 5 H) 7.29 - 7.41 (m, 10 H).

### Examples F4, F5, F6, and F7: Optimised preparations from Intermediates E2 and E3

### Example F4a: Dimethyl (2S,4R)-2-{[bis(benzyloxy)phosphoryl]methyl}-4-fluoropentanedioate:

### Example F5a: Dimethyl (2R,4R)-2-{[bis(benzyloxy)phosphoryl]methyl}-4-fluoropentanedioate

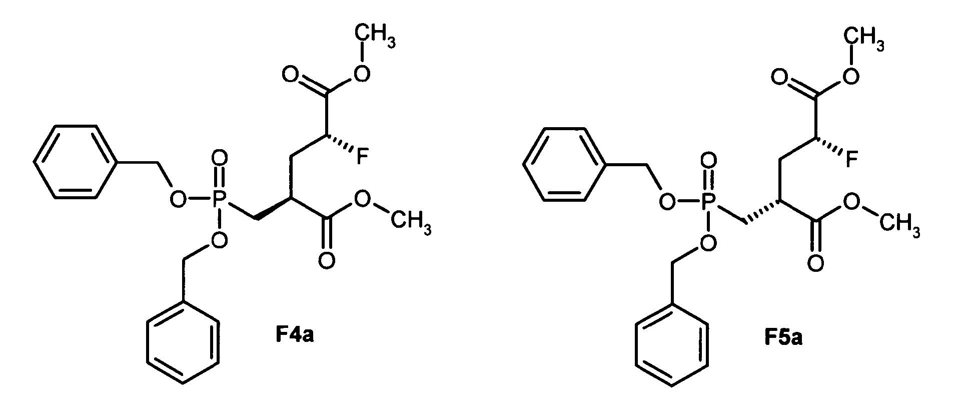

To a solution of Example E3 (500 mg, 2.39 mmol, 91 % purity) in DMF (4.0 mL) was added finely powdered potassium carbonate (325 mesh, 595 mg, 1.80 eq) and dibenzyl phosphite (1.00 g, 1.60 eq), and the resulted mixture was stirred at 50°C for 2 h in a microwave oven. After cooling to room temperature, the mixture was partitioned between EtOAc and 5 % aqueous citric acid, and the organic layer was washed with brine, dried over sodium sulfate, and evaporated. Column chromatography over silica gel (hexane / EtOAc) gave 1.06 g (98 % yield) of F4a and F5a as a mixture of isomers, which was separated by means of chiral HPLC (HPLC method P6) to give examples F4a (480 mg, 44 % yield) and F5a (340 mg, 31 % yield).

### Example F4a

t_{R} (HPLC method A6) = 8.8 min
¹H NMR (300 MHz, CDCl₃) δ ppm 1.86 - 2.02 (m, 1 H) 2.09 - 2.42 (m, 3 H) 2.93 - 3.10 (m, 1 H) 3.58 (s, 3 H) 3.77 (s, 3 H) 4.79 - 5.11 (m, 5 H) 7.27 - 7.44 (m, 10 H).

### Example F5a

t_{R} (HPLC method A6) = 9.4 min
¹H NMR (300 MHz, CDCl₃) δ ppm 1.90 - 2.06 (m, 1 H) 2.11 - 2.39 (m, 3 H) 2.89 - 3.06 (m, 1 H) 3.55 (s, 3 H) 3.76 (s, 3 H) 4.82 - 5.11 (m, 5 H) 7.27 - 7.43 (m, 10 H).

The preparation was adapted to larger scale starting from Intermediate E3 (2.58 g) being reacted with 1.40 eq. of dibenzyl phosphite and 1.60 eq. of potassium carbonate under otherwise unchanged conditions to give examples F4a (2.28 g; t_{R} = 9.1 min, HPLC method A6) and F5a (1.49 g, t_{R} = 10.1 min, HPLC method A6).

### Example F6a: Dimethyl (2S,4S)-2-{[bis(benzyloxy)phosphoryl]methyl} -4-fluoropentanedioate

### Example F7a: Dimethyl (2R,4S)-2-{[bis(benzyloxy)phosphoryl]methyl} -4-fluoropentanedioate

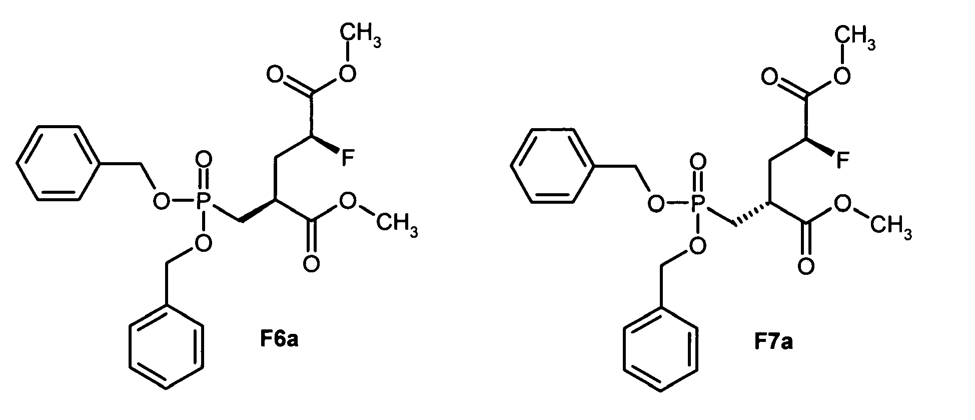

To a solution of Example E2 (500 mg, 2.39 mmol, 91 % purity) in DMF (4.0 mL) was added finely powdered potassium carbonate (325 mesh, 595 mg, 1.80 eq) and dibenzyl phosphite (1.00 g, 1.60 eq), and the resulted mixture was stirred at 50°C for 2 h in a microwave oven. After cooling to room temperature, the mixture was partitioned between EtOAc and 5 % aqueous citric acid, and the organic layer was washed with brine, dried over sodium sulfate, and evaporated. Column chromatography over silica gel (hexane / EtOAc) gave 1.04 g (96 % yield) of F6a and F7a as a mixture of isomers, which was separated by means of chiral HPLC (HPLC method P6) to give examples F6a (340 mg, 31 % yield) and F7a (470 mg, 43 % yield).

### Example F6a

t_{R} = 10.7 min (HPLC Method A6)
t_{R} = 12.9-15.0 min (HPLC method P6, preparative t_{R})
¹H NMR (300 MHz, CDCl₃) δ ppm 1.90 - 2.06 (m, 1 H) 2.11 - 2.39 (m, 3 H) 2.89 - 3.06 (m, 1 H) 3.55 (s, 3 H) 3.76 (s, 3 H) 4.82 - 5.11 (m, 5 H) 7.27 - 7.43 (m, 10 H).

### Example F7a

t_{R} = 7.1 min (HPLC Method A6)
t_{R} = 9.6 - 11.5 min (HPLC method P6, preparative t_{R})
¹H NMR (300 MHz, CDCl₃) δ ppm 1.86 - 2.02 (m, 1 H) 2.09 - 2.42 (m, 3 H) 2.93 - 3.10 (m, 1 H) 3.58 (s, 3 H) 3.77 (s, 3 H) 4.79 - 5.11 (m, 5 H) 7.27 - 7.44 (m, 10 H).

The preparation was adapted to larger scale starting from Intermediate E2 (2.75 g) being reacted with 1.40 eq. of dibenzyl phosphite and 1.60 eq. of potassium carbonate under otherwise unchanged conditions to give examples F6a (2.02 g; t_{R} = 12.4-14.5 min (HPLC method P6, preparative t_{R}); t_{R} = 6.9 min, HPLC method A7) and F7a (3.03 g; t_{R} = 9.4 - 11.2 min (HPLC method P6, preparative t_{R}); t_{R} = 4.4 min, HPLC method A7).

### Example F8: (2R,4S)-2-Fluoro-4-(phosphonomethyl)pentanedioic acid

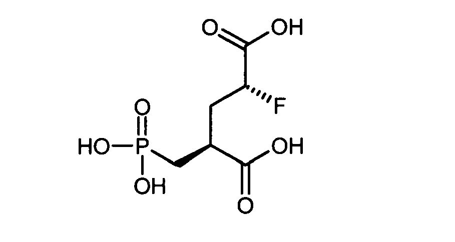

A solution of Example F4 (82 mg, 181 µmol) in a mixture of water (1.7 mL) and trifluoroacetic acid (1.7 mL) was stirred at 100°C for 1.5 h in a microwave oven. The mixture was, after dilution with water and lyophilisation, purified by preparative HPLC (HPLC Method P8) to give Example F8 (15.7 mg, 35 % yield based on gross weight). The compound was not entirely pure, analytical data supported some contamination *inter alia* by a monomethyl ester species.
¹H NMR (600 MHz, d₄-MeOD) δ ppm 1.85 - 1.94 (m, 1 H) 2.16 - 2.34 (m, 3 H) 2.91 - 3.06 (m, 1 H) 4.94 - 5.06 (m, 1 H).
MS (ESI): [M + H]⁺ = 245.

### Example F9: (2R,4R)-2-Fluoro-4-(phosphonomethyl)pentanedioic acid

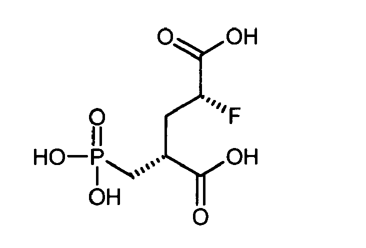

A solution of Example F5 (40 mg, 89 µmol) in a mixture of water (0.8 mL) and trifluoroacetic acid (0.8 mL) was stirred at 100 °C for 1.5 h in a microwave oven. The mixture was, after dilution with water and lyophilisation, purified by preparative HPLC (HPLC Method P8) to give Example F9 (10.6 mg, 49 % yield based on gross weight). The compound was not entirely pure, analytical data supported some contamination *inter alia* by a monomethyl ester species.
¹H NMR (600 MHz, d₄-MeOD) δ ppm 1.91 - 2.00 (m, 1 H) 2.14 - 2.37 (m, 3 H) 2.89 - 2.98 (m, 1 H) 5.06 (ddd, 1 H).
MS (ESI): [M + H]⁺ = 245.

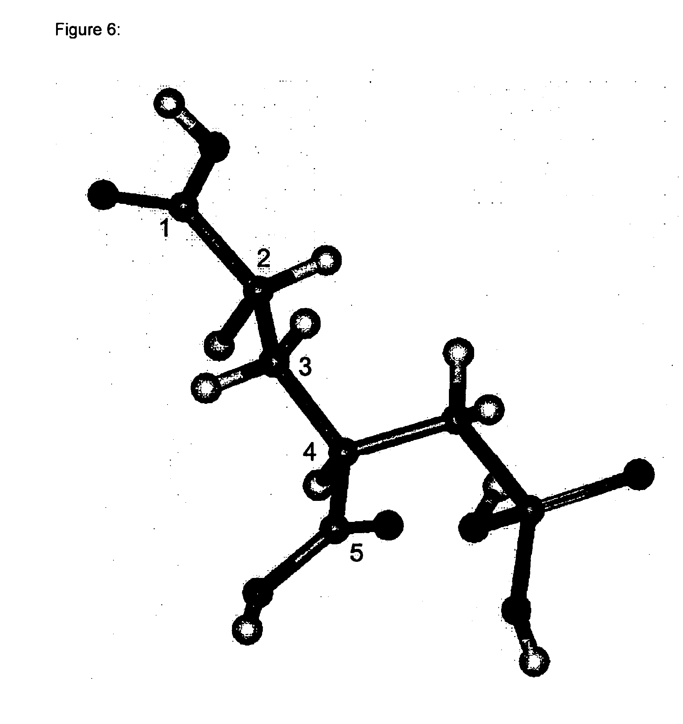

**Example F9a: (2*R*,4*R*)-2-Fluoro-4-(phosphonomethyl)pentanedioic acid (alternative preparation)** 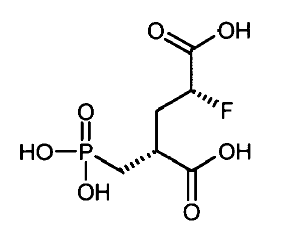 To a solution of Example F5a (340 mg, 752 µmol) in THF (6.5 mL) was added 6 N aqueous hydrochloric acid (6.5 mL) and stirred at 100 °C for 1.5 h in a microwave oven. The mixture was evaporated, and analysed by HNMR. To drive the reaction to completion, the residue was treated with 6 N aqueous hydrochloric acid (6.5 mL) and stirred again at 100 °C for 1.5 h. All volatiles were evaporated and the residue was purified by preparative reversed-phase HPLC to give the target compound (158 mg) which solidified upon standing. An aliquot (20 mg) was subjected to crystallisation from MTB / THF / hexane / toluene to give crystals suitable for X-ray analysis.
¹H NMR (600 MHz, d₆-DMSO) δ ppm 1.69 - 1.79 (m, 1 H), 1.87 - 1.96 (m, 1 H), 2.02 - 2.12 (m, 1 H), 2.14 - 2.25 (m, 1 H), 2.66 - 2.75 (m, 1 H), 5.01 (ddd, 1 H).
MS (ESI): [M + H]⁺ = 245.
The crystals were subjected to X-ray analysis revealing their (2*R*, 4*R*) absolute configuration (Figure 6).

### Example F10: (2S,4S)-2-Fluoro-4-(phosphonomethyl)pentanedioic acid

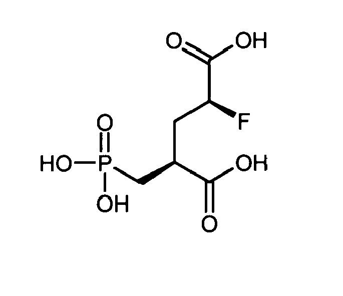

A solution of Example F6 (30 mg, 66 µmol) in a mixture of water (0.6 mL) and trifluoroacetic acid (0.6 mL) was stirred at 100 °C for 1.5 h in a microwave oven. The mixture was, after dilution with water and lyophilisation, purified by preparative HPLC (HPLC Method P8) to give Example F10 (6.0 mg, 37 % yield based on gross weight). The compound was not entirely pure, analytical data supported some contamination *inter alia* by a monomethyl ester species.
¹H NMR (600 MHz, d₄-MeOD) δ ppm 1.86 - 1.94 (m, 1 H) 2.19 - 2.37 (m, 3 H) 2.90 - 2.99 (m, 1 H) 5.06 (ddd, 1 H).
MS (ESI): [M + H]⁺ = 245.

### Example F11: (2S,4R)-2-Fluoro-4-(phosphonomethyl)pentanedioic acid

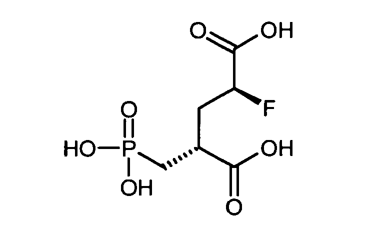

A solution of Example F7 (58 mg, 128 µmol) in a mixture of water (1.2 mL) and trifluoroacetic acid (1.2 mL) was stirred at 100 °C for 1.5 h in a microwave oven. The mixture was, after dilution with water and lyophilisation, purified by preparative HPLC (HPLC Method P8) to give Example F11 (18.5 mg, 59 % yield based on gross weight). The compound was not entirely pure, analytical data supported some contamination *inter alia* by a monomethyl ester species.
¹H NMR (600 MHz, d₄-MeOD) δ ppm 1.85 - 1.94 (m, 1 H) 2.16 - 2.34 (m, 3 H) 2.95 - 3.06 (m, 1 H) 4.95 - 5.06 (m, 1 H).
MS (ESI): [M + H]⁺ = 245.

### Example F12: Dimethyl-(2S,4S)-2-Fluoro-4-(phosphonomethyl)pentanedloate

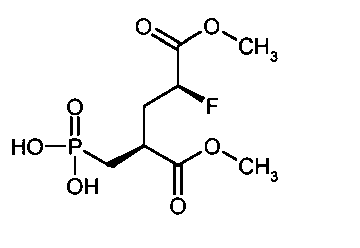

To a solution of Example F6a (310 mg, 686 µmol; from upscaling experiment) in THF (5 mL) was added a 10% palladium on charcoal catalyst (50 mg) and the resulting mixture was stirred at room temperature under an atmosphere of hydrogen for 2 hours. The catalyst was filtered off over a plug of Celite, washed with acetonitrile and all volatiles were removed *in vacuo.* The residue was analysed by HNMR, dissolved in dichloromethane (10 mL) and was cooled to -20 °C for 4 days, whereupon 130 mg of the crystalline product could be obtained. A fraction of the crystalline product was re-dissolved in dichloromethane and was allowed to concentrate slowly at -20 °C to give crystals suitable for x-ray analysis.
¹H NMR (300 MHz, d₆-DMSO) δ ppm 1.67 - 1.96 (m, 2 H), 2.01 - 2.36 (m, 2 H), 2.70 - 2.88 (m, 1 H), 3.57 (s, 3 H); 3.69 (s, 3 H); 5.21 (ddd, 1 H).
No isomeric contamination could be detected as judged by comparison with the respective isomeric mixture prepared from Example F1 as described above.
MS (ESI): [M + H]⁺ = 273.

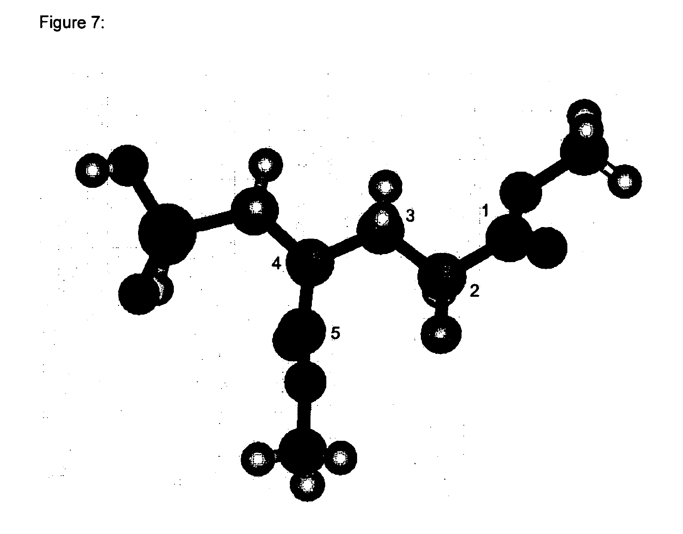

The crystals were subjected to X-ray analysis revealing their (2*S*, 4*S*) absolute configuration (Figure 7).

### Example compounds of the invention (¹⁸F compounds)

### Example F13: (2S,4S)-2-(¹⁸F)-Fluoro-4-(phosphonomethyl)pentanedloic acid

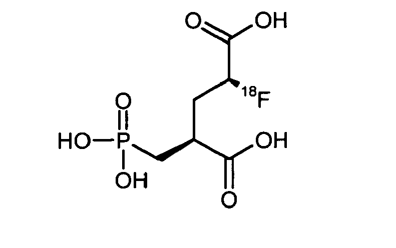

[¹⁸F]Fluoride (18 GBq) was immobilized on a preconditioned QMA (Waters) cartridge (preconditioned by washing the cartridge with 5ml 0.5M K₂CO₃ and 10 ml water), The [F-18]fluoride was eluted using a solution of Cs₂CO₃ (2,3 mg) in 500 µl water and K222 (5 mg) in 1500 µl acetonitrile. This solution was dried at 120°C with stirring under vacuum, with a stream of nitrogen. Additional acetonitrile (1 ml) was added and the drying step was repeated. A solution of Example P9 (4mg) in acetonitrile:amyl alcohol (1:1, 500 µl) was added and heated at 120°C for 15 min. The mixture was diluted with 20ml water and passed through a C18 light SepPak (preconditioned with 5ml ethanol and with 10ml water). The SepPak was washed with 5ml water and eluted with 1ml MeCN. The eluted solution was diluted with 3ml water and purified over prep. HPLC (ACE 5µ C18, 250 x 10mm, isocratic 60% MeCN in 40% water + 0.1%TFA, flow: 4ml/min). The product peak was collected and diluted with 20ml water and passed through a C18 light SepPak (preconditioned with 5ml ethanol and with 10ml water). The SepPak was washed with 5ml water and was eluted with 1ml ethanol. The ethanol solution was dried under gentle N₂-stream for 10min at 90°C. 500µl 4M HCl were added and the mixture was incubated for 10min at 120°C. After cooling the reaction mixture was diluted with 3ml water and purified via prep HPLC (Synergi Hydro RP 250 x 10 mm Phenomenex, water pH 2 (adjusted HCl), flow: 4ml/min) to give the desired product, 340,6 MBq (4,2% d.c.).

### Example F14: (2R,4S)-21(¹⁸F)-Fluoro-4-(phosphonomethyl)pentanedioic acid

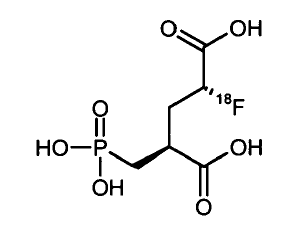

[¹⁸F]Fluoride (27 GBq) was immobilized on a preconditioned QMA (Waters) cartridge (preconditioned by washing the cartridge with 5ml 0.5M K₂CO₃ and 10 ml water), The [F-18]fluoride was eluted using a solution of Cs₂CO₃ (2,3 mg) in 500 µl water and K222 (5 mg) in 1500 µl acetonitrile. This solution was dried at 120°C with stirring under vacuum, with a stream of nitrogen. Additional acetonitrile (1 ml) was added and the drying step was repeated. A solution of Example P7 (4mg) in acetonitrile:amyl alcohol (1:1, 500 pl) was added and heated at 120°C for 15 min. The mixture was diluted with 20ml water and passed through a C18 light SepPak (preconditioned with 5ml ethanol and with 10ml water). The SepPak was washed with 5ml water and eluted with 1ml MeCN. The eluted solution was diluted with 3ml water and purified over prep. HPLC (ACE 5µ C18, 250 x 10mm, isocratic 60% MeCN in 40% water + 0.1%TFA, flow: 4ml/min). The product peak was collected and diluted with 20ml water and passed through a C18 light SepPak (preconditioned with 5ml ethanol and with 10ml water). The SepPak was washed with 5ml water and was eluted with 1ml ethanol. The ethanol solution was dried under gentle N₂-stream for 10min at 90°C. 500µl 4M HCl were added and the mixture was incubated for 10min at 120°C. After cooling the reaction mixture was diluted with 3ml water and purified via prep HPLC (Synergi Hydro RP 250 x 10 mm Phenomenex, water pH 2 (adjusted HCI), flow: 4ml/min) to give the desired product, 340,6 MBq (3,8% d.c.).

### Example F15: rac-2-(¹⁸F)-Fluoro-4-(phosphonomethyl)pentanedioic acid

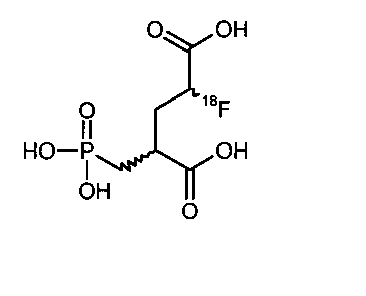

[¹⁸F]Fluoride (21,8 GBq) was immobilized on a preconditioned QMA (Waters) cartridge (preconditioned by washing the cartridge with 5ml 0.5M K₂COₛ and 10 ml water), The [F-18]fluoride was eluted using a solution of Cs₂CO₃ (2,3 mg) in 500 µl water and K222 (5 mg) in 1500 µl acetonitrile. This solution was dried at 120°C with stirring under vacuum, with a stream of nitrogen. Additional acetonitrile (1 ml) was added and the drying step was repeated. A solution of Example P4a (4mg) in acetonitrile:amyl alcohol (1:1, 500 µl) was added and heated at 120°C for 15 min. The mixture was diluted with 20ml water and passed through a C18 light SepPak (preconditioned with 5ml ethanol and with 10ml water). The SepPak was washed with 5ml water and eluted with 1ml MeCN. The eluted solution was diluted with 3ml water and purified over prep. HPLC (ACE 5µ C18, 250 x 10mm, isocratic 60% MeCN in 40% water + 0.1%TFA, flow: 4ml/min). The product peak was collected and diluted with 20ml water and passed through a C18 light SepPak (preconditioned with 5ml ethanol and with 10ml water). The SepPak was washed with 5ml water and was eluted with 1ml ethanol. The ethanol solution was dried under gentle N₂-stream for 10min at 90°C. 500µl 4M HCI were added and the mixture was incubated for 10min at 120°C. After cooling the reaction mixture was diluted with 3ml water and purified via prep HPLC (Synergi Hydro RP 250 x 10 mm Phenomenex, water pH 2 (adjusted HCl), flow: 4ml/min) to give the desired product, 454,5 MBq (5,3% d.c.).

### Example F15a: rac-2-(¹⁸F)-Fluoro-4-(phosphonomethyl)pentanedioic acid (modified process)

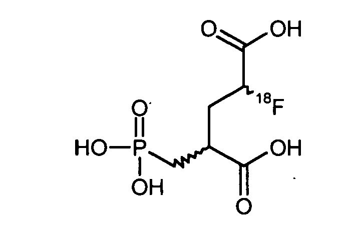

[¹⁸F]Fluoride (4,6 GBq) was immobilized on a preconditioned QMA (Waters) cartridge (preconditioned by washing the cartridge with 5ml 0.5M K₂CO₃ and 10 ml water), The [F-18]fluoride was eluted using a solution of Cs₂CO₃ (2,3 mg) in 500 µl water and K222 (5 mg) in 1500 µl acetonitrile. This solution was dried at 120°C with stirring under vacuum, with a stream of nitrogen. Additional acetonitrile (1 ml) was added and the drying step was repeated. A solution of Example P4a (4mg) in DMSO (500 µl) was added and heated at 120°C for 15 min. The mixture was diluted with 20ml water and passed through a C18 light SepPak (preconditioned with 5ml ethanol and with 10ml water). The SepPak was washed with 5ml water and eluted with 1ml MeCN. The eluted solution was diluted with 3ml water and purified over prep. HPLC (ACE 5µ C18, 250 x 10mm, isocratic 60% MeCN in 40% water + 0.1 %TFA, flow: 4ml/min). The product peak was collected and diluted with 20ml water and passed through a C18 light SepPak (preconditioned with 5ml ethanol and with 10ml water). The SepPak was washed with 5ml water and was eluted with 1ml ethanol. The ethanol solution was dried under gentle N₂-stream for 10min at 90°C. 1000µl 6M HCl were added and the mixture was incubated for 15min at 120°C. After cooling the reaction mixture was diluted with 1ml water and passed through a cartridge containing AG11A8 resin (ion retardation resin, -20g) connected in series with a C18 light SepPak (preconditioned with 5ml ethanol and with 10ml water), the cartridges were then washed with saline (5ml) and eluents were collected to give the desired product, 430 MBq (25% d.c.).

### Biology Example 1

### In vitro inhibition of NAALADase activity

Since PSMA (FOLH1) is a membrane bound enzyme with NAALADase activity (reference Robinson et al. Journal of Biological Chemistry, Vol. 262, No. 30, Issue of October 25, pp. 14498-14506.(1987)) and strongly expressed in LNCaP cells (Troyer et al. International Journal of Cancer 62, 552-558(1995)), various compounds described in this application were tested for NAALADase inhibition in vitro using LNCaP cell extracts. NAALADase activity was assayed essentially as described previously (Slusher BS, Tsai G, Yoo G, Coyle JT. Immunocytochemical localization of the N-acetyl-aspartyl-glutamate hydrolyzing enzyme N-acetylated alpha-linked acidic dipeptidase [NAALADase].J Comp Neurol 1992;315:217-229.) in crude membrane extracts from LNCaP tissue culture cells (ref ATCC CRL-1740). Briefly, the release of [3H]-Glutamate from [3H] N-acetyl-aspartyl-glutamate (NAAG) in 50 mM Tris-Cl buffer pH7.4 after 30 min at 37° C was measured using 1-5 µg/ml of protein extracted from the membrane fraction of LNCaP cells; substrate and product were resolved by cation-exchange liquid chromatography. 2-PMPA, a well known potent inhibitor of PSMA, was included in a parallel assay tube to confirm the specificity of measurements. IC50 values were determined by incubating the assay reagents in the presence of increasing concentrations of various compounds described in this application (results see Table 1)

**Table 1: IC50 of compounds with respect to NAALADase activity.**

| Examples | IC50 |
|---|---|
| F3 | 30 nM |
| F10 | 7,0 nM |
| F8 | 14 nM |
| 2-PMPA | 1,6 nM |

### Biology Example 2

### PET/CT-imaging in LNCaP-tumor bearing mice of Example F15a

### Compound F15a

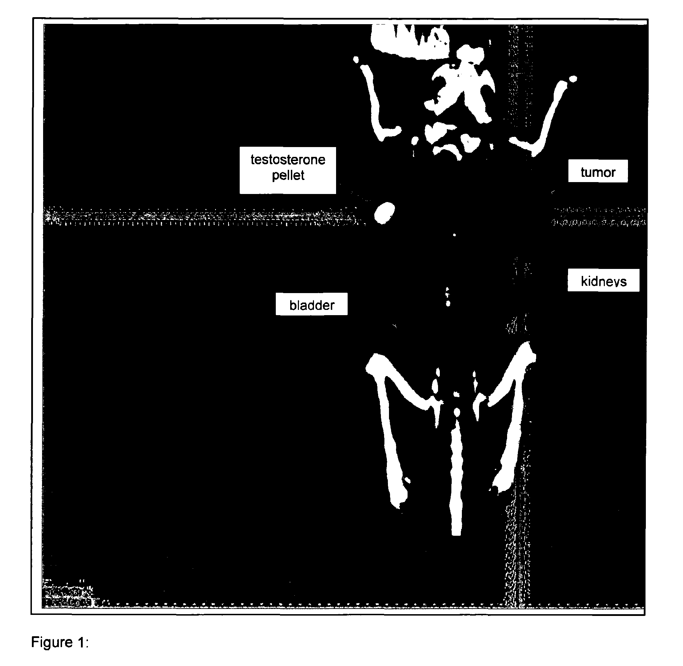

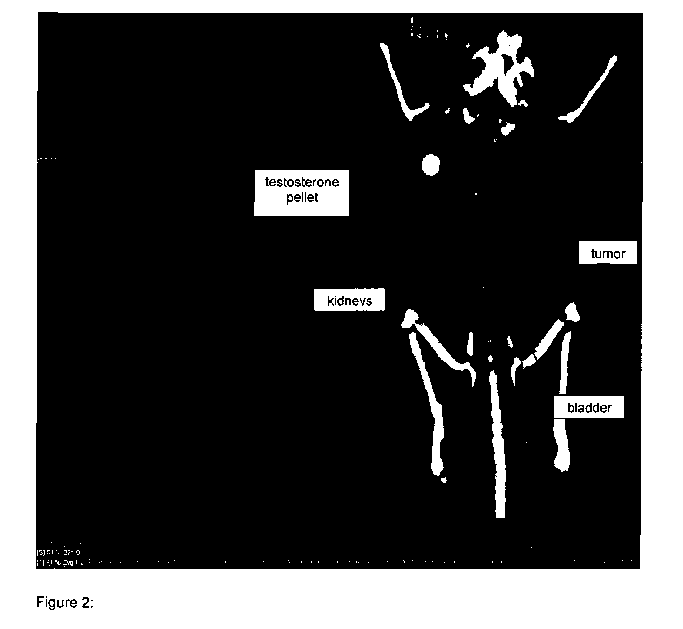

An isomeric mixture of F-PMPA (Example F15a was imaged on a microPET/ CT (Inveon, Siemens) in LNCaP tumor-bearing mice 110-130min after injection of approximately - 10 MBq radiotracer Example F15a . Due to the rapid renal clearance of this PSMA ligand very low background activity was observed except for strong kidney and bladder uptake. High tumor-contrast visible in LNCaP xenografts was effectively blocked by 22 µg of the corresponding non-radioactive Example F3 (Reference to Figures 1 and 2).

### Biology Example 3

### PET/CT-imaging in LNCaP-tumor bearing mice Example F13

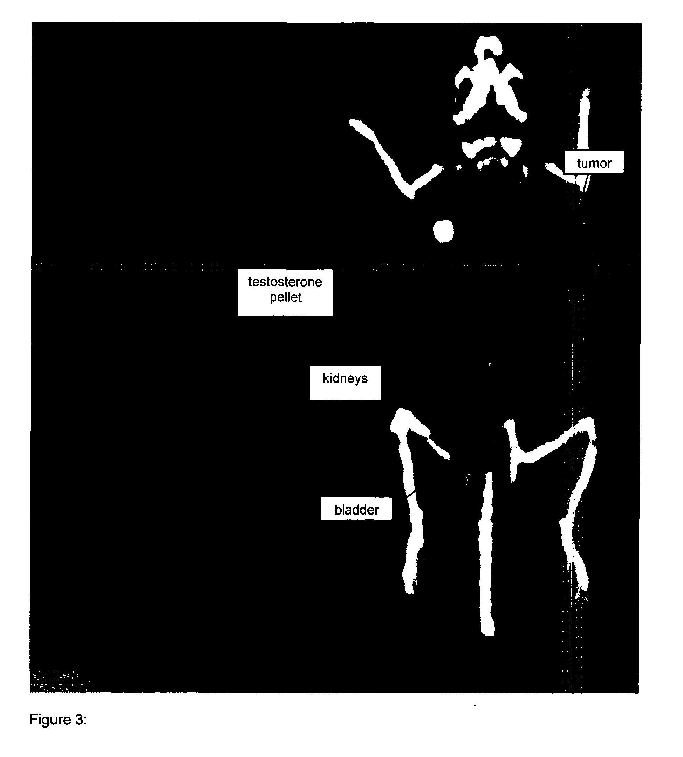

Example F13 was imaged on a microPET/ CT (Inveon, Siemens) in LNCaP tumor-bearing mice 50-70min after injection of ∼ 10 MBq radiotracer Example F13. High tumor-contrast was visible in LNCaP xenografts. Due to rapid renal clearance of this PSMA ligand very low background activity was observed except for strong kidney and bladder uptake (Reference to Figure 3).

### Biology Example 4

### PET/CT-imaging in LNCaP-tumor bearing mice of Example F14

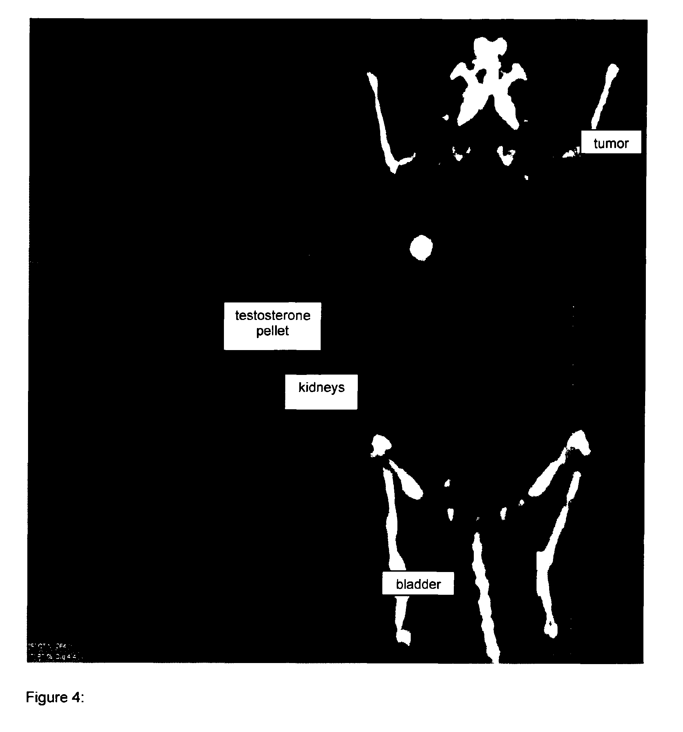

Example F14 was imaged on a microPET/ CT (Inveon, Siemens) in LNCaP tumor-bearing mice 50-70min after injection of - 10 MBq radiotracer Example F14. High tumor-contrast was visible in LNCaP xenografts. Due to rapid renal clearance of this PSMA ligand very low background activity was observed except for strong kidney and bladder uptake (Reference to Figure 4).

### Biology Example 5

### Biodistribution of Example F13

Male nude mice were implanted subcutaneously with 10 million LNCaP tumor cells, which were freshly expanded in a sterilized solution phosphate-buffered saline (PBS, pH 7.4). 4 weeks after inoculation the mice were injected into the tail vein with 150kBq of radiolabeled Example F13, diluted in PBS (total injected volume 100µl). At 30, 60, 120, and 180 min intervals, the mice (in groups of 3) were sacrificed and the organs of interest were collected, rinsed of excess blood, weighed and counted in a gamma-counter. Results are presented in Table 2.

**Table 2: Biodistribution of Example F13 in LNCaP bearing nude mice.**

| **Zeitpunkt/ timepont** | **0,5** h | | **1,0** h | | **2,0 h** | | **3,0 h** | |
|---|---|---|---|---|---|---|---|---|
| **Gewicht/weight(g):** | | **22,86** | | **21,67** | | **22,16** | | **19,96** |
| | | | | | | | | |
| **%Dosis/g** | S.D. | | S.D. | | S.D. | | S D. | |
| **Milz/ spleen** | **3,00** | 0,48 | **2,48** | 0,26 | **0,42** | 0,19 | **0,41** | 0,06 |
| **Leber/liver** | **0,40** | 0,06 | **0,42** | 0,06 | **0,24** | 0,04 | **0,35** | 0,07 |
| **Nlere/kidney** | **105,78** | 24,49 | **105,40** | 6,10 | **41,15** | 16,38 | **46,96** | 23,92 |
| **Lunge/lung** | **1.12** | 0,04 | **0,49** | 0,22 | **0,17** | 0,07 | **0,16** | 0,03 |
| **Knochen/bone** | **2,61** | 0,35 | **2,53** | 0,26 | **1,52** | 0,33 | **2,12** | 0,48 |
| **Herz/heart** | **0,53** | 0,01 | **0,27** | 0,06 | **0,06** | 0,02 | **0,08** | 0,01 |
| **Him/brain** | **0,05** | 0,01 | **0,06** | 0,02 | **0,04** | 0,02 | **0,03** | 001 |
| **Fett/fat** | **0,78** | 0,15 | **0,36** | 0,08 | **0,17** | 0,09 | **0,35** | 0,17 |
| S**childdrüse/thyroid** | **0,68** | 0,13 | **0,45** | 0,11 | **0,18** | 0,04 | **0,30** | 0,07 |
| **Hoden./tastes** | **0,76** | 0,19 | **0,50** | 0,02 | **0,15** | 0,01 | **0,20** | 0,01 |
| **Muskel/muscle** | **0,26** | 0,14 | **0,21** | 0,05 | **0,12** | 0,12 | **0,06** | 0,02 |
| ***Tumor*/*tumor*** | ***5,31*** | *1,83* | ***4,08*** | *1,24* | ***3,13*** | *0.54* | ***4,54*** | *0,93* |
| **Haut/skin** | **0,85** | 0,01 | **0,76** | 0,20 | **0,29** | 0,06 | **0,24** | 0,04 |
| **Blut/blood** | **0,79** | 0,05 | **0,33** | 0,12 | **0,07** | 0,02 | **0.08** | 0.02 |
| **Schwanz/tail** | **4,69** | 1,55 | **2,37** | 1,01 | **1,73** | 0,09 | **2,52** | 0.68 |
| **Magen/stomach** | **0,51** | 0,05 | **0,26** | 0,06 | **0,11** | 0,03 | **0,20** | 0,05 |
| **Prostata/prostate** | **0,69** | 0,26 | **0,70** | 0,15 | **0,66** | 0,95 | **0,53** | 0,29 |
| **Darm/intestine** | **0,23** | 0,01 | **0,23** | 0,08 | **0,28** | 0,16 | **1,03** | 0,80 |
| **Beuchspdr./pankreas** | **0,45** | 0,10 | **0,22** | 0,04 | **0,07** | 0,01 | **0,09** | 0,02 |
| **Nebenn./adrenals** | **3,31** | 0,46 | **2,46** | 0,36 | **0,51** | 0,31 | **0,573** | 0.17 |

### Biology Example 6

### Stability in human plasma of Example F13

Stability of Example F13 was investigated in human plasma in vitro. The Example was analyzed by TLC at different time points. Example F13 did not show increased release of free fluoride upon incubation in human plasma. After two hours, 82% of Example F13 was still intact.

### Biology Example 7

### Metabolic Stability of Example F13

Metabolic stability of Example F13 was assessed by incubating F13 in the presence of mouse, rat, and human microsomes and rat hepatocytes. After different timepoints TLC analysis was performed. No metabolism by microsomes or hepatocytes was found.

## Claims

1. A compound of the formula I 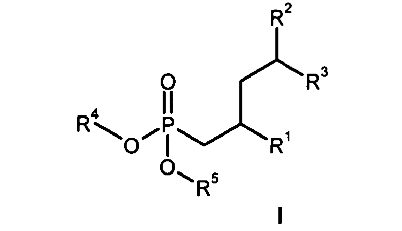 wherein
R¹ is C(=O)OR⁶;
R² is C(=O)OR⁷, or 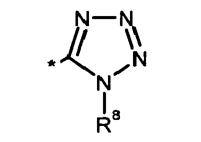 wherein the asterisk indicates the point of attachment to formula I;
R³ is selected from the group comprising ¹⁹F, ¹⁸F, and LG,
wherein LG is an appropriate leaving group, selected from the group comprising chloro, bromo, iodo, and -OS(=O)₂R⁹;
R⁴ and R⁵ are selected independently from each other from the group comprising hydrogen, optionally substituted C₁-C₁₀-alkyl, optionally substituted C₃-C₇-cydoalkyl, optionally substituted C₆-C₁₀-aryl, and optionally substituted C₇-C₁₄-arylalkyl, wherein zero, one or two of the carbon atoms constituting said alkyl group, cycloalkyl group, or the alkyl portion of said arylalkyl group, is optionally replaced by -C(=O)-, -NR¹⁰-, or -O-, or
R⁴ and R⁵ together form an optionally substituted C₂-C₆ alkylene tether;
R⁶ and R⁷ are selected independently from each other from the group comprising hydrogen, optionally substituted C₁-C₁₀-alkyl, optionally substituted C₃-C₇-cycloalkyl, optionally substituted C₆-C₁₀-aryl, and optionally substituted C₇-C₁₄-arylalkyl, wherein zero, one or two of the carbon atoms constituting said alkyl group, cycloalkyl group, or the alkyl portion of said arylalkyl group, is optionally replaced by -C(=O)-, -NR¹⁰-, or -O-;
R⁸ is hydrogen, benzyl or triphenylmethyl;
R⁹ is selected from the group comprising C₁-C₄-alkyl, C₁-C₄-haloalkyl, and phenyl, wherein alkyl and phenyl are optionally substituted by one or multiple groups, selected independently from each other, from the group comprising C₁-C₄-alkyl, C₁-C₄-haloalkyl, C₁-C₄-alkoxy, halo, cyano, and nitro;
R¹⁰ is selected from the group comprising hydrogen, C₁-C₄-alkyl, and acetyl;
and stereoisomers, stereoisomeric mixtures, and suitable salts thereof.

2. The compound according to claim 1 wherein
R¹ is C(=O)OR⁶_{;}
R² is C(=O)OR⁷;
R³ is selected from the group comprising ¹⁹F, ¹⁸F, and -OS(=O)₂R⁹;
R⁴ and R⁵ are selected independently from each other from the group comprising hydrogen, optionally substituted C₁-C₈-alkyl, and optionally substituted C₇-C₁₀-arylalkyl;
R⁶ and R⁷ are selected independently from each other from the group comprising hydrogen, optionally substituted C₁-C₈-alkyl, and optionally substituted C₇-C₁₀-arylalkyl; and
R⁹ is selected from the group comprising C₁-C₄-alkyl and phenyl, wherein phenyl is optionally substituted by one or two groups, selected independently from each other, from the group comprising C₁-C₄ alkyl, C₁-C₄-alkoxy, halo, and nitro.

3. The compound according to claims 1 or 2 wherein R³ is ¹⁸F that corresponds to compound of formula **I-F18.**

4. The compound according to claim 3 (2*S*,4*S*)-2-(¹⁸F)-Fluoro-4-(phosphonomethyl)pentanedioic acid and (2*R*,4*S*)-2-(¹⁸F)-Fluoro-4-(phosphonomethyl)pentanedioic acid,
and mixtures and suitable salts thereof.

5. The compound according to claims 1 or 2 wherein R³ is ¹⁹F that corresponds to compound of formula **I-F19**.

6. The compound according to claim 5
(2*S*,4*S*)-2-Fluoro-4-(phosphonomethyl)pentanedioic acid and
(2*R*,4*S*)-2-Fluoro-4-(phosphonomethyl)pentanedioic acid,
and mixtures thereof and suitable salts thereof.

7. The compounds according to claims 1 or 2 wherein R³ is -OS(=O)₂R⁹;
R⁹ is selected from the group comprising C₁-C₄-alkyl and phenyl, wherein phenyl is optionally substituted by one or two groups, selected independently from each other, from the group comprising C₁-C₄ alkyl, C₁-C₄-alkoxy, halo, and nitro.

8. The compound according to claim 7
Dimethyl (2*S*,4*S*)-2-{[bis(benzyloxy)phosphoryl]methyl}-4-(tosyloxy)pentanedioate and Dimethyl (2*S*,4*R*)-2-{[bis(benzyloxy)phosphoryl]methyl}-4-(tosyloxy)pentanedioate, and mixtures thereof.

9. A composition comprising compounds of formula 1, I-F18, I-F19, or I-LG according to claims 1 to 8 or mixtures thereof and a pharmaceutically acceptable carrier or diluent.

10. A method for obtaining compounds of formula I, I-F18, I-F19, or I-LG according to claims 1 to 8 or mixtures thereof.

11. The method according to claim 10 for obtaining compounds of formula I-F18 according to claim 3 comprises the steps
- Coupling compound of Formula I-LG according to claim 7 with a Fluorine atom (F) containing moiety wherein the Fluorine atom (F) containing moiety comprises ¹⁸F;
- Optionally deprotecting compound of formula I-F18 and/or
- Optionally converting obtained compound into a suitable salts of inorganic or organic bases thereof, hydrates, complexes, and solvates thereof.

12. The method according to claim 10 for obtaining compounds of formula I-F18 according to claim 3 comprises the steps
- Coupling compound of Formula X with a Fluorine atom (F) containing moiety wherein the Fluorine atom (F) containing moiety comprises ¹⁸F for obtaining a compound of formula **X-F18**
wherein 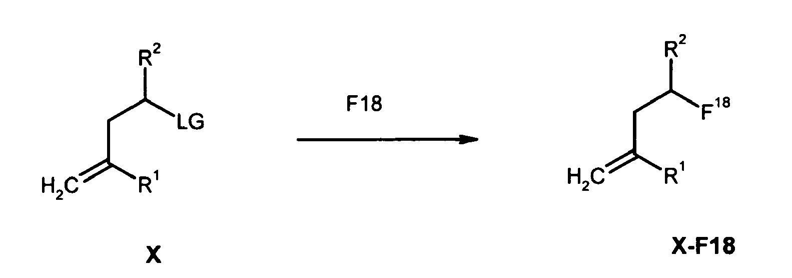 wherein
R¹ is C(=O)OR⁶;
R² is selected from the group comprising
C(=O)OR⁷, or 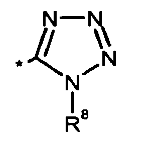 wherein the asterisk indicates the point of attachment to formula X and X-F18;
R⁶ and R⁷ are selected independently from each other from the group comprising hydrogen, optionally substituted C₁-C₁₀-alkyl, optionally substituted C₃-C₇-cycloalkyl, optionally substituted C₆-C₁₀-aryl, or optionally substituted C₇-C₁₄-arylalkyl, wherein zero, one or two of the carbon atoms constituting said alkyl group, cycloalkyl group, or the alkyl portion of said arylalkyl group, is optionally replaced by -C(=O)-, -NR¹⁰-, or -O-;
R⁸ is selected from the group comprising hydrogen, benzyl or triphenylmethyl; LG is an appropriate leaving group, selected from the group comprising chloro, bromo, iodo, and -OS(=O)₂R⁹;
R⁹ is selected from the group comprising C₁-C₄-alkyl, C₁-C₄-haloalkyl, and phenyl, wherein alkyl and phenyl are optionally substituted by one or multiple groups, selected independently from each other, from the group comprising C₁-C₄-alkyl, C₁-C₄-haloalkyl, C₁-C₄-alkoxy, halo, cyano, and nitro;
R¹⁰ is selected from the group comprising hydrogen, C₁-C₄-alkyl, and acetyl;
- Coupling a compound of Formula X-F18 with a compound of formula XI for obtaining a compound of formula I-F18 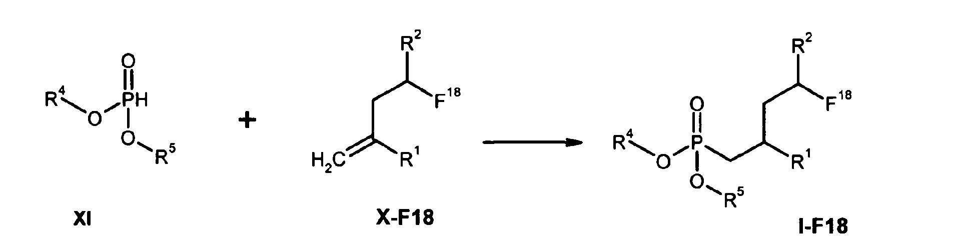 wherein
R¹ is C(=O)OR⁶;
R² is selected from the group comprising
C(=O)OR⁷, or 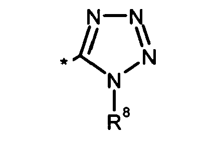 wherein the asterisk indicates the point of attachment to formula X-F18 and IF18;
R⁴ and R⁵ are selected independently from each other from the group comprising hydrogen, optionally substituted C₁-C₁₀-alkyl, optionally substituted C₃-C₇-cydoalkyl, optionally substituted C₆-C₁₀-aryl, and optionally substituted C₇-C₁₄-arylalkyl, wherein zero, one or two of the carbon atoms constituting said alkyl group, cycloalkyl group, or the alkyl portion of said arylalkyl group, is optionally replaced by -C(=O)-, -NR¹⁰-, or -O-, or
R⁴ and R⁵ together form an optionally substituted C₂-C₆ alkylene tether;
R⁶ and R⁷ are selected independently from each other from the group comprising hydrogen, optionally substituted C₁-C₁₀-alkyl, optionally substituted C₃-C₇-cycloalkyl, optionally substituted C₆-C₁₀-ryl, or optionally substituted C₇-C₁₄-arylalkyl, wherein zero, one or two of the carbon atoms constituting said alkyl group, cycloalkyl group, or the alkyl portion of said arylalkyl group, is optionally replaced by -C(=O)-, -NR¹⁰-, or -O-;
R⁸ is selected from the group comprising hydrogen, benzyl or triphenylmethyl;
R¹⁰ is selected from the group comprising hydrogen, C₁-C₄-alkyl, and acetyl;
- Optionally deprotecting compound of formula I-F18 and/or
- Optionally converting obtained compound into a suitable salts of inorganic or organic bases thereof, hydrates, complexes, and solvates thereof.

13. The method according to claim 10 for obtaining compounds of formula I-F19 according to claim 5 comprises the steps
- Reacting a compound of formula I-R11 with a Fluorine atom (F) containing moiety wherein the Fluorine atom (F) containing moiety comprises ¹⁹F;
- Optionally deprotecting compound of formula I-F19 and/or
- Optionally converting obtained compound into suitable salts of inorganic or organic bases thereof, hydrates, complexes, and solvates thereof.

14. The method according to claim 10 for obtaining compounds of formula I-F19 according to claim 5 comprises the steps
- Reacting compound of Formula XII with a Fluorine atom (F) containing moiety wherein the Fluorine atom (F) containing moiety comprises ¹⁹F for obtaining a compound of formula X-F19
wherein 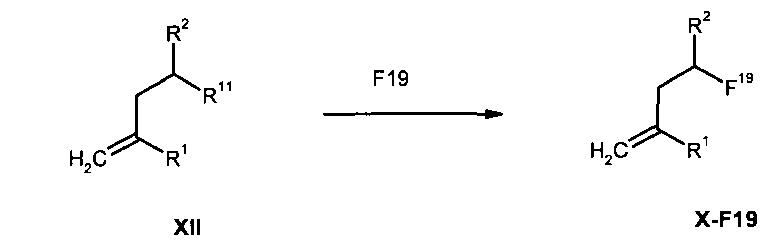 wherein
R¹ is C(=O)OR⁶;
R² is C(=O)OR⁷, or 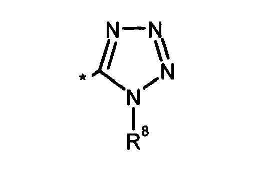 wherein the asterisk indicates the point of attachment into formula XII and X-F19;
R⁶ and R⁷ are selected independently from each other from the group comprising hydrogen, optionally substituted C₁-C₁₀-alkyl, optionally substituted C₃-C₇-cycloalkyl, optionally substituted C₆-C₁₀-aryl, and optionally substituted C₇-C₁₄-arylalkyl, wherein zero, one or two of the carbon atoms constituting said alkyl group, cycloalkyl group, or the alkyl portion of said arylalkyl group, is optionally replaced by -C(=O)-, -NR¹⁰-, or -O-;
R⁸ is selected from the group comprising hydrogen, benzyl, or triphenylmethyl;
R⁹ is selected from the group comprising C₁-C₄-alkyl, C₁-C₄-haloalkyl, and phenyl, wherein alkyl and phenyl are optionally substituted by one or multiple groups, selected independently from each other, from the group comprising C₁-C₄-alkyl, C₁-C₄-haloalkyl, C₁-C₄-alkoxy, halo, cyano, and nitro;
R¹⁰ is selected from the group comprising hydrogen, C₁-C₄-alkyl, and acetyl,;
R¹¹ is OH or OS(=O)2R⁹,
- Coupling a compound of Formula X-F19 with a compound of formula XI for obtaining a compound of formula I-F19
wherein 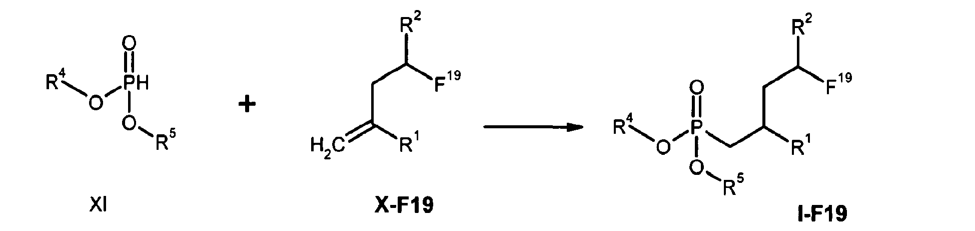 wherein
R¹ is C(=O)OR⁶;
R² is C(=O)OR⁷, or 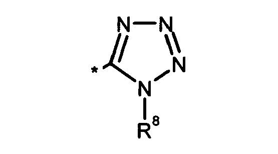 wherein the asterisk indicates the point of attachment to formula X-F19 and IF19;
R⁴ and R⁵ are selected independently from each other from the group comprising hydrogen, optionally substituted C₁-C₁₀-alkyl, optionally substituted C₃-C₇-cycloalkyl, optionally substituted C₆-C₁₀-aryl, and optionally substituted C₇-C₁₄-arylalkyl, wherein zero, one or two of the carbon atoms constituting said alkyl group, cycloalkyl group, or the alkyl portion of said arylalkyl group, is optionally replaced by -C(=O)-, -NR¹⁰-, or -O-, or,
R⁴ and R⁵ together form an optionally substituted C₂-C₆ alkylene tether;
R⁶ and R⁷ are selected independently from each other from the group comprising hydrogen, optionally substituted C₁-C₁₀-alkyl, optionally substituted C₃-C₇-cycloalkyl, optionally substituted C₆-C₁₀-aryl, and optionally substituted C₇-C₁₄-arylalkyl, wherein zero, one or two of the carbon atoms constituting said alkyl group, cycloalkyl group, or the alkyl portion of said arylalkyl group, is optionally replaced by -C(=O)-, -NR¹⁰-, or -O-;
R⁸ is selected from the group comprising hydrogen, benzyl, or triphenylmethyl;
R¹⁰ is selected from the group comprising hydrogen, C₁-C₄-alkyl, and acetyl;
- Optionally deprotecting compound of formula I-F19 and/or
- Optionally converting obtained compound into a suitable salts of inorganic or organic bases thereof, hydrates, complexes, and solvates thereof.

15. The method according to claim 10 for obtaining compounds of formula I-LG according to claim 7 comprises the steps
- Coupling compound of Formula I-R12 with an agent suitable for conversion of R¹² into an LG moiety as defined *supra,* such as an appropriate sulfonyl halide, sulfonyl anhydride (for the introduction of OS-(=O)₂R⁹), or a combination of phosphane, such as triphenyl phosphane, and a carbon tetrahalide, such as tetrabromomethane (for the introduction of chloro, bromo, and iodo). 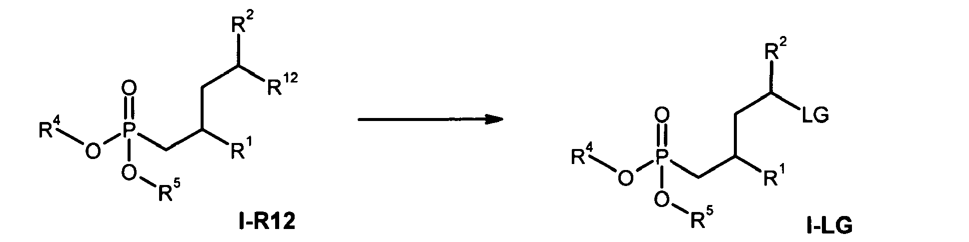 wherein
R¹ is C(=O)OR⁶;
R² is C(=O)OR⁷, or 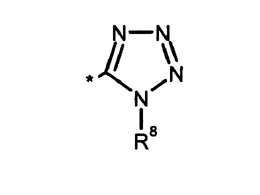 wherein the asterisk indicates the point of attachment to formula I-R12 and I-LG; R¹² is OH, 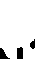 LG is an appropriate leaving group, selected from the group comprising chloro, bromo,
iodo, and -OS(=O)₂R⁹,
R⁴ and R⁵ are selected independently from each other from the group comprising
hydrogen, optionally substituted C₁-C₁₀-alkyl, optionally substituted C₃-C₇-cycloalkyl, optionally substituted C₆-C₁₀-aryl, and optionally substituted C₇-C₁₄-arylalkyl, wherein zero, one or two of the carbon atoms constituting said alkyl group, cycloalkyl group, or the alkyl portion of said arylalkyl group, is optionally replaced by -C(=O)-, -NR¹⁰-, or -O-,
or R⁴ and R⁵ together form an optionally substituted C₂-C₆ alkylene tether;
R⁶ and R⁷ are selected independently from each other, from the group comprising
hydrogen, optionally substituted C₁-C₁₀-alkyl, optionally substituted C₃-C₇-cycloalkyl, optionally substituted C₆-C₁₀-aryl, and optionally substituted C₇-C₁₄-arylalkyl, wherein zero, one or two of the carbon atoms constituting said alkyl group, cycloalkyl group, or the alkyl portion of said arylalkyl group, is optionally replaced by -C(=O)-, -NR¹⁰-, or -O-;
R⁸ is selected from the group comprising hydrogen, benzyl, or triphenylmethyl;
R⁹ is selected from the group comprising C₁-C₄-alkyl, C₁-C₄-haloalkyl, and phenyl,
wherein alkyl and phenyl are optionally substituted by one ore multiple groups, selected independently from each other, from the group comprising C₁-C₄-alkyl, C₁-C₄-haloalkyl, C₁-C₄-alkoxy, halo, cyano, and nitro;
R¹⁰ is selected from the group comprising hydrogen, C₁-C₄-alkyl, and acetyl;
and stereoisomers, stereoisomeric mixtures, and suitable salts thereof,
- Optionally deprotecting compound of formula I-LG and/or
- Optionally converting obtained compound into a suitable salts of inorganic or organic acids thereof, hydrates, complexes, esters, amides, and solvates thereof.

16. The method according to claim 10 for obtaining compounds of formula I-LG according to claim 7 comprises the steps
- Coupling a compound of Formula XI with a compound of formula X-LG for obtaining a compound of formula I-LG 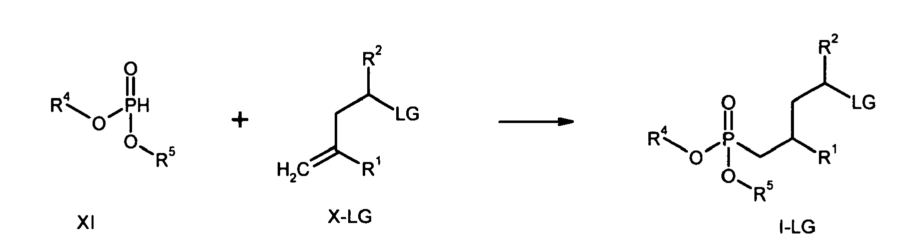 wherein
R¹ is C(=O)OR⁶ ;
R² is selected from the group comprising C(=O)OR⁷, or 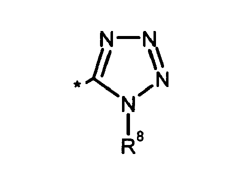 wherein the asterisk indicates the point of attachment to formula X-LG and I-LG;
LG is an appropriate leaving group, selected from the group comprising chloro, bromo, iodo, and -OS(=O)₂R⁹;
R⁴ and R⁵ are selected independently from each other from the group comprising hydrogen, optionally substituted C₁-C₁₀-alkyl, optionally substituted C₃-C₇-cycloalkyl, optionally substituted C₆-C₁₀-aryl, and optionally substituted C₇-C₁₄-arylalkyl, wherein zero, one or two of the carbon atoms constituting said alkyl group, cycloalkyl group, or the alkyl portion of said arylalkyl group, is optionally replaced by -C(=O)-, -NR¹⁰-, or -O- or
R⁴ and R⁵ together form an optionally substituted C₂-C₆ alkylene tether;
R⁶ and R⁷ are selected independently from each other, from the group comprising hydrogen, optionally substituted C₁-C₁₀-alkyl, optionally substituted C₃-C₇-cycloalkyl, optionally substituted C₆-C₁₀-aryl, and optionally substituted C₇-C₁₄-arylalkyl, wherein zero, one or two of the carbon atoms constituting said alkyl group, cycloalkyl group, or the alkyl portion of said arylalkyl group, is optionally replaced by -C(=O)-, -NR¹⁰-, or -O-;
R⁸ is selected from the group comprising hydrogen, benzyl, or triphenylmethyl; R⁹ is selected from the group comprising C₁-C₄-alkyl, C₁-C₄-haloalkyl, and phenyl, wherein alkyl and phenyl are optionally substituted by one or multiple groups, selected independently from each other, from the group comprising C₁-C₄-alkyl, C₁-C₄-haloalkyl, C₁-C₄-alkoxy, halo, cyano, and nitro;
R¹⁰ is selected from the group comprising hydrogen, C₁-C₄-alkyl, and acetyl;
- Optionally deprotecting compound of formula I-LG and/or
- Optionally converting obtained compound into a suitable salts of inorganic or organic acids thereof, hydrates, complexes, and solvates thereof.

17. A compound of general formula I or I-F18 according to claims 1 to 3 or mixture thereof for the manufacture of an imaging tracer for imaging diseases associated with altered expression of Prostate Specific Membrane Antigen PSMA, preferably elevated expression of Prostate Specific Membrane Antigen PSMA.

18. Use of compounds of general formula I, I-F18 or I-F19 according to claims 1 to 6 for conducting biological assays and chromatographic identification.

19. A method for inhibiting NAALADase activity by contacting compounds of formula I according to claim 1 with proteins exhibiting NAALADase activity in-vitro or in-vivo.

20. A kit comprising a sealed vial containing a predetermined quantity of a compound of Formula I, I-F18, I-F19 or I-LG according to claims 1 to 8, stereoisomers thereof and their mixtures, and suitable salts of inorganic or organic bases and acids thereof, hydrates, complexes, and solvates thereof.
